(19) **Europäisches Patentamt European Patent Office Office européen des brevets**

(11) **EP 3 788 172 B1**

(12) **EUROPEAN PATENT SPECIFICATION**

(45) Date of publication and mention of the grant of the patent:
**02.04.2025 Bulletin 2025/14**

(21) Application number: **19796085.9**

(22) Date of filing: **03.05.2019**

(51) International Patent Classification (IPC):
**G16B 20/10** (2019.01)    **G16B 30/00** (2019.01)
**G16B 40/00** (2019.01)

(52) Cooperative Patent Classification (CPC):
**G16B 20/10; G16B 30/00; G16B 40/00;**
C12Q 1/6881; C12Q 1/6886

(86) International application number:
**PCT/CN2019/085426**

(87) International publication number:
**WO 2019/210873 (07.11.2019 Gazette 2019/45)**

(54) **SIZE-TAGGED PREFERRED ENDS AND ORIENTATION-AWARE ANALYSIS FOR MEASURING PROPERTIES OF CELL-FREE MIXTURES**

GRÖSSENMARKIERTE BEVORZUGTE ENDEN UND AUSRICHTUNGSBEWUSSTE ANALYSE ZUR MESSUNG DER EIGENSCHAFTEN ZELLFREIER MISCHUNGEN

EXTRÉMITÉS PRÉFÉRÉES ÉTIQUETÉES PAR TAILLE ET ANALYSE SENSIBLE À L'ORIENTATION POUR MESURES DES PROPRIÉTÉS DE MÉLANGES SANS CELLULE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2018 US 201862666574 P**
**17.09.2018 US 201862732509 P**

(43) Date of publication of application:
**10.03.2021 Bulletin 2021/10**

(73) Proprietors:
• **The Chinese University Of Hong Kong**
**Hong Kong 999077 (HK)**
• **Grail, Inc.**
**Menlo Park, California 94025 (US)**

(72) Inventors:
• **LO, Yuk-Ming Dennis**
**Hong Kong (HK)**
• **CHIU, Rossa Wai Kwun**
**Hong Kong (HK)**
• **CHAN, Kwan Chee**
**Hong Kong (HK)**
• **JIANG, Peiyong**
**Hong Kong (HK)**
• **SUN, Kun**
**Hong Kong (HK)**

(74) Representative: **J A Kemp LLP**
**80 Turnmill Street**
**London EC1M 5QU (GB)**

(56) References cited:
**WO-A1-2017/012592      WO-A1-2017/012592**
**US-A1- 2016 201 142**

## Description

### BACKGROUND

Presence of circulating cell-free DNA (cfDNA) in human plasma was first reported by Mandel and Metais (86). Later on, discoveries of fetal-derived DNA in the plasma of pregnant women (82), donor-derived DNA in transplantation patients (83) and tumor-derived DNA in cancer patients (100) opened up the door of plasma DNA-based noninvasive prenatal testing (108), transplantation monitoring (97) and cancer liquid biopsies (57, 91, 61). CfDNA has thus become a biomarker class that is actively researched globally.

There is global interest in adopting circulating cell-free DNA analysis in human plasma for molecular diagnostics and monitoring. The discoveries of fetal DNA in the plasma of pregnant women (1), donor-specific DNA in organ-transplantation patients (2) and tumor-derived DNA in cancer patients (3) have enabled technologies for noninvasive prenatal testing, cancer liquid biopsies, transplant monitoring, and organ damage assessment (4-8). Despite the numerous clinical applications, the biological characteristics of the plasma DNA have not received sufficient research attention.

WO 2017/012592 A1 discloses use of a property of a fragmentation pattern of cell-free DNA to determine a proportional contribution of a particular tissue type.

### BRIEF SUMMARY

In accordance with a first aspect of the invention there is provided a method of analyzing a biological sample, including a mixture of cell-free DNA molecules from a plurality of tissues types that includes a first tissue type, to determine a classification of a proportional contribution of the first tissue type in the mixture, the method comprising: identifying a first set of genomic positions at which ends of short cell-free DNA molecules occur at a first rate above a first threshold for samples containing the first tissue type, wherein the short cell-free DNA molecules have a first size; analyzing a first plurality of cell-free DNA molecules from the biological sample of a subject, wherein analyzing a cell-free DNA molecule includes: determining a genomic position in a reference genome corresponding to at least one end of the cell-free DNA molecule; based on the analyzing of the first plurality of cell-free DNA molecules, determining that a first number of the first plurality of cell-free DNA molecules end within one of a plurality of windows, each window including at least one of the first set of genomic positions; computing a relative abundance of the first plurality of cell-free DNA molecules ending within one of the plurality of windows by normalizing the first number of the first plurality of cell-free DNA molecules using a second number of cell-free DNA molecules, wherein the second number of cell-free DNA molecules includes cell-free DNA molecules ending at a second set of genomic positions outside of the plurality of windows including the first set of genomic positions; and determining the classification of the proportional contribution of the first tissue type by comparing the relative abundance to one or more calibration values determined from one or more calibration samples whose proportional contributions of the first tissue type are known.

In one example, the fragmentation patterns of different sized cell-free DNA molecules are analyzed. Short and long DNA molecules can be associated with different preferred DNA end positions, referred to as size-tagged preferred ends. The short preferred DNA end positions correlate with certain tissue types (e.g., fetal, tumor, or transplant tissue). The preferred ending positions for short (and potentially long) DNA molecules can be identified and DNA molecules ending at such positions can be used in various applications.

In some embodiments, a relative abundance of cell-free DNA molecules ending on the preferred ending positions for short DNA molecules can be used to determine a proportional contribution of a first tissue type in a test mixture, e.g., by comparing to a similar measurement in a calibration sample for which the proportional contribution is known.

These and other embodiments of the invention are described in detail below. For example, other embodiments are directed to systems, devices, and computer readable media associated with methods described herein.

A better understanding of the nature and advantages of embodiments of the present disclosure may be gained with reference to the following detailed description and the accompanying drawings.

### BRIEF DESCRIPTION OF THE DRAWINGS

FIG. 1 shows an analysis of fragment end sites for plasma DNA fragments according to embodiments of the present disclosure.

FIG. 2 shows size distributions of the plasma DNA reads covering Set S preferred end sites (red) versus those covering Set L preferred end sites (blue) in 24 maternal plasma samples.

FIG. 3 shows a size distribution of the plasma DNA reads covering Set S and Set L preferred end sites in one maternal plasma sample according to embodiments of the present disclosure.

FIG. 4A shows the correlation between the relative abundance (S/L ratio) of plasma DNA molecules with size-tagged preferred end sites and fetal DNA fraction in 26 maternal plasma samples. FIG. 4B shows the correlation between size ratios (number of short reads to long reads) and fetal DNA fractions for the 26 maternal plasma samples.

FIG. 5A shows a comparison of relative abundance of chr21 reads between control cases and trisomy 21 cases according to embodiments of the present disclosure. FIG. 5B shows ROC comparison between reads covering Set S preferred end sites and random reads for trisomy 21 testing according to embodiments of the present disclosure.

FIG. 6 shows size distributions of the plasma DNA reads covering Set S preferred end sites versus those covering Set L preferred end sites in 24 healthy subjects.

FIG. 7A shows size distribution of the plasma DNA reads covering Set S and Set L preferred end sites in a healthy subject according to embodiments of the present disclosure. FIG. 7B shows a comparison of the relative abundance of plasma DNA reads with Set S versus Set L preferred end sites (S/L ratio) in pregnant women and healthy subjects according to embodiments of the present disclosure.

FIG. 8 shows a size distribution of the plasma DNA reads covering Set S and Set L preferred end sites in a hepatocellular carcinoma (HCC) patient according to embodiments of the present disclosure.

FIG. 9 shows size distributions of the plasma DNA reads covering Set S preferred end sites versus those covering Set L preferred end sites in a representative set of 24 hepatocellular carcinoma patients.

FIG. 10 shows the correlation between the relative abundance (S/L ratio) of plasma DNA molecules with size-tagged preferred end sites and tumor DNA fraction in 72 hepatocellular carcinoma patients with tumor DNA fraction higher than 1% in the plasma according to embodiments of the present disclosure.

FIG. 11 shows the relative abundance (S/L ratio) of plasma DNA molecules with size-tagged preferred end sites among healthy subjects and hepatocellular carcinoma patients.

FIG. 12 shows the normalized read count covering the Set S ends on chr1p among healthy subjects, HBV carriers without or with cirrhosis, and HCC patients according to embodiments of the present disclosure.

FIG. 13 shows the normalized read count covering the Set S ends on chr1q among healthy subjects, HBV carriers without or with cirrhosis, and HCC patients according to embodiments of the present disclosure.

FIG. 14 shows the normalized read count covering the Set S ends on chr8p among healthy subjects, HBV carriers without or with cirrhosis, and HCC patients according to embodiments of the present disclosure.

FIG. 15 shows the normalized read count covering the Set S ends on chr8q among healthy subjects, HBV carriers without or with cirrhosis, and HCC patients according to embodiments of the present disclosure.

FIG. 16 shows a distribution of the distance between any two closest preferred end sites in Set S and Set L preferred end sites according to embodiments of the present disclosure.

FIG. 17A shows a snapshot of the plasma DNA coverage, Set S, and Set L preferred end sites according to embodiments of the present disclosure. FIG. 17B shows a distribution of the preferred end sites surrounding the common open chromatin regions shared by placental tissues and T-cells according to embodiments of the present disclosure.

FIG. 18A shows a distribution of the size-tagged preferred end sites in pregnant plasma DNA relative to the nucleosome structure according to embodiments of the present disclosure. FIG. 18B shows a distribution of the size-tagged preferred end sites relative to the nucleosome centers predicted by Straver et al (23) according to embodiments of the present disclosure.

FIG. 19 shows the distribution of autosomal fragment ends for short and long DNA molecules in relation to the nucleosome structure in healthy non-pregnant subjects according to embodiments of the present disclosure.

FIG. 20A shows an illustration of the nucleosomal structure. FIG. 20B shows a distribution of fetal- and maternal-specific preferred end sites in the nucleosome structure. FIG. 20C shows a distribution of the chrY fragment ends of pregnant cases and healthy male subjects in the nucleosome structure. FIG. 20D shows the distribution of chrY fragment ends for short and long DNA molecules in the nucleosome structure in pregnant cases. FIG. 20E shows the distribution of chrY fragment ends for short and long DNA molecules in the nucleosome structure in healthy subjects.

FIGS. 21A and 21B show fragment size distribution from ATAC-seq data of (A) buffy coat samples and (B) placental tissues.

FIG. 22 shows the relationship between a relative abundance of cell-free DNA molecules ending on short-tagged ending positions and the proportional contribution of tissue A to DNA in a mixture determined by analysis of two or more calibration samples with known proportional concentrations of DNA from tissue A.

FIG. 23 is a flowchart of a method of analyzing a biological sample to determine a classification of a proportional contribution of the first tissue type in a mixture according to embodiments of the present disclosure.

FIG. 24 is a flowchart of a method of analyzing a biological sample to determine whether the first tissue type exhibits a sequence imbalance in a chromosomal region in the mixture of cell-free DNA molecules according to embodiments of the present disclosure.

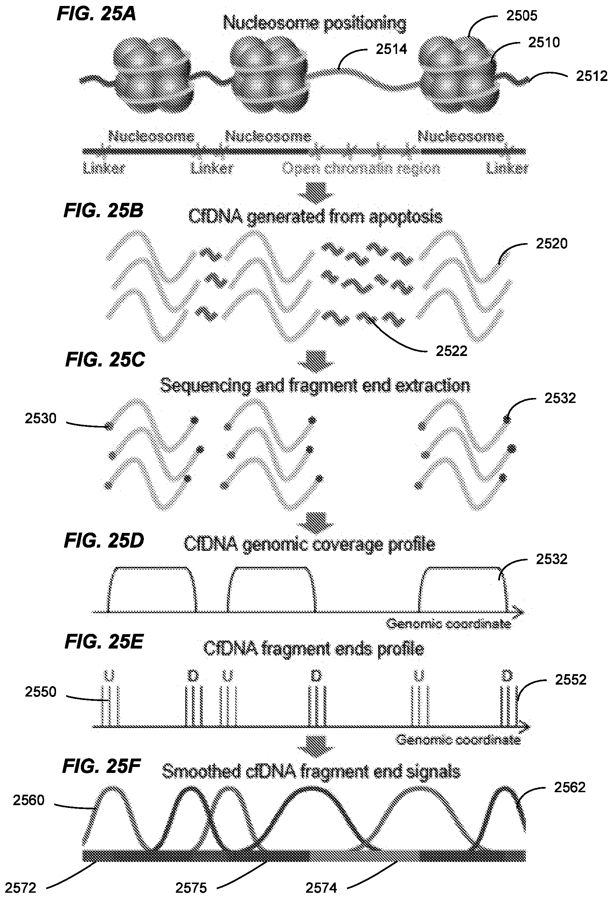

FIGS. 25A-25F show a conceptual framework of cell-free DNA (cfDNA) fragmentation analysis according to embodiments of the present disclosure. FIG. 25A is an illustration of nucleosomes with wrapped DNA (yellow line), linkers (brown line), and active regulatory elements (green line). FIG. 25B shows an illustration of cfDNA generated from apoptotic DNA fragmentation. FIG. 25C is an illustration of the sequenced reads and extraction of the two ends. Red and blue represent the U (upstream) and D (downstream) plasma DNA ends, respectively. FIG. 25D shows the genomic coverage. FIG. 25E shows U and D fragment end profiles of cfDNA in relation to the genomic coordinate. FIG. 25F shows smoothed plasma DNA end signals and deduced nucleosome positioning.

FIGS. 26A and 26B show plasma DNA fragmentation pattern in the chr12p11.1 region in pooled healthy non-pregnant subjects according to embodiments of the present disclosure. FIG. 26A shows the raw signal. FIG. 26B shows the smoothed signal and the deduced nucleosome positioning. FIG. 26C shows the plasma DNA coverage and end signals around the active promoters of housekeeping genes. FIG. 26D shows the plasma DNA coverage and end signals around inactive promoters.

FIGS. 27A, 27B, and 27C show plasma DNA fragmentation pattern in pooled healthy non-pregnant subjects according to embodiments of the present disclosure. FIG. 27A shows the pattern in common open chromatin regions shared by T-cells and liver cells (deduced nucleosome positioning was also plotted). FIG. 27B shows the pattern in embryonic stem cell (ESC)-specific open chromatin regions. FIG. 27C is an illustration of the concept of OCF (Orientation-aware cfDNA fragmentation) value.

FIGS. 28A-28G show plasma DNA fragmentation pattern in tissue-specific open chromatin regions in a healthy subject according to embodiments of the present disclosure. Each figure shows the result from tissue-specific open chromatin regions corresponding to one tissue type: FIG. 28A T-cells; FIG. 28B placenta; FIG. 28C liver; FIG. 28D lungs; FIG. 28E ovary; FIG. 28F breast; FIG. 28G intestines.

FIG. 29A shows the plasma DNA fragmentation pattern in intestine-specific open chromatin regions in one CRC patient according to embodiments of the present disclosure.

FIG. 29B shows the plasma DNA fragmentation pattern in lung-specific open chromatin regions in one lung cancer patient according to embodiments of the present disclosure.

FIG. 30 shows the quantification of plasma DNA fragmentation pattern (OCF values) among various tissues in the healthy non-pregnant subject cohort according to embodiments of the present disclosure.

FIG. 31 shows a table of OCF values for tissue types in healthy individuals according to embodiments of the present disclosure.

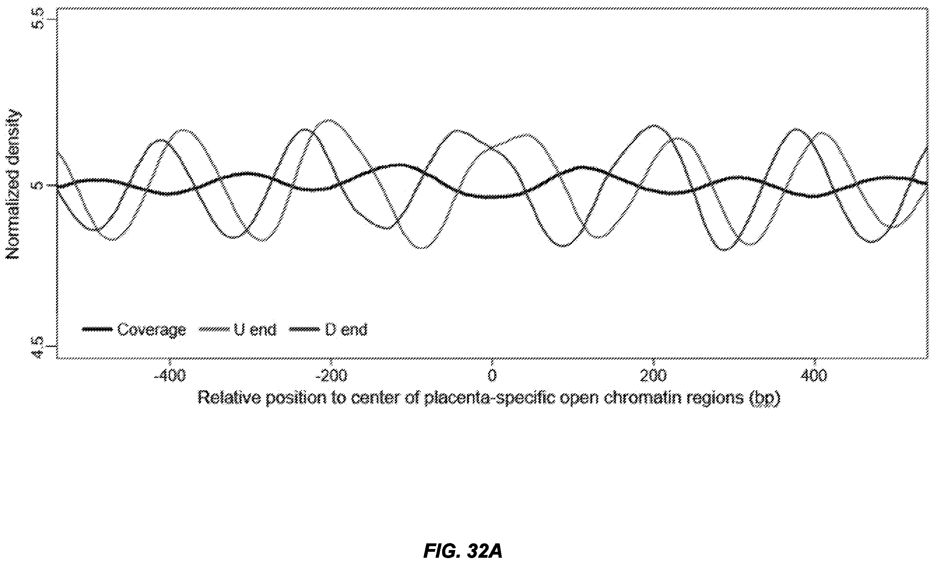

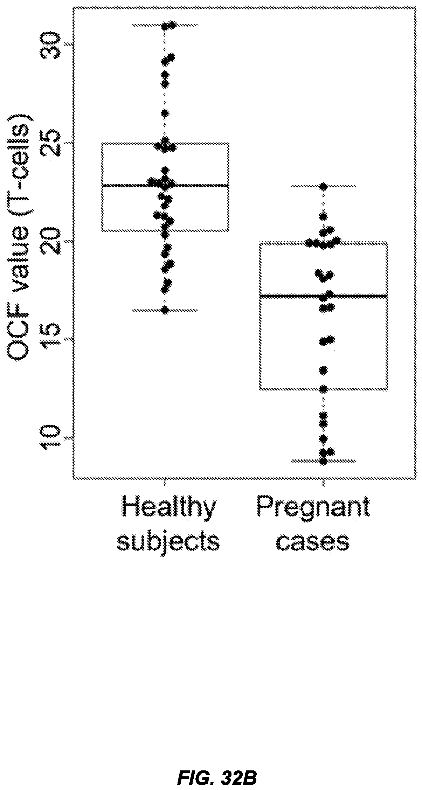

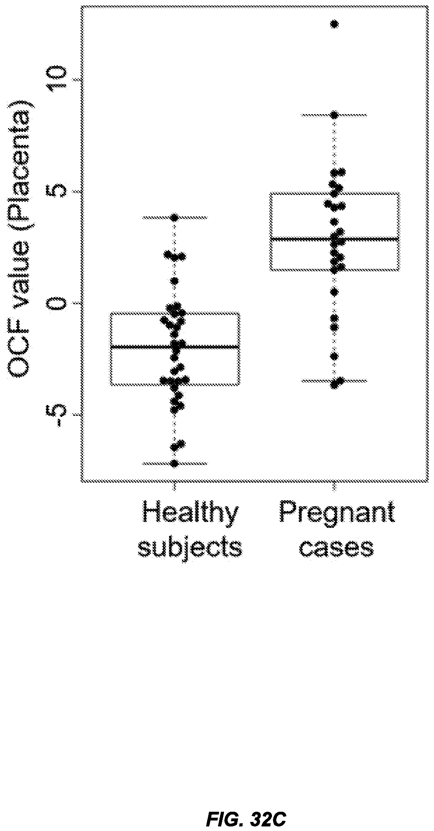

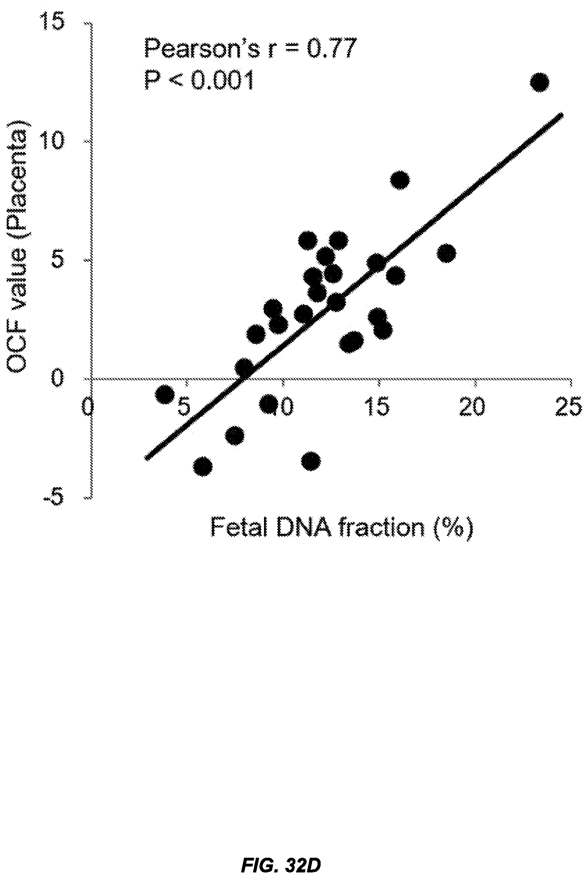

FIGS. 32A-32D show the application of the plasma DNA fragmentation pattern analysis in noninvasive prenatal testing according to embodiments of the present disclosure. FIG. 32A shows plasma DNA fragmentation pattern in the placenta-specific open chromatin regions in one pregnant case. FIG. 32B shows a comparison of OCF values for T-cells between healthy non-pregnant subjects and pregnant women. FIG. 32C shows a comparison of the OCF values for the placenta between healthy non-pregnant subjects and pregnant women. FIG. 32D shows a correlation between OCF values for placenta and fetal DNA fractions in a cohort of 26 pregnant women.

FIG. 33 shows a table of OCF values tissue types in pregnant subjects according to embodiments of the present disclosure

FIG. 34 shows a table of OCF values tissue types in liver transplantation patients according to embodiments of the present disclosure.

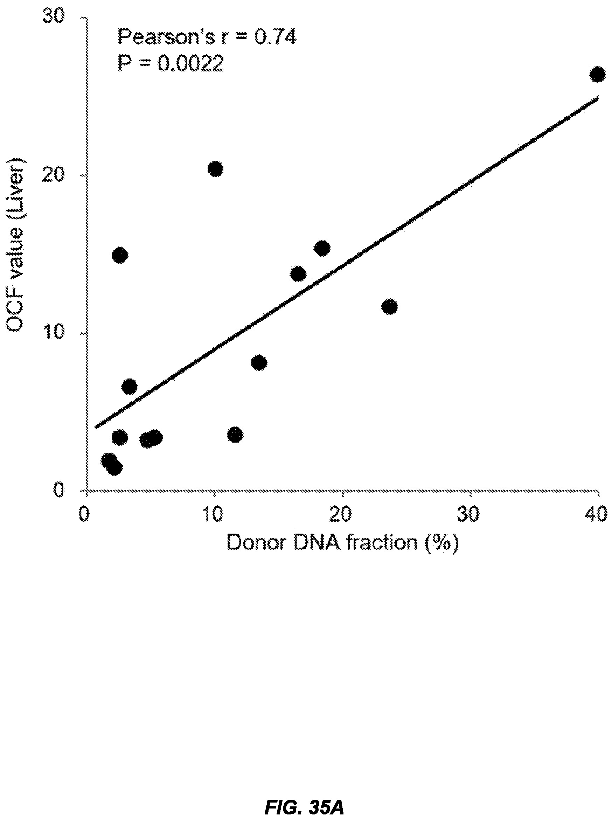

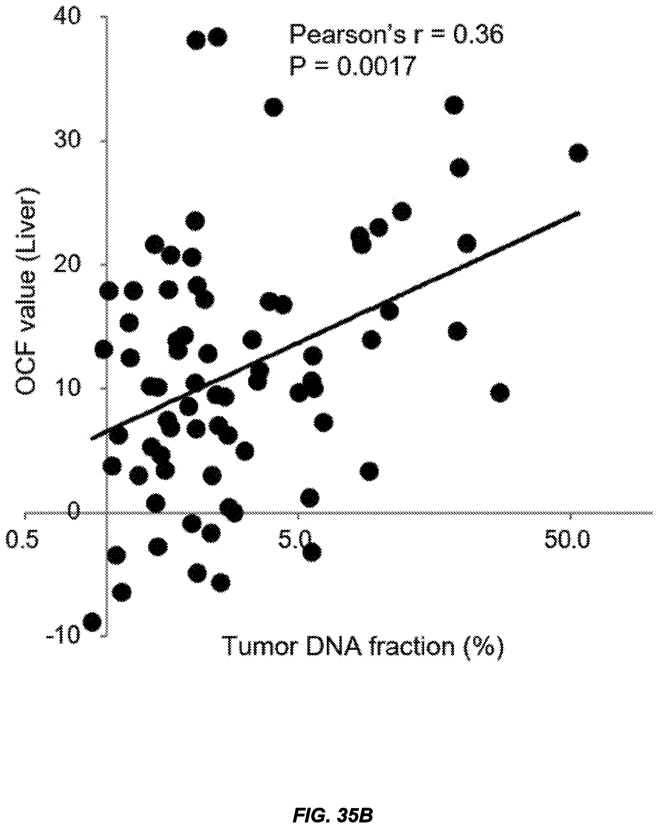

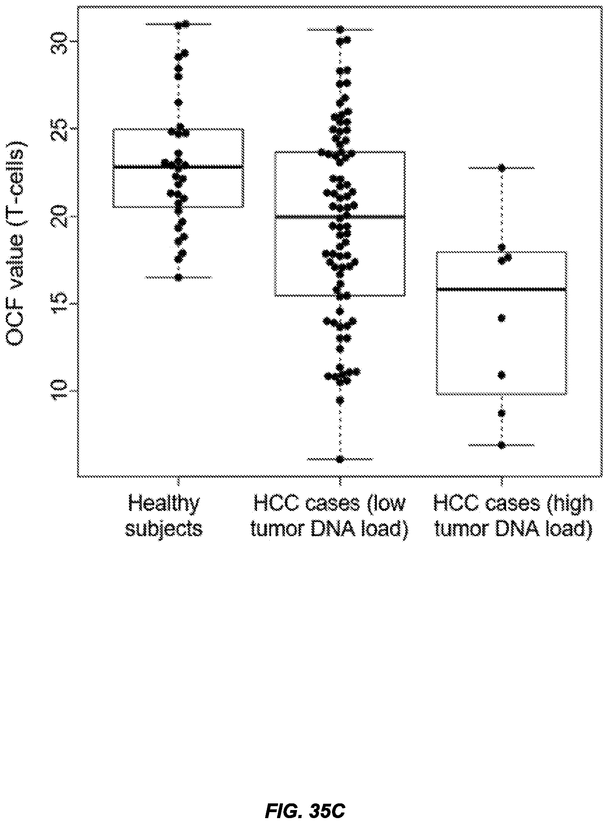

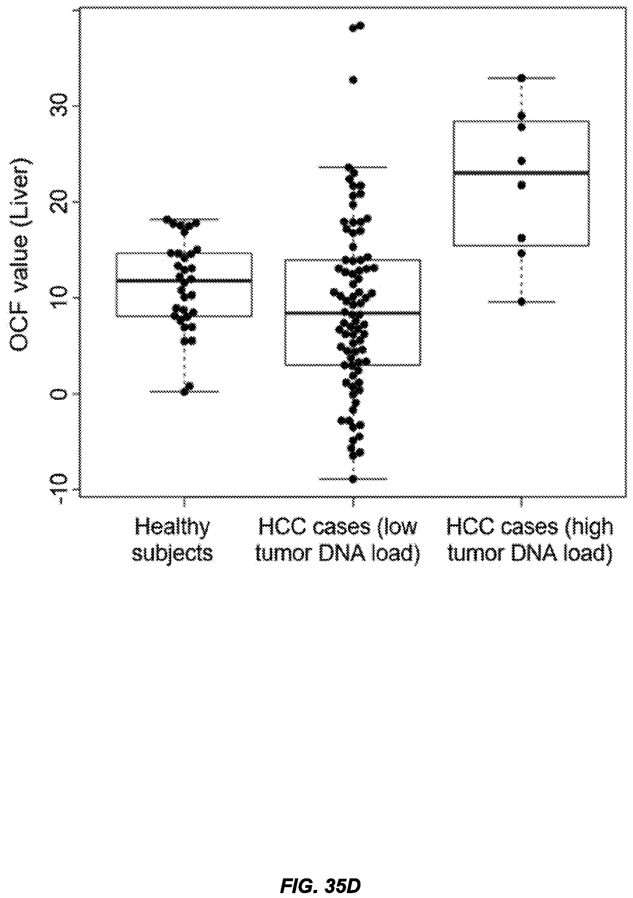

FIGS. 35A, 35B, and 35C show the application of the plasma DNA fragmentation pattern analysis in liver transplantation and HCC patients according to embodiments of the present disclosure. FIG. 35A shows the correlation between OCF values for the liver and donor DNA fractions in liver transplantation patients. FIG. 35B shows the tumor DNA fraction in HCC cases. FIG. 35C shows the comparison of OCF values for T-cells among healthy subjects and HCC cases (separated into 2 groups based on the tumor DNA load in plasma). FIG. 35D shows the comparison of OCF values for the liver among healthy subjects and HCC cases (separated into 2 groups based on the tumor DNA load in plasma).

FIGS. 36A-36D shows a table of OCF values tissue types in hepatocellular carcinoma patients according to embodiments of the present disclosure.

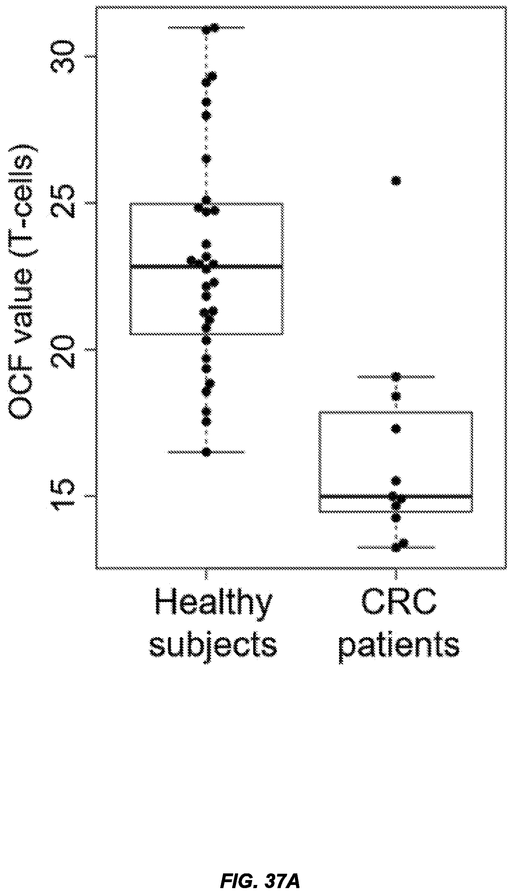

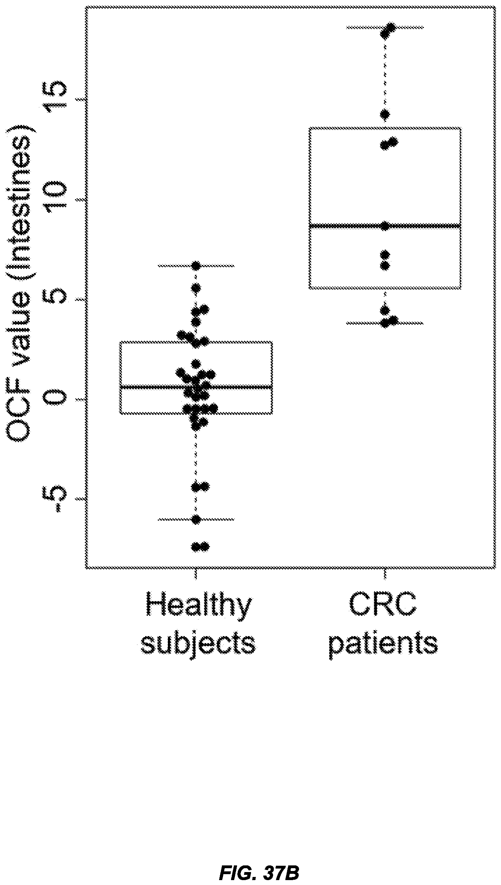

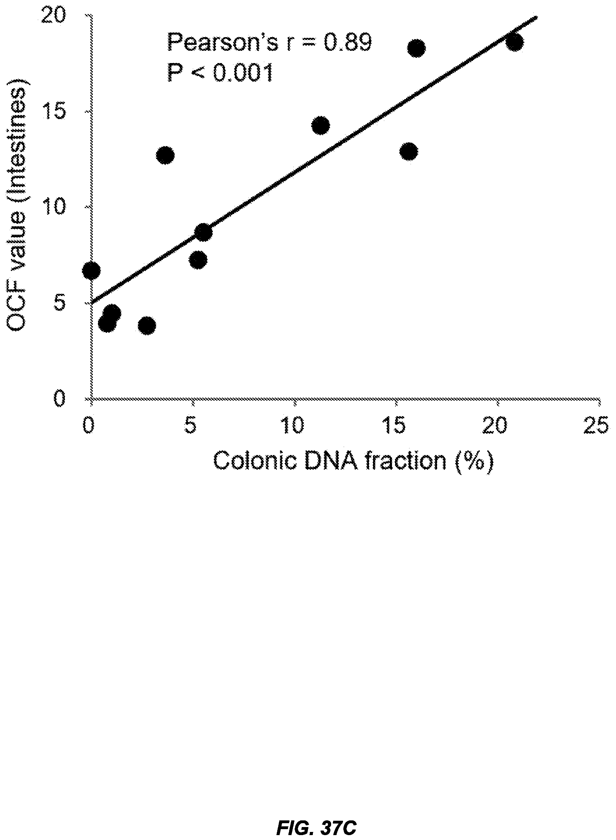

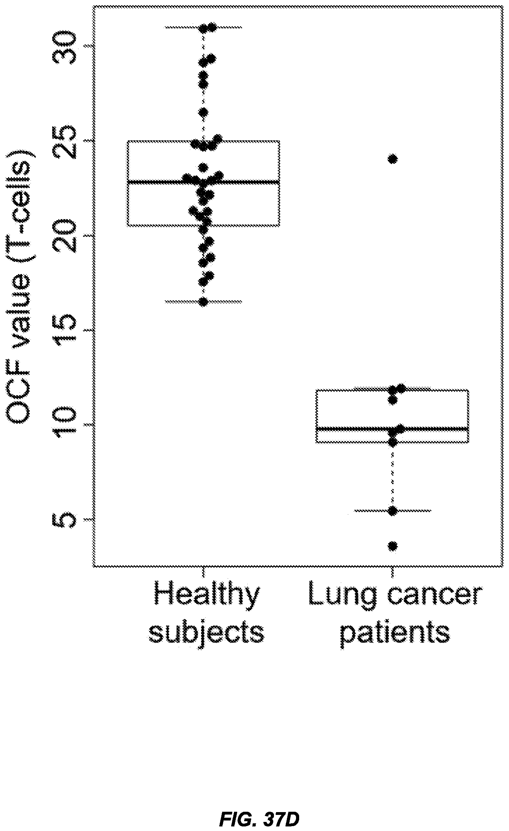

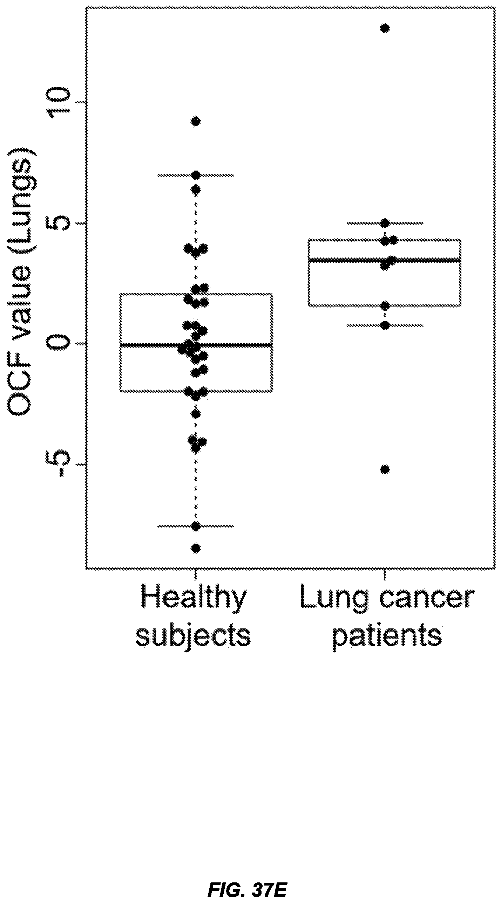

FIGS. 37A-37E show the application of the plasma DNA fragmentation pattern analysis in CRC and lung cancer patients according to embodiments of the present disclosure. FIG. 37A shows a comparison of OCF values for T-cells between healthy subjects and CRC patients. FIG. 37B shows a comparison of OCF values for intestines between healthy subjects and CRC patients. FIG. 37C shows the correlation between OCF values for intestines and colonic DNA fractions (deduced by plasma DNA tissue mapping method) in CRC patients. FIG. 37D shows a comparison of OCF values for T-cells between healthy subjects and lung cancer patients. FIG. 37E shows a comparison of OCF values for lungs between healthy subjects and lung cancer patients.

FIG. 38 shows a table of OCF values tissue types in lung cancer patients according to embodiments of the present disclosure.

FIG. 39 shows a table of OCF values tissue types in colorectal cancer patients according to embodiments of the present disclosure.

FIG. 40 is a flowchart of a method of analyzing a biological sample to determine a classification of a proportional contribution of the first tissue type in a mixture according to embodiments of the present disclosure.

FIG. 41 is a flowchart of a method of analyzing a biological sample to determine a classification of whether a pathology exists for the first tissue type in the mixture according to embodiments of the present disclosure.

FIG. 42 illustrates a measurement system according to an embodiment of the present disclosure.

FIG. 43 shows a block diagram of an example computer system usable with systems and methods according to embodiments of the present disclosure.

TERMS

A *"tissue"* corresponds to a group of cells that group together as a functional unit. More than one type of cells can be found in a single tissue. Different types of tissue may consist of different types of cells (e.g., hepatocytes, alveolar cells or blood cells), but also may correspond to tissue from different organisms (mother vs. fetus) or to healthy cells vs. tumor cells. "Reference tissues" can correspond to tissues used to determine tissue-specific methylation levels. Multiple samples of a same tissue type from different individuals may be used to determine a tissue-specific methylation level for that tissue type.

A *"biological sample"* refers to any sample that is taken from a subject (*e.g.,* a human, such as a pregnant woman, a person with cancer, or a person suspected of having cancer, an organ transplant recipient or a subject suspected of having a disease process involving an organ (e.g., the heart in myocardial infarction, or the brain in stroke, or the hematopoietic system in anemia) and contains one or more nucleic acid molecule(s) of interest. The biological sample can be a bodily fluid, such as blood, plasma, serum, urine, vaginal fluid, fluid from a hydrocele (e.g. of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (e.g. thyroid, breast), etc. Stool samples can also be used. In various embodiments, the majority of DNA in a biological sample that has been enriched for cell-free DNA (e.g., a plasma sample obtained via a centrifugation protocol) can be cell-free, e.g., greater than 50%, 60%, 70%, 80%, 90%, 95%, or 99% of the DNA can be cell-free. The centrifugation protocol can include, for example, 3,000 g x 10 minutes, obtaining the fluid part, and re-centrifuging at for example, 30,000 g for another 10 minutes to remove residual cells.

The term *"haplotype"* as used herein refers to a combination of alleles at multiple loci that are transmitted together on the same chromosome or chromosomal region. A haplotype may refer to as few as one pair of loci or to a chromosomal region, or to an entire chromosome. The term *"alleles"* refers to alternative DNA sequences at the same physical genomic locus, which may or may not result in different phenotypic traits. In any particular diploid organism, with two copies of each chromosome (except the sex chromosomes in a male human subject), the genotype for each gene comprises the pair of alleles present at that locus, which are the same in homozygotes and different in heterozygotes. A population or species of organisms typically includes multiple alleles at each locus among various individuals. A genomic locus where more than one allele is found in the population is termed a polymorphic site. Allelic variation at a locus is measurable as the number of alleles (i.e., the degree of polymorphism) present, or the proportion of heterozygotes (i.e., the heterozygosity rate) in the population.

The term "*fragment*" (e.g., a DNA fragment), as used herein, can refer to a portion of a polynucleotide or polypeptide sequence that comprises at least 3 consecutive nucleotides. A nucleic acid fragment can retain the biological activity and/or some characteristics of the parent polypeptide. A nucleic acid fragment can be double-stranded or single-stranded, methylated or unmethylated, intact or nicked, complexed or not complexed with other macromolecules, e.g. lipid particles, proteins. A fragment can be derived from a particular tissue type, e.g., fetal, tumor, a transplanted organ, etc.

The term *"assay"* generally refers to a technique for determining a property of a nucleic acid. An assay (e.g., a first assay or a second assay) generally refers to a technique for determining the quantity of nucleic acids in a sample, genomic identity of nucleic acids in a sample, the copy number variation of nucleic acids in a sample, the methylation status of nucleic acids in a sample, the fragment size distribution of nucleic acids in a sample, the mutational status of nucleic acids in a sample, or the fragmentation pattern of nucleic acids in a sample. Any assay known to a person having ordinary skill in the art may be used to detect any of the properties of nucleic acids mentioned herein. Properties of nucleic acids include a sequence, quantity, genomic identity, copy number, a methylation state at one or more nucleotide positions, a size of the nucleic acid, a mutation in the nucleic acid at one or more nucleotide positions, and the pattern of fragmentation of a nucleic acid (e.g., the nucleotide position(s) at which a nucleic acid fragments). The term "assay" may be used interchangeably with the term "method". An assay or method can have a particular sensitivity and/or specificity, and their relative usefulness as a diagnostic tool can be measured using ROC-AUC statistics.

A *"sequence read"* refers to a string of nucleotides sequenced from any part or all of a nucleic acid molecule. For example, a sequence read may be the entire nucleic acid fragment that exists in the biological sample. Also as an example, a sequence read may be a short string of nucleotides (e.g., 20-150 bases) sequenced from a nucleic acid fragment, a short string of nucleotides at one or both ends of a nucleic acid fragment, or the sequencing of the entire nucleic acid fragment that exists in the biological sample. Paired sequence reads can be aligned to a reference genome, which can provide a length of the fragment. A sequence read may be obtained in a variety of ways, e.g., using sequencing techniques or using probes, e.g., in hybridization arrays or capture probes, or amplification techniques, such as the polymerase chain reaction (PCR) or linear amplification using a single primer or isothermal amplification, or based on biophysical measurements, such as mass spectrometry. A sequence read may be obtained from a single-molecule sequencing. *"Single-molecule sequencing"* refers to sequencing of a single template DNA molecule to obtain a sequence read without the need to interpret base sequence information from clonal copies of a template DNA molecule. The single-molecule sequencing may sequence the entire molecule or only part of the DNA molecule. A majority of the DNA molecule may be sequenced, e.g., greater than 50%, 55%, 60%, 65%, 70%, 75%, 80%, 85%, 90%, 95%, or 99%.

Examples of *"clinically-relevant"* DNA include fetal DNA in maternal plasma and tumor DNA in the patient's plasma. Another example include the measurement of the amount of graft-associated DNA in the plasma of a transplant patient. A further example include the measurement of the relative amounts of hematopoietic and nonhematopoietic DNA in the plasma of a subject. This latter embodiment can be used for detecting or monitoring or prognosticating pathological processes or injuries involving hematopoietic and/or nonhematopoietic tissues.

An *"ending position"* or *"end position"* (or just *"end"*) can refer to the genomic coordinate or genomic identity or nucleotide identity of the outermost base, i.e. at the extremities, of a cell-free DNA molecule, e.g. plasma DNA molecule. The end position can correspond to either end of a DNA molecule. In this manner, if one refers to a start and end of a DNA molecule, both would correspond to an ending position. In practice, one end position is the genomic coordinate or the nucleotide identity of the outermost base on one extremity of a cell-free DNA molecule that is detected or determined by an analytical method, such as but not limited to

massively parallel sequencing or next-generation sequencing, single molecule sequencing, double- or single-stranded DNA sequencing library preparation protocols, polymerase chain reaction (PCR), or microarray. Such *in vitro* techniques may alter the true *in vivo* physical end(s) of the cell-free DNA molecules. Thus, each detectable end may represent the biologically true end or the end is one or more nucleotides inwards or one or more nucleotides extended from the original end of the molecule e.g. 5' blunting and 3' filling of overhangs of non-blunt-ended double stranded DNA molecules by the Klenow fragment. The genomic identity or genomic coordinate of the end position could be derived from results of alignment of sequence reads to a reference genome, e.g. hg19 or other human reference genome. It could be derived from a catalog of indices or codes that represent the original coordinates of the human genome. It could refer to a position or nucleotide identity on a cell-free DNA molecule that is read by but not limited to target-specific probes, mini-sequencing, DNA amplification.

A *"preferred end"* (or *"recurrent ending position"*) refers to an end that is more highly represented or prevalent (e.g., as measured by a rate) in a biological sample having a physiological (e.g. pregnancy) or pathological (disease) state (e.g. cancer) than a biological sample not having such a state or than at different time points or stages of the same pathological or physiological state, e.g., before or after treatment. A preferred end therefore has an increased likelihood or probability for being detected in the relevant physiological or pathological state relative to other states. The increased probability can be compared between the pathological state and a non-pathological state, for example in patients with and without a cancer and quantified as likelihood ratio or relative probability. The likelihood ratio can be determined based on the probability of detecting at least a threshold number of preferred ends in the tested sample or based on the probability of detecting the preferred ends in patients with such a condition than patients without such a condition. Examples for the thresholds of likelihood ratios include but not limited to 1.1, 1.2, 1.3, 1.4, 1.5, 1.6, 1.8, 2.0, 2.5, 3.0, 3.5, 4.0, 4.5, 5, 6, 8, 10, 20, 40, 60, 80 and 100. Such likelihood ratios can be measured by comparing relative abundance values of samples with and without the relevant state. Because the probability of detecting a preferred end in a relevant physiological or disease state is higher, such preferred ending positions would be seen in more than one individual with that same physiological or disease state. With the increased probability, more than one cell-free DNA molecule can be detected as ending on a same preferred ending position, even when the number of cell-free DNA molecules analyzed is far less than the size of the genome. Thus, the preferred or recurrent ending positions are also referred to as the "frequent ending positions." In some embodiments, a quantitative threshold may be used to require that ends be detected at least multiple times (e.g., 3, 4, 5, 6, 7, 8, 9, 10, 15, 20, or 50) within the same sample or same sample aliquot to be considered as a preferred end. A relevant physiological state may include a state when a person is healthy, disease-free, or free from a disease of interest. Similarly, a *"preferred ending window"* corresponds to a contiguous set of preferred ending positions.

A *"rate"* of DNA molecules ending on a position relates to how frequently a DNA molecule ends on the position. The rate may be based on a number of DNA molecules that end on the position normalized against a number of DNA molecules analyzed. Accordingly, the rate corresponds to a frequency of how many DNA molecules end on a position, and does not relate to a periodicity of positions having a local maximum in the number of DNA molecules ending on the position.

A *"calibration sample"* can correspond to a biological sample whose tissue-specific DNA fraction is known or determined via a calibration method, e.g., using an allele specific to the tissue. As another example, a calibration sample can correspond to a sample from which preferred ending positions can be determined. A calibration sample can be used for both purposes.

A *"calibration data point"* includes a *"calibration value"* and a measured or known proportional distribution of the DNA of interest (i.e., DNA of particular tissue type). The calibration value can be a relative abundance as determined for a calibration sample, for which the proportional distribution of the tissue type is known. The calibration data point can include the calibration value (e.g., measured using size-tagged ending positions or orientation-aware fragmentation) and the known (measured) the proportional distribution of the tissue type. The calibration data points may be defined in a variety of ways, e.g., as discrete points or as a calibration function (also called a calibration curve or calibration surface). The calibration function could be derived from additional mathematical transformation of the calibration data points. The calibration function can be linear or non-linear.

A *"site"* (also called a *"genomic site"*) corresponds to a single site, which may be a single base position or a group of correlated base positions, e.g., a size-preferred site, a CpG site, or larger group of correlated base positions. A *"locus"* may correspond to a region that includes multiple sites. A locus can include just one site, which would make the locus equivalent to a site in that context.

*"DNA methylation"* in mammalian genomes typically refers to the addition of a methyl group to the 5' carbon of cytosine residues (i.e. 5-methylcytosines) among CpG dinucleotides. DNA methylation may occur in cytosines in other contexts, for example CHG and CHH, where H is adenine, cytosine or thymine. Cytosine methylation may also be in the form of 5-hydroxymethylcytosine. Non-cytosine methylation, such as N6-methyladenine, has also been reported.

The *"methylation index"* for each genomic site (e.g., a CpG site) can refer to the proportion of DNA fragments (e.g., as determined from sequence reads or probes) showing methylation at the site over the total number of reads covering that site. A "read" can correspond to information (e.g., methylation status at a site) obtained from a DNA fragment. A read can be obtained using reagents (e.g. primers or probes) that preferentially hybridize to DNA fragments of a particular methylation status. Typically, such reagents are applied after treatment with a process that differentially modifies or differentially recognizes DNA molecules depending of their methylation status, e.g. bisulfite conversion, or methylation-sensitive restriction enzyme, or methylation binding proteins, or anti-methylcytosine antibodies. In another embodiment, single molecule sequencing techniques that recognize methylcytosines and hydroxymethylcytosines can be used for elucidating the methylation status and for determining a methylation index.

The *"methylation density"* of a region can refer to the number of reads at sites within the region showing methylation divided by the total number of reads covering the sites in the region. The sites may have specific characteristics, e.g., being CpG sites. Thus, the "CpG methylation density" of a region can refer to the number of reads showing CpG methylation divided by the total number of reads covering CpG sites in the region (e.g., a particular CpG site, CpG sites within a CpG island, or a larger region). For example, the methylation density for each 100-kb bin in the human genome can be determined from the total number of cytosines not converted after bisulfite treatment (which corresponds to methylated cytosine) at CpG sites as a proportion of all CpG sites covered by sequence reads mapped to the 100-kb region. This analysis can also be performed for other bin sizes, e.g. 500 bp, 5 kb, 10 kb, 50-kb or 1-Mb, etc. A region could be the entire genome or a chromosome or part of a chromosome (e.g. a chromosomal arm). The methylation index of a CpG site is the same as the methylation density for a region when the region only includes that CpG site. The "proportion of methylated cytosines" can refer the number of cytosine sites, "C's", that are shown to be methylated (for example unconverted after bisulfite conversion) over the total number of analyzed cytosine residues, i.e. including cytosines outside of the CpG context, in the region. The methylation index, methylation density and proportion of methylated cytosines are examples of *"methylation levels,"* which may include other ratios involving counts of methylated reads at sites. Apart from bisulfite conversion, other processes known to those skilled in the art can be used to interrogate the methylation status of DNA molecules, including, but not limited to enzymes sensitive to the methylation status (e.g. methylation-sensitive restriction enzymes), methylation binding proteins, single molecule sequencing using a platform sensitive to the methylation status (e.g. nanopore sequencing (Schreiber et al. Proc Natl Acad Sci 2013; 110: 18910-18915) and by the Pacific Biosciences single molecule real time analysis (Flusberg et al. Nat Methods 2010; 7: 461-465)).

*"Methylation-aware sequencing"* refers to any sequencing method that allows one to ascertain the methylation status of a DNA molecule during a sequencing process, including, but not limited to bisulfite sequencing, or sequencing preceded by methylation-sensitive restriction enzyme digestion, immunoprecipitation using anti-methylcytosine antibody or methylation binding protein, or single molecule sequencing that allows elucidation of the methylation status. A *"methylation-aware assay"* or *"methylation-sensitive assay"* can include both sequencing and non-sequencing based methods, such as MSP, probe based interrogation, hybridization, restriction enzyme digestion followed by density measurements, anti-methylcytosine immunoassays, mass spectrometry interrogation of proportion of methylated cytosines or hydroxymethylcytosines, immunoprecipitation not followed by sequencing, etc.

The term *"sequencing depth"* refers to the number of times a locus is covered by a sequence read aligned to the locus. The locus could be as small as a nucleotide, or as large as a chromosome arm, or as large as the entire genome. Sequencing depth can be expressed as 50x, 100x, etc., where "x" refers to the number of times a locus is covered with a sequence read. Sequencing depth can also be applied to multiple loci, or the whole genome, in which case x can refer to the mean number of times the loci or the haploid genome, or the whole genome, respectively, is sequenced. Ultra-deep sequencing can refer to at least 100x in sequencing depth.

A *"separation value"* (or relative abundance) corresponds to a difference or a ratio involving two values, e.g., two amounts of DNA molecules, two fractional contributions, or two methylation levels, such as a sample (mixture) methylation level and a reference methylation level. The separation value could be a simple difference or ratio. As examples, a direct ratio of x/y is a separation value, as well as x/(x+y). The separation value can include other factors, e.g., multiplicative factors. As other examples, a difference or ratio of functions of the values can be used, e.g., a difference or ratio of the natural logarithms (ln) of the two values. A separation value can include a difference and/or a ratio.

A *"relative abundance"* is a type of separation value that relates an amount (one value) of cell-free DNA molecules ending within one window of genomic position to an amount (other value) of cell-free DNA molecules ending within another window of genomic positions. The two windows may overlap, but would be of different sizes. In other implementations, the two windows would not overlap. Further, the windows may be of a width of one nucleotide, and therefore be equivalent to one genomic position. A *"separation value"* and a *"relative abundance"* are two examples of a parameter (also called a metric) that provides a measure of a sample that varies between different classifications (states), and thus can be used to determine different classifications.

The term *"classification"* as used herein refers to any number(s) or other characters(s) that are associated with a particular property of a sample. For example, a "+" symbol (or the word "positive") could signify that a sample is classified as having deletions or amplifications. The classification can be binary (e.g., positive or negative) or have more levels of classification (e.g., a scale from 1 to 10 or 0 to 1).

The terms *"cutoff"* and *"threshold"* refer to predetermined numbers used in an operation. For example, a cutoff size can refer to a size above which fragments are excluded. A threshold value may be a value above or below which a particular classification applies, e.g., a classification of a condition, such as whether a subject has a condition or a severity of the condition. A cutoff or threshold may be "a reference value" or derived from a reference value that is representative of a particular classification or discriminates between two or more classifications. Such a reference value can be determined in various ways, e.g., chosen after and based on output of the test data, as will be appreciated by the skilled person. For example, metrics can be determined for two different cohorts of subjects with different known classifications, and a reference value can be selected as representative of one classification (e.g., a mean) or a value that is between two clusters of the metrics. Accordingly, reference subjects with known classifications of one or more conditions and measured characteristic values (e.g., a methylation level, a statistical size value, or a count) can be used to determine reference levels to discriminate between the different conditions and/or classifications of a condition (e.g., whether the subject has the condition).

As another example, a reference value can be determined based on statistical simulations of samples. Any of these terms can be used in any of these contexts. As will be appreciated by one of skilled in the art, a cutoff can be selected to achieve a desired sensitivity and specificity.

The term *"chromosome aneuploidy"* as used herein means a variation in the quantitative amount of a chromosome from that of a diploid genome. The variation may be a gain or a loss. It may involve the whole of one chromosome or a region of a chromosome. A chromosomal region may correspond to a whole of one chromosome, an arm of a chromosome, or a smaller region, e.g., 50 kb, 500 kb, 1 Mb, 2 Mb, 5 Mb, or 10 Mb.

The term *"sequence imbalance"* or *"aberration"* as used herein means any significant deviation as defined by at least one cutoff value in a quantity of a clinically relevant chromosomal region (i.e., one being tested) from a reference quantity. A sequence imbalance can include chromosome dosage imbalance, allelic imbalance, mutation dosage imbalance, copy number imbalance, haplotype dosage imbalance, and other similar imbalances. As an example, an allelic imbalance can occur when a tumor has one allele of a gene deleted or one allele of a gene amplified or differential amplification of the two alleles in its genome, thereby creating an imbalance at a particular locus in the sample. As another example, a patient could have an inherited mutation in a tumor suppressor gene. The patient could then go on to develop a tumor in which the non-mutated allele of the tumor suppressor gene is deleted. Thus, within the tumor, there is mutation dosage imbalance. When the tumor releases its DNA into the plasma of the patient, the tumor DNA will be mixed in with the constitutional DNA (from normal cells) of the patient in the plasma. Through the use of methods described herein, a mutational dosage imbalance of this DNA mixture in the plasma can be detected. An aberration can include a deletion or amplification of a chromosomal region.

The term *"level of cancer"* (or more generally *"level of disease"*, *"level of pathology,"* or *"level of condition"*) can refer to whether cancer exists (i.e., presence or absence), a stage of a cancer, a size of tumor, whether there is metastasis, the total tumor burden of the body, the cancer's response to treatment, and/or other measure of a severity of a cancer (e.g. recurrence of cancer). The level of cancer may be a number (e.g., a probability) or other indicia, such as symbols, alphabet letters, and colors. The level may be zero. The level of cancer may also include premalignant or precancerous conditions (states). The level of cancer can be used in various ways. For example, screening can check if cancer is present in someone who is not known previously to have cancer. Assessment can investigate someone who has been diagnosed with cancer to monitor the progress of cancer over time, study the effectiveness of therapies or to determine the prognosis. In one embodiment, the prognosis can be expressed as the chance of a patient dying of cancer, or the chance of the cancer progressing after a specific duration or time, or the chance of cancer metastasizing. Detection can mean 'screening' or can mean checking if someone, with suggestive features of cancer (e.g. symptoms or other positive tests), has cancer. Various embodiments can determine a level of cancer for liver, lung, pancreatic, brain, colorectal, nasopharyngeal, ovarian, stomach, and blood cancers.

The terms "control", "control sample", "reference", "reference sample", "normal", and "normal sample" may be interchangeably used to generally describe a sample that does not have a particular condition, or is otherwise healthy. In an example, a method as disclosed herein may be performed on a subject having a tumor, where the reference sample is a sample taken from a healthy tissue of the subject. In another example, the reference sample is a sample taken from a subject with the disease, e.g. cancer or a particular stage of cancer. A reference sample may be obtained from the subject, or from a database. The reference generally refers to a reference genome that is used to map sequence reads obtained from sequencing a sample from the subject. A reference genome generally refers to a haploid or diploid genome to which sequence reads from the biological sample and the constitutional sample can be aligned and compared. For a haploid genome, there is only one nucleotide at each locus. For a diploid genome, heterozygous loci can be identified, with such a locus having two alleles, where either allele can allow a match for alignment to the locus.

The phrase "healthy," as used herein, generally refers to a subject possessing good health. Such a subject demonstrates an absence of any malignant or non-malignant disease. A "healthy individual" may have other diseases or conditions, unrelated to the condition being assayed, that may normally not be considered "healthy".

The terms "cancer" or "tumor" may be used interchangeably and generally refer to an abnormal mass of tissue wherein the growth of the mass surpasses and is not coordinated with the growth of normal tissue. A cancer or tumor may be defined as "benign" or "malignant" depending on the following characteristics: degree of cellular differentiation including morphology and functionality, rate of growth, local invasion, and metastasis. A "benign" tumor is generally well differentiated, has characteristically slower growth than a malignant tumor, and remains localized to the site of origin. In addition, a benign tumor does not have the capacity to infiltrate, invade, or metastasize to distant sites. A "malignant" tumor is generally poorly differentiated (anaplasia), has characteristically rapid growth accompanied by progressive infiltration, invasion, and destruction of the surrounding tissue. Furthermore, a malignant tumor has the capacity to metastasize to distant sites. "Stage" can be used to describe how advance a malignant tumor is. Early stage cancer or malignancy is associated with less tumor burden in the body, generally with less symptoms, with better prognosis, and with better treatment outcome than a late stage malignancy. Late or advanced stage cancer or malignancy is often associated with distant metastases and/or lymphatic spread.

The term "false positive" (FP) can refer to subjects not having a condition. False positive generally refers to subjects not having a tumor, a cancer, a precancerous condition (e.g., a precancerous lesion), a localized or a metastasized cancer, a non-malignant disease, or are otherwise healthy. The term false positive generally refers to subjects not having a condition, but are identified as having the condition by an assay or method of the present disclosure.

The terms "sensitivity" or "true positive rate" (TPR) can refer to the number of true positives divided by the sum of the number of true positives and false negatives. Sensitivity may characterize the ability of an assay or method to correctly identify a proportion of the population that truly has a condition. For example, sensitivity may characterize the ability of a method to correctly identify the number of subjects within a population having cancer. In another example, sensitivity may characterize the ability of a method to correctly identify one or more markers indicative of cancer.

The terms "specificity" or "true negative rate" (TNR) can refer to the number of true negatives divided by the sum of the number of true negatives and false positives. Specificity may characterize the ability of an assay or method to correctly identify a proportion of the population that truly does not have a condition. For example, specificity may characterize the ability of a method to correctly identify the number of subjects within a population not having cancer. In another example, specificity may characterize the ability of a method to correctly identify one or more markers indicative of cancer.

The term "ROC" or "ROC curve" can refer to the receiver operator characteristic curve. The ROC curve can be a graphical representation of the performance of a binary classifier system. For any given method, an ROC curve may be generated by plotting the sensitivity against the specificity at various threshold settings. The sensitivity and specificity of a method for detecting the presence of a tumor in a subject may be determined at various concentrations of tumor-derived nucleic acid in the plasma sample of the subject. Furthermore, provided at least one of the three parameters (e.g., sensitivity, specificity, and the threshold setting), and ROC curve may determine the value or expected value for any unknown parameter. The unknown parameter may be determined using a curve fitted to the ROC curve. The term "AUC" or "ROC-AUC" generally refers to the area under a receiver operator characteristic curve. This metric can provide a measure of diagnostic utility of a method, taking into account both the sensitivity and specificity of the method. Generally, ROC-AUC ranges from 0.5 to 1.0, where a value closer to 0.5 indicates the method has limited diagnostic utility (e.g., lower sensitivity and/or specificity) and a value closer to 1.0 indicates the method has greater diagnostic utility (e.g., higher sensitivity and/or specificity). See, e.g., Pepe et al, "Limitations of the Odds Ratio in Gauging the Performance of a Diagnostic, Prognostic, or Screening Marker," Am. J. Epidemiol 2004, 159 (9): 882-890. Additional approaches for characterizing diagnostic utility using likelihood functions, odds ratios, information theory, predictive values, calibration (including goodness-of-fit), and reclassification measurements are summarized according to Cook, "Use and Misuse of the Receiver Operating Characteristic Curve in Risk Prediction," Circulation 2007, 115: 928-935.

The term "about" or "approximately" can mean within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term "about" or "approximately" can mean within an order of magnitude, within 5-fold, and more preferably within 2-fold, of a value. Where particular values are described in the application and claims, unless otherwise stated the term "about" meaning within an acceptable error range for the particular value should be assumed. The term "about" can have the meaning as commonly understood by one of ordinary skill in the art. The term "about" can refer to ±10%. The term "about" can refer to ±5%.

DETAILED DESCRIPTION

Cell-free DNA in human plasma is non-randomly fragmented and reflects genomewide nucleosomal organization. In particular, cfDNA molecules possess information related to their tissues of origin. Pathologies causing death of cells from particular tissues result in perturbations in the relative distribution of DNA from the affected organs. Such tissue-of-origin analysis is particularly useful in the development of liquid biopsies for cancer, prenatal testing, and transplant monitoring. It is therefore of value to accurately determine the relative contributions of the tissues that contribute to the plasma DNA pool in a simultaneous manner.

Various novel aspects of the non-random fragmentation can be determined and used for practical applications, such as biological measurements. For example, a relationship of fragmentation, including preferred positions at the end of DNA fragments, to the size of DNA fragments was measured. This relationship can be utilized for practical applications, such as measuring a proportional contribution of a particular tissue type (e.g., fetal, tumor, or transplant tissue) and detecting a sequence imbalance in a chromosomal region of a particular tissue type. As another example, a relationship of fragmentation and tissue-specific open chromatin regions, including which ends (upstream or downstream) of DNA fragments lie near the tissue-specific open chromatin regions, was measured. A quantitative pattern of upstream ends relative to downstream ends can be used for practical applications, such as measuring a proportional contribution of a particular tissue type and detecting a pathology in a particular tissue type.

For the size analysis, we conducted an in-depth investigation on the fragmentation pattern of plasma DNA and to explore if the fragmentation mechanisms are related to the size profiles of plasma DNA. Accordingly, we studied if such preferred end sites might bear any relationship with fragment lengths of plasma DNA. We called such end sites as 'size-tagged preferred ends'. We identified preferred end sites that were preferentially associated with long and short plasma DNA molecules. Short and long plasma DNA molecules were generally associated with different preferred DNA end sites. We found that these 'size-tagged' ends showed improved accuracy in fetal DNA fraction estimation (proportional contribution) and enhanced noninvasive fetal trisomy 21 (sequence imbalance) testing, as the plasma of pregnant women exhibit non-random fragmentation with preferred end sites. Such 'size-tagged' ends can be used for other tissue types (e.g., tumor or transplant) to estimate a proportional contribution of a particular tissue type or detect a sequence imbalance.

Further analysis revealed that the fetal and maternal preferred ends were generated from different locations within the nucleosomal structure. Fetal DNA was frequently cut within the nucleosome core while maternal DNA was mostly cut within the linker region. We further demonstrate that the nucleosome accessibility in placental cells was higher than that for white blood cells, which explains the difference in the cutting positions and the shortness of fetal DNA in maternal plasma. Interestingly, the plasma DNA molecules covering the preferred ends mined from the short reads were generally shorter than those covering the preferred ends mined from the long reads even in non-pregnant healthy subjects. Because these latter samples did not contain fetal DNA, the data suggested that the interrelationship of preferred DNA ends, chromatin accessibility and plasma DNA size profile is likely a general one, extending beyond the context of pregnancy. Plasma DNA fragment end patterns have thus shed light on production mechanism and show utility in future developments in plasma DNA-based noninvasive molecular diagnostics.

We also investigated the localization of DNA fragment ends in relationship to the nucleosomal structure. In open chromatin regions, cfDNA molecules showed characteristic fragmentation patterns reflected by sequencing coverage imbalance and differentially phased fragment end signals. The latter refers to differences in the read densities of sequences corresponding to the orientation of the upstream and downstream ends of cfDNA molecules in relation to the reference genome. Such cfDNA fragmentation patterns preferentially occurred in tissue-specific open chromatin regions where the corresponding tissues contributed DNA into the plasma. Quantitative analyses of such signals allowed measuring the relative contributions of various tissues towards the plasma DNA pool, as well as detection of pathologies in particular tissue types.. These findings were validated by plasma DNA sequencing data obtained from pregnant women, organ transplantation recipients, and cancer patients. Orientation-aware plasma DNA fragmentation analysis therefore has diagnostic applications in noninvasive prenatal testing, organ transplantation monitoring, and cancer liquid biopsy.

## I. OVERVIEW OF FRAGMENTATION AND TECHNIQUES

It has been demonstrated that plasma DNA is not randomly fragmented. High resolution plasma DNA size profiling revealed a predominant peak at 166 bp and a 10-bp periodicity below 150 bp (9). This size profile has been proposed to be closely related to the nucleosomal structure (9). In this regard, the nucleosome is composed of an octamer of 4 core histone proteins (forming a "nucleosome core" wrapped by 147 bp of DNA with a ~10 bp helical repeat), linker histones, and linker DNA (mean size around 20 bp) (10). Furthermore, the fetal DNA in maternal plasma (mostly originating from placental tissues (11)) has been found to be shorter than the maternal ones (mostly originating from the hematopoietic system (12-14). The size differences in the fetal and maternal DNA molecules had been utilized in noninvasive prenatal testing, allowing fetal DNA fraction estimation, fetal chromosomal aneuploidy detection, and fetal methylome analysis (15-19). However, the mechanistic basis for this relative shortening of circulating fetal DNA is still poorly understood (9, 14, 20).

Recent studies further explored the ending pattern of plasma DNA. Ultra-deep sequencing of plasma DNA in pregnant women revealed the existence of fetal- and maternal-specific preferred end sites (21). Although these preferred end sites demonstrated potential for noninvasive prenatal testing, the molecular basis for their existence is largely unknown. In addition, plasma DNA is believed to be released from apoptotic cells (22), suggesting that the fragmentation pattern is correlated with the nucleosomal structure and chromatin states (23-25).

In this disclosure, we show that there exists a non-random fragmentation process of cell-free DNA. The non-random fragmentation process takes place to some extent in various types of biological samples that contain cell-free DNA, e.g. plasma, serum, urine, saliva, cerebrospinal fluid, pleural fluid, amniotic fluid, peritoneal fluid, and ascitic fluid. Further, the non-random fragmentation occurs for DNA fragments of different size. Cell-free DNA occurs naturally in the form of short fragments. Cell-free DNA fragmentation refers to the process whereby high molecular weight DNA (such as DNA in the nucleus of a cell) are cleaved, broken, or digested into short fragments when cell-free DNA molecules are generated or released.

Not all cell-free DNA molecules are of the same length. Some molecules are shorter than others. It has been shown that cell-free DNA, such as plasma DNA, is generally shorter and less intact, namely of poor intact probability, or poorer integrity, within open chromatin domains, including around transcription start sites, and at locations between nucleosomal cores, such as at the linker positions (Straver et al Prenat Diagn 2016, 36:614-621). Each different tissue has its characteristic gene expression profile which in turn is regulated by means including chromatin structure and nucleosomal positioning. Thus, cell-free DNA patterns of intact probability or integrity at certain genomic locations, such as that of plasma DNA, are signatures or hallmarks of the tissue origin of those DNA molecules. Similarly, when a disease process, e.g. cancer, alters the gene expression profile and function of the genome of a cell, the cell-free DNA intact probability profile derived from the cells with disease would be reflective of those cells. The cell-free DNA profile, hence, would provide evidence for or are hallmarks of the presence of the disease.

Some embodiments further enhance the resolution for studying the profile of cell-free DNA fragmentation. Instead of just summating reads over a stretch of nucleotides to identify regions with higher or lower intact probability or integrity, we studied the actual ending positions or termini of individual cell-free DNA molecules, especially plasma DNA molecules. Remarkably, our data reveal that the specific locations of where cell-free DNA molecules are cut are non-random. High molecular weight genomic tissue DNA that are sheared or sonicated *in vitro* show DNA molecules with ending positions randomly scattered across the genome. However, there are certain ending positions of cell-free DNA molecules that are highly represented within a sample, such as plasma. The number of occurrence or representation of such ending positions is statistically significantly higher than expected by chance alone. These data bring our understanding of cell-free DNA fragmentation one step beyond that of regional variation of integrity (Snyder et al Cell 2016, 164: 57-68). Here, we show that the process of cell-free DNA fragmentation is orchestrated even down to the specific nucleotide position of cutting or cleavage. We termed these non-random positions of cell-free DNA ending positions as the preferred ending positions or preferred ends.

In the present disclosure, we show that there are cell-free DNA ending positions that commonly occur across individuals of different physiological states or disease states and that occur for fragments of certain sizes. For example, there are common preferred ends shared by short DNA fragments (e.g., 60-155 bases), long DNA fragments (e.g., 170-250 bases), pregnant and non-pregnant individuals, shared by a pregnant and a cancer patient, and shared with individuals with and without cancer. On the other hand, there are preferred ends that mostly occur only in short DNA fragments, long DNA fragments, in pregnant women, only in cancer patients, or only in non-pregnant individuals without cancer. Interestingly, these pregnancy-specific or cancer-specific or disease-specific ends are also highly represented in other individuals with comparable physiological or disease state. For example, preferred ends identified in the plasma of one pregnant woman are detectable in plasma of other pregnant women.

The quantity of a proportion of such preferred ends (e.g. for short fragments) correlated with the fetal DNA fraction in plasma of other pregnant women. Such preferred ends are indeed associated with the pregnancy or the fetus because their quantities are reduced substantially in non-pregnant plasma samples. Similarly, in cancer, preferred ends identified in the plasma of one cancer patient are detectable in plasma of another cancer patient. Furthermore, the quantity of a proportion of such preferred ends (e.g., for short fragments) can correlate with the tumor DNA fraction in plasma of other cancer patients. Such preferred ends are associated with cancer because their quantities are reduced following treatment of cancer, e.g. surgical resection.

There are a number of applications or utilities for the analysis of cell-free DNA size-preferred (size-tagged) ends. They could provide information about the fetal DNA fraction in pregnancy and hence the health of the fetus. For example, a number of pregnancy-associated disorders (e.g., preeclampsia, preterm labor, intrauterine growth restriction (IUGR), fetal chromosomal aneuploidies and others) have been reported to be associated with perturbations in the fractional concentration of fetal DNA (also referred to as fetal DNA fraction, fetal fraction, or proportional contribution from fetal tissue), as compared with gestational age matched control pregnancies. Accordingly, thresholds for fractional concentrations of fetal DNA can be determined from such control pregnancies. Measured fractional concentrations of fetal DNA in new samples can be compared to the thresholds to determine a classification of a pregnancy-associated disorder. Thus, measurements of fetal DNA fraction using size-preferred ends have utility for such pregnancy-associated disorders.

The cell-free plasma DNA preferred ends associated with short DNA fragments can also reveal the tumor DNA fraction or fractional concentration in a plasma sample. Knowing the tumor DNA fraction provides information about the stage of cancer, prognosis and aid in monitoring for treatment efficacy or cancer recurrence.

A catalog of preferred ends relevant to particular physiological states or pathological states (or to different sizes of fragments) can be identified by comparing the cell-free DNA profiles of preferred ends among individuals with different physiological or pathological states (or to different sizes of fragments), e.g. non-pregnant compared with pregnant samples, cancer compared with non-cancer samples, or profile of pregnant woman without cancer compared with profile of non-pregnant cancer patients. Another approach is to compare the cell-free DNA profiles of preferred ends at different time of a physiological (e.g. pregnancy) or pathological (e.g. cancer) process. Examples of such time points include before and after pregnancy, before and after delivery of a fetus, samples collected across different gestational ages during pregnancy, before and after treatment of cancer (e.g. targeted therapy, immunotherapy, chemotherapy, surgery), different time points following the diagnosis of cancer, before and after progression of cancer, before and after development of metastasis, before and after increased severity of disease, or before and after development of complications.

A preferred end can be considered relevant for a physiological or disease state (or for a certain size of fragment) when it has a high likelihood or probability (rate) for being detected in that physiological or pathological state. In other embodiments, a preferred end is of a certain probability more likely to be detected in the relevant physiological or pathological state than in other states. Because the probability of detecting a preferred end in a relevant physiological or disease state is higher, such preferred or recurrent ends (or ending positions) would be seen in more than one individual with that same physiological or disease state. The high probability would also render such preferred or recurrent ends to be detectable many times in the same cell-free DNA sample or aliquot of the same individual. In some embodiments, a quantitative threshold may be set to limit the inclusion of ends that are detected at least a specified number of times (e.g., 5, 10, 15, 20, etc.) within the same sample or same sample aliquot to be considered as a preferred end.

After a catalog of cell-free DNA preferred ends is established for any physiological or pathological state (or for different sizes), targeted or non-targeted methods could be used to detect their presence in cell-free DNA samples, e.g. plasma, or other individuals to determine a classification of the other tested individuals having a similar health, physiologic or disease state. The cell-free DNA preferred ends could be detected by random non-targeted sequencing. The sequencing depth would need to be considered so that a reasonable probability of identifying all or a portion of the relevant preferred ends could be achieved. Alternatively, hybridization capture of loci with high density of preferred ends could be performed on the cell-free DNA samples to enrich the sample with cell-free DNA molecules with such preferred ends following but not limited to detection by sequencing, microarray, or the PCR. Yet, alternatively, amplification based approaches could be used to specifically amplify and enrich for the cell-free DNA molecules with the preferred ends, e.g. inverse PCR, rolling circle amplification. The amplification products could be identified by sequencing, microarray, fluorescent probes, gel electrophoresis and other standard approaches known to those skilled in the art.

In practice, one end position can be the genomic coordinate or the nucleotide identity of the outermost base on one extremity of a cell-free DNA molecule that is detected or determined by an analytical method, such as but not limited to massively parallel sequencing or next-generation sequencing, single molecule sequencing, double- or single-stranded DNA sequencing library preparation protocols, PCR, other enzymatic methods for DNA amplification (e.g. isothermal amplification) or microarray. Such *in vitro* techniques may alter the true *in vivo* physical end(s) of the cell-free DNA molecules. Thus, each detectable end may represent the biologically true end or the end is one or more nucleotides inwards or one or more nucleotides extended from the original end of the molecule. For example, the Klenow fragment is used to create blunt-ended double-stranded DNA molecules during DNA sequencing library construction by blunting of the 5' overhangs and filling in of the 3' overhangs. Though such procedures may reveal a cell-free DNA end position that is not identical to the biological end, clinical relevance could still be established. This is because the identification of the preferred being relevant or associated with a particular physiological or pathological state could be based on the same laboratory protocols or methodological principles that would result in consistent and reproducible alterations to the cell-free DNA ends in both the calibration sample(s) and the test sample(s). A number of DNA sequencing protocols use single-stranded DNA libraries (Snyder et al Cell 2016, 164: 57-68). The ends of the sequence reads of single-stranded libraries may be more inward or extended further than the ends of double-stranded DNA libraries.

The genome identity or genomic coordinate of the end position could be derived from results of alignment of sequence reads to a reference genome for the subject, e.g. hg19 or other human reference genome. It could be derived from a catalog of indices or codes that represent the original coordinates of the human genome. While an end is the nucleotide at one or both extremities of a cell-free DNA molecule, the detection of the end could be done through the recognition of other nucleotide or other stretches of nucleotides on the plasma DNA molecule. For example, the positive amplification of a plasma DNA molecule with a preferred end detected via a fluorescent probe that binds to the middle bases of the amplicon. For instance, an end could be identified by the positive hybridization of a fluorescent probe that binds to some bases on a middle section of a plasma DNA molecule, where the fragment size known. In this way, one could determine the genomic identity or genomic coordinate of an end by working out how many bases are external to the fluorescent probe with known sequence and genomic identity. In other words, an end could be identified or detected through the detection of other bases on the same plasma DNA molecule. An end could be a position or nucleotide identity on a cell-free DNA molecule that is read by but not limited to target-specific probes, mini-sequencing, and DNA amplification. Further details can be found in PCT Publication WO2017/012592.

## II. FRAGMENTATION OF SHORT AND LONG FRAGMENTS

Integrative analysis of plasma DNA size and preferred DNA end sites was performed. A difference between the ending positions of short DNA fragments and long DNA fragments is observed, thereby illustrating size-tagged preferred ends. Various definitions of short and long DNA fragments may be used, e.g., various ranges of lengths can be used. For example, the short DNA fragments correspond to a range that has a minimum and/or a maximum that is less than a minimum and/or a maximum of a range for the long DNA fragments. Although examples may be used with plasma, other cell-free samples may be used, as the cell-free DNA in the samples also result for a natural fragmentation process.

### A. Size-tagged preferred end sites.

Fetally-derived DNA molecules are generally shorter than maternally-derived DNA molecules in maternal plasma (9, 14). Size profiling of DNA molecules in maternal plasma was performed using paired-end sequencing and alignment to a reference genome, although sequencing of an entire DNA fragment can be performed. We pooled the previously published plasma DNA paired-end sequencing data of two maternal plasma samples (20) together to attain a total of ~470-fold human haploid genome coverage. We separated the plasma DNA reads into SHORT and LONG categories, as described herein. We then determined if certain locations in the human genome might have a significantly increased probability of being present at an end of a plasma DNA molecule in the SHORT and/or LONG categories using a Poisson distribution based statistical model, as described below. Other distributions may be used, e.g., binomial distribution, negative binomial distribution, normal distribution, and Gamma distribution.

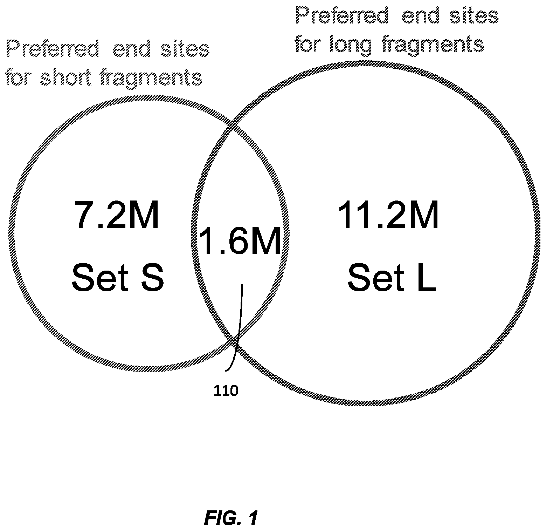

FIG. 1 shows an analysis of fragment end sites for plasma DNA fragments according to embodiments of the present disclosure. Set S and Set L include the preferred end sites for short and long plasma DNA molecules, respectively. The overlapping set 110 in the middle included the preferred end sites for both short and long plasma DNA molecules. As described in more detail below, a quantitative measurement of the cell-free DNA molecules that have ending positions that correspond to the set S can be used to characterize a particular tissue type, e.g., determine a proportional contribution of a tissue type or a sequence imbalance for the tissue type.

We obtained 8,832,009 and 12,889,647 preferred ends for the SHORT and LONG categories, respectively. Among these preferred ends, 1,649,575 ends were found to be shared by the two categories. We then collected the preferred ends across the genome that only appeared in the SHORT category ($n$ = 7,182,434) or LONG category ($n$ = 11,240,072) and defined them as Set S and Set L, respectively. These two sets contained the size-tagged preferred end sites. Subsets of set S and/or set L may be used.

A similar process may be performed for other classes of subjects, e.g., subjects with cancer or with transplanted organs that have a tissue type (e.g., tumor or transplant) that is generally shorter than DNA fragments from healthy tissue. However, size-preferred ending sites may be re-used across classes of subjects. Different definitions for short and long could be used for different classes of subjects.

### B. Identification of preferred ending sites

For the fetal analysis, we pooled the previously published plasma DNA sequencing data of two pregnant women (21) together, which achieved a total of ~470-fold human haploid genome coverage. We then separated the sequencing reads into two categories based on the size of the DNA molecules: one category for reads within a size range of 60 bp to 155 bp (denoted as SHORT) and the other for reads within a size range of 170 bp to 250 bp (denoted as LONG). The exact selection of size range settings can involve trade-offs between the difference in apparent fetal DNA fractions in the two categories and the sequencing depths of the data for both categories. As a result, ~30% and ~35% reads of the pooled data, which responded to ~140- and 165-fold human haploid genome coverages, fell in SHORT and LONG categories, respectively. These reads were collected and used in the following analyses.

Other examples of short DNA molecules include 70-145 bp, 80-145 bp, 90-145 bp, 80-135 bp, 90-135 bp, etc. Other examples of long DNA molecules include 160-210 bp, 160-220 bp, 160-230 bp, 160-240 bp, 180-260 bp, 160-260 bp, etc. Further the ranges can overlap, e.g., short being 60-155 bp and long being 150-230 bp, or short being 90-185 bp and long being 170-250 bp. In such overlap situations, the first range of sizes is still less than the second range of sizes in that a first maximum of the first range of sizes is less than a second maximum of the second range of sizes. As even another example, the long fragments could be all fragment lengths.

For the reads in each size category, we screened all nucleotide positions in a genomewide manner to search for the loci showing a significant overrepresentation of being an end of a plasma DNA molecule. For each nucleotide position, we counted the occurrences of plasma DNA ends and compared the results to those from locations surrounding that position, e.g., using a window of 1,000 bp, although other window sizes may be used, such as 500 bp or larger. The window can have a center at the location being analyzed.

A Poisson distribution based p-value would be calculated to determine if a particular position had a significantly increased probability for being an end for the reads, namely a preferred end site:

$$P \text{ value} = \text{Poisson}(N_{actual}, N_{predict})$$

where Poisson() is the Poisson probability function, $N_{actual}$ is the actual number of molecules terminating at a particular nucleotide (genomic position), and $N_{predict}$ is the total number of reads within an adjacent 1,000-bp window (e.g., centered around the particular nucleotide) divided by the mean fragment size of DNA fragments that window (or a mean size of DNA fragments generally in the sample). In various examples, a read may be defined as being within a window when the entire fragment is within the window or just when the fragment is partially within the window. In other implementations, $N_{predict}$ for a genomic position can be the number of reads that cover that position divided by a mean or expected fragment size. Accordingly, implementations can determine a global parameter and compare all sites to the global parameter instead of a local window. $N_{predict}$ is an example of a reference value (reference rate) for determining whether a rate of short (or long) DNA molecules ending on a position is above a threshold (e.g., determining whether there is a statistically significant difference from the reference value). Such examples illustrate a reference value being determined using a number of DNA fragments ending at a window centered around a particular genomic position divided by a mean size of cell-free DNA molecules.

The p-values may be further adjusted using the Benjamini method. A p-value of < 0.01 was used to indicate statistically significant end sites. Such a p-value is an example of a threshold used to determine if the rate of cell-free DNA molecules ending at the positions is sufficiently high to be considered a preferred end.

In other examples, a relative amount of short DNA molecules ending at positions can be tracked and peaks in the distribution can be determined, e.g., as shown in later figures. The tracking of peaks effectively compares the number of short DNA molecules ending at a position relative to the number ending at other positions, which act as a reference value.

Per the above examples and other herein, the reference value (also referred to as reference rate) can be determined from the numbers of the second plurality of cell-free DNA molecules ending at genomic positions outside of the particular genomic position (or a small window around that position). In this manner, it can be determine that more DNA fragments are ending on a particular positon than around other positions (e.g. around that particular position) by a statistically significant amount. This would include identifying a particular genomic position at a peak relative to numbers of DNA fragments ending at the genomic positions within a window around the particular genomic position.

Accordingly, in various examples, a first set of genomic positions at which ends of cell-free DNA molecules of a certain size (e.g., short) occur at a rate above a threshold can be identified in the following manner. A first tissue type can be associated with short DNA fragments, and thus also with preferred ending positions for short DNA fragments. A calibration sample can be analyzed in a similar manner as the test sample, where the two samples of a same type (e.g., plasma, serum, urine, etc.) and the calibration sample is known to include the first tissue type (e.g., fetal tissue from a sample of a pregnant female or tumor tissue of the liver for an HCC patient). A number of cell-free DNA molecules ending in a genomic window (e.g., of width one or more) can be compared to a reference value to determine whether a rate of ending positions is above a threshold for that position. In some embodiments, if the rate exceeds the reference value, each of the genomic positions within the first genomic window can be identified as having the rate be above the threshold when the corresponding number exceeds the reference value. Such a process can identify preferred ending windows, which include preferred ending positions.

The reference value can be such that only the top N genomic windows have a rate above the threshold. For example, the first set of genomic positions can have the highest N values for the corresponding numbers. As examples, N can be at least 10,000; 50,000; 100,000; 500,000; 1,000,000; or 5,000,000.

As another example, the reference value can be an expected number of cell-free DNA molecules ending within the genomic window according to a probability distribution and an average length of cell-free DNA molecules in a sample, in a similar manner as described above. A p-value can be determined using the corresponding number and the expected number, wherein the threshold corresponds to a cutoff p-value (e.g., 0.01). The p-value being less than the cutoff p-value indicates that the rate is above the threshold. As yet another example, the reference value can include a measured number of cell-free DNA molecules ending within the genomic window from a sample identified as having a reduced amount of the first tissue type.

### III. FETAL USE OF SIZE-TAGGED PREFERRED END SITES

The preferred ending sites can be used for measuring clinically-relevant DNA, e.g., fetal DNA, tumor DNA, or donor DNA, which have different fragmentation patterns than healthy DNA. The preferred ending sites could be mined from historical datasets derived from clinically-relevant samples. The practice of the technology on subsequent samples or specimens could be based on searching for the presence or absence or quantifying those preferred ending sites in each test sample. This section describes applications of size-tagged preferred end sites in noninvasive prenatal testing.

To investigate the potential application of size-tagged preferred end sites for noninvasive prenatal testing, we reanalyzed a maternal plasma DNA sequencing dataset that we had previously generated from 26 first-trimester pregnant women (21). For each case, we examined the reads that ended on the Set S and Set L preferred ends, respectively.

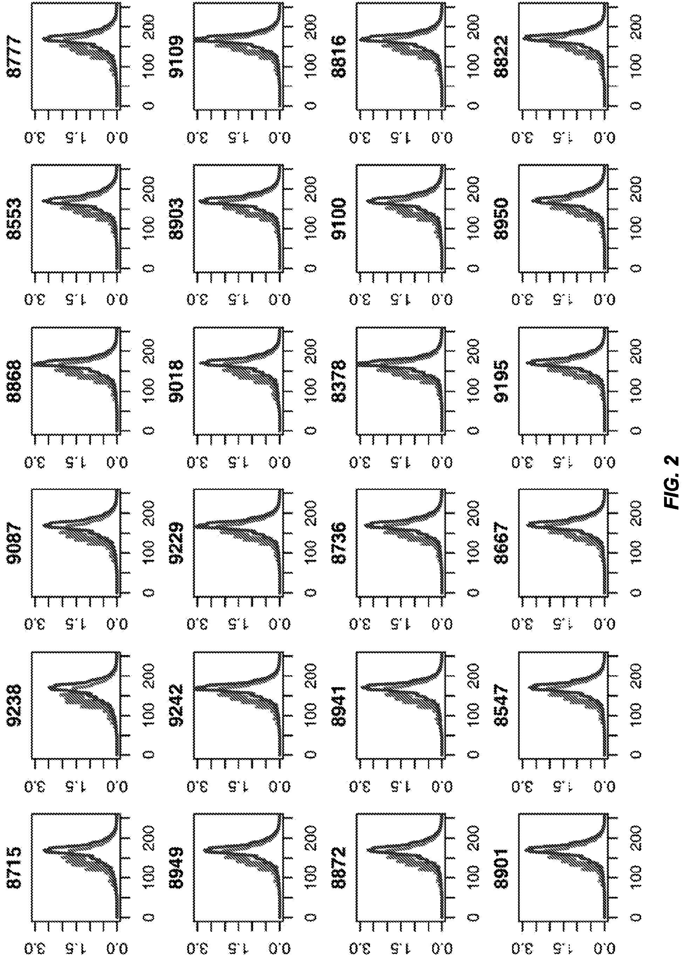

FIG. 2 shows size distributions of the plasma DNA reads covering Set S preferred end sites (red) versus those covering Set L preferred end sites (blue) in 24 maternal plasma samples. The X-axis denotes the fragment size (bp) and Y-axis denotes the frequency (%). We observed that for all these cases, the plasma DNA reads covering the Set S preferred end sites were shorter than those covering Set L preferred end sites.

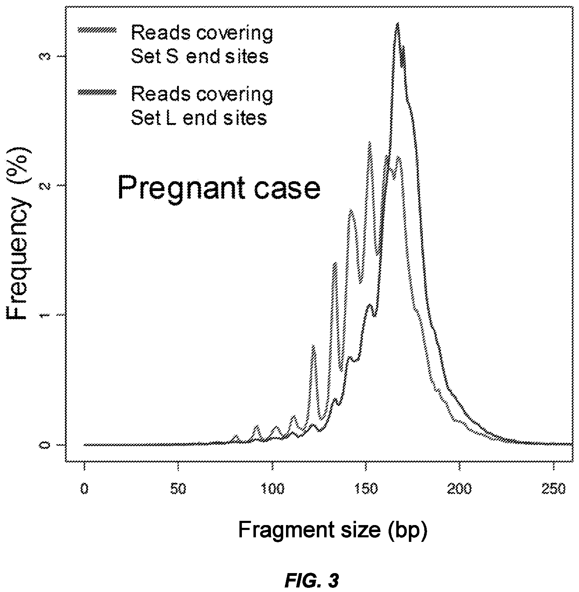

FIG. 3 shows a size distribution of the plasma DNA reads covering Set S and Set L preferred end sites in one maternal plasma sample according to embodiments of the present disclosure. As for FIG. 2, the X-axis denotes the fragment size (bp) and Y-axis denotes the frequency (%). The size distribution of the reads covering set S end sites has a well-defined periodicity with peaks and valleys between the peak for sizes between about 80 bp to about 150 bp. Each peak is about every 10 bp.

*A. Determining fetal fraction*

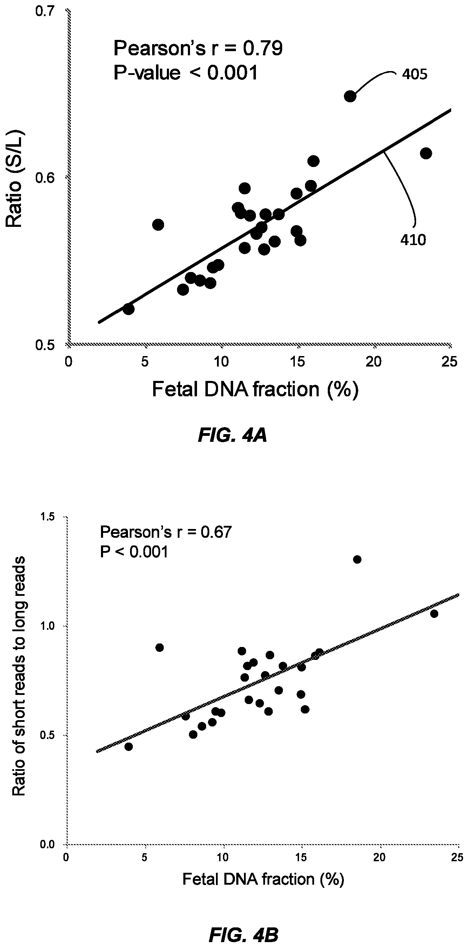

FIG. 4A shows the correlation between the relative abundance (S/L ratio) of plasma DNA molecules with size-tagged preferred end sites and fetal DNA fraction in 26 maternal plasma samples. The relative abundance can be determined by counting a first number of cell-free DNA molecules that end at one of the set S sites and dividing by a second number of cell-free DNA molecules that end at one of the set L sites. Each calibration data point 405 corresponds to a difference sample whose relative abundance and fetal DNA fraction was determined. The fetal DNA fraction can be determined using a fetal-specific marker, e.g., a paternal-specific allele, a Y chromosome marker, or a fetal-specific epigenetic marker, such as methylation.

A positive correlation was observed between the relative abundance of plasma DNA with Set S versus Set L preferred end sites [denoted as S/L ratio] and the fetal DNA fraction (R = 0.79, P < 0.001, Pearson correlation). Other values for the relative abundance may be used, e.g., the first number divided by a sum of the first number and the second number or the first number divided by all reads. Other examples of separation values may also be used, e.g., as defined in the Terms section above.

To determine a fetal DNA fraction for a new sample, a system can determine the relative abundance of cell-free DNA molecules ending at a set of short-preferred end positions compared to other cell-free DNA molecules (e.g., ones ending at a set of long-preferred end positions). Then, the newly measured relative abundance can be compared to one or more of the calibration data points 405. For example, a calibration function 410 can be fit to the calibration data points 405, where the newly measured relative abundance can be used as an input to the calibration function 410, which provides an output of fetal DNA fraction. The proportional contribution for other tissue types can be measured in a similar manner.

Notably, this R value was higher than the R value obtained by preferred end sites mined using a SNP-based approach (which was 0.66) (21). Of note, the mining of size-tagged preferred end sites did not require knowledge about fetomaternal genetic polymorphisms. On the other hand, our group had previously demonstrated that the size information alone could indicate the fetal DNA fraction in plasma DNA (17). We therefore calculated the size ratio of maternal plasma DNA without selection for molecules with specific ends and assessed its relationship with the fetal DNA fraction.

FIG. 4B shows the correlation between size ratios (number of short reads to long reads) and fetal DNA fractions for the 26 maternal plasma samples. The size ratio was positively correlated with the fetal DNA fraction (R = 0.67, P < 0.001, Pearson correlation). While the R value was comparable to that of the previous study (17), it is lower than the correlation based on size-tagged preferred ends. Together, the results suggested that the size-tagged preferred ends allowed improved fetal DNA fraction estimation in the plasma DNA.

Accordingly, the use of the preferred end positions for short DNA molecules can provide a classification of the proportional contribution of fetal tissue by comparing the relative abundance to one or more calibration values determined from one or more calibration samples whose proportional contributions of fetal tissue are known. As described herein, the classification can be a specific percentage or a range of percentages. For other tissue types, such as tumor tissue, the classification can be whether any tumor tissue is measured, or at least an appreciable amount (e.g., above a minimum threshold for detection).

In some embodiments, the size-tagged preferred ending positions can be extended to include the neighboring nucleotides. Thus, a set of short-preferred ending positions can include an expanded set S of ending sites. In either case, a number of DNA fragments ending on short-preferred positions (set S or expanded set S) can be normalized to obtain a relative abundance using a second number of DNA fragments, at least some of which end at positions outside of the short-preferred set. The second number may be inclusive of the first number for the short-preferred set. In one example, a window-based relative abundance (e.g., a ratio) can be taken between the numbers of fragments ending within Window A (smaller) and those ending outside of the window or within a larger Window B around the short-preferred ending position, therefore including some non-preferred positions. The size of Window A and Window B can be adjusted to achieve the desired performance. The performance of difference window sizes can be obtained experimentally. The size of Window A can be set, for example but not limited to 2 bp, 3 bp, 4 bp, 5 bp, 6 bp, 7 bp, 8 bp, 9 bp, 10 bp, 15 bp, 20 bp, 25 bp and 30 bp. The size of Window B would be larger than that of Window A and can be set, for example but not limited to 20 bp, 25 bp, 30 bp, 40 bp, 50 bp, 60 bp, 70 bp, 80 bp, 100 bp, 120 bp, 140 bp, 160 bp, 180 bp and 200 bp.

*B. Fetal aneuploidy detection*

In addition, we investigated whether the size-tagged preferred end sites can be used to detect a sequence imbalance in the fetal tissue for a chromosomal region, e.g., to detect copy number aberrations. The DNA molecules ending on the size-tagged preferred end sites will have a higher probability of being from the fetus than selecting any DNA fragment at random. Such enrichment of fetal DNA can increase the accuracy of techniques for performing non-invasive prenatal testing. As examples, such techniques can use an amount of cell-free DNA molecules ending at the short-preferred end sites, as well as a statistical value of a size distribution or a methylation level of such cell-free DNA molecules, which can then be compared to a reference value.

To this end, we investigated whether the size-tagged preferred end sites could improve the noninvasive prenatal testing of fetal trisomy 21. To do this, we collected a dataset from our previous study which contained 36 trisomy 21 cases and 108 control cases (17). We took advantage of the reads covering the Set S preferred ends for this analysis. Notably, the median number of reads with Set S preferred ends in these samples was 133,702 (range: 52,072 - 353,260).

Some implementations can normalize a first number of such reads mapped to chr21 by a second number of reads with Set S preferred ends mapped to all autosomes using a Z-score-based method (26) to obtain a parameter value that can be compared to a reference value that discriminates between the two classifications. In this case, a reference value can be determined from euploid cases, with a standard deviation of 3 or other suitable deviation. Thus, a reference value can be determined from control samples. The normalization can account for differences in a size of samples, e.g., a test sample and a control sample, as different numbers of DNA molecules may be analyzed. Any suitable normalization technique can be used for any of the applications for any of the tissue types, e.g., by analyzing the same number of sequence reads across samples.

Other parameter values for count-based techniques can include various ratios involving the first number, such as an S/L ratio for the region, divided by a second number (e.g., an S/L ratio) for one or more reference regions. The one or more reference regions can include at least one other regions that is expected to not have a sequence imbalance (e.g., have two chromosome copies). The use of only DNA fragments that end on the short preferred ends is a way to enrich for fetal DNA, and thus obtain greater accuracy, e.g., since the fetal DNA will be a greater percentage of the sample and larger percentage deviations from the reference value will occur.

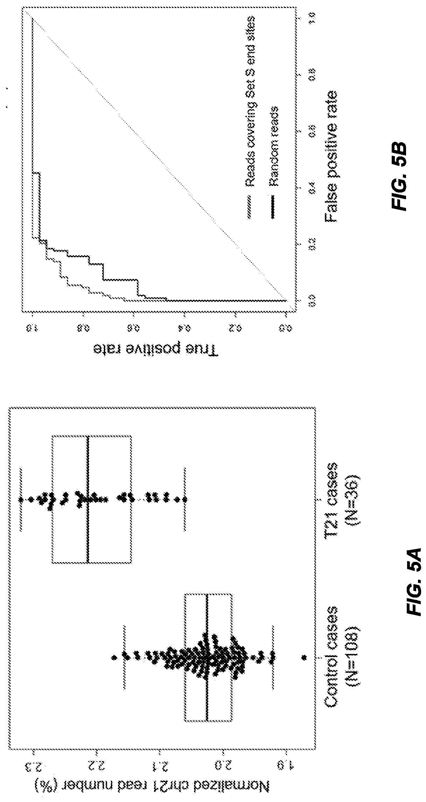

FIG. 5A shows a comparison of relative abundance of chr21 reads between control cases and trisomy 21 cases according to embodiments of the present disclosure. Only the reads covering the Set S preferred end sites (median read number: 133,702) was considered in this analysis. As shown in FIG. 5A, the trisomy 21 cases showed a significantly elevated normalized chr21 reads with Set S preferred ends than the control cases (P < 0.001, Mann-Whitney rank-sum test).

FIG. 5B shows ROC comparison between reads covering Set S preferred end sites and random reads for trisomy 21 testing according to embodiments of the present disclosure. The random read analysis just uses any reads, as opposed to filtering for preferred end sites. Using Receiver Operating Characteristic (ROC) curve analysis, we obtained an Area Under the Curve (AUC) value of 0.97. To achieve a fair comparison in terms of read number, we down-sampled the sequencing data for each sample by randomly selecting equal number of reads as those covering the Set S preferred end sites and re-calculating the normalized chr21 read number in the down-sampled dataset. As a result, the random reads showed a lower AUC value (0.93) in trisomy 21 detection compared to the reads covering the Set S preferred end sites (P = 0.033, DeLong test (27); FIG. 5B). These results suggested that the Set S preferred end sites could potentially enhance trisomy 21 testing in assays designed to exploit their characteristics (see Discussion).

Besides a fetal aneuploidy caused by deletion or amplification of a chromosome copy, other copy number aberrations can be detected, e.g., amplifications or deletions for a particular region. For instance, a microdeletion or a microamplification of a few Mb can be detected. Such sequence imbalances occur between the two haplotypes, e.g., a duplicated haplotype causes it to be overrepresented or a deletion in a haplotype causes it to be underrepresented.

*C. Determination of fetal genotype*

Given that short-preferred end positions can correlate to a particular tissue type, cell-free DNA molecules ending at such preferred ending positions have high likelihood of being from that tissue (e.g., fetal, cancer, or transplant). In some situations, a particular tissue type in a cell-free DNA mixture can have a different genotype at a particular genomic position relative to other tissue types. For example, fetal tissue or tumor tissue can have a different genotype. As the cell-free DNA molecules ending at a short-preferred site have a high likelihood of being from the tissue type of interest, the cell-free DNA molecule ending at such a position can be analyzed to determine a genotype of the tissue type at that position. In this manner, the size-preferred ending position can be used as a filter to identify DNA from the tissue type.

The information regarding the size-preferred ending positions of the cell-free DNA fragments (e.g., sequenced from plasma) can be used for determining which maternal allele has been inherited by the fetus from the pregnant woman. Here, we use a hypothetical example to illustrate the principle of this method. We assume that the genotypes of the mother, the father and the fetus are AT, TT and TT, respectively. To determine the fetal genotype, we need to determine if the fetus has inherited the A or the T allele from the mother. We have previously described a method called relative mutation dosage (RMD) analysis (Lun et al. Proc Natl Acad Sci USA 2008;105:19920-5). In this method, the dosage of the two maternal alleles in the maternal plasma would be compared. If the fetus has inherited the maternal T allele, the fetus would be homozygous for the T allele. In this scenario, the T allele would be overrepresented in the maternal plasma compared with the A allele. On the other hand, if the fetus has inherited the A allele from the mother, the genotype of the fetus would be AT. In this scenario, the A and T alleles would be present in approximately the same dosage in the maternal plasma because both the mother and the fetus would be heterozygous for AT. Thus, in RMD analysis, the relative dosage of the two maternal alleles in the maternal plasma would be compared.

The ending positions of the reads can be analyzed for improving the accuracy of the RMD approach. For example, the reads can be filtered to include only those that end at a short-preferred site and cover the position that is being genotyped.

In an illustrative example, two molecules ending on a short-preferred ending position carry the T allele (e.g., at the preferred ending position or at a nearby position that is covered by the two corresponding reads). In one embodiment, when only the two molecules ending on the short-preferred ending position were used for downstream analysis, the fetal genotype would be deduced as TT. Thus, a sequence imbalance of only T-associated reads (or a high percentage, e.g., greater than 70%) can indicate a homogenous genotype. A sequence balance (e.g., less than 60% for either allele) can indicate a heterozygous genotype.

In another embodiment, the two fetally-derived molecules carrying the T allele would be given a higher weight in the RMD analysis because these two molecules ended on a short-preferred ending position. Different weight can be given to the molecules ending on the short-preferred ending positions, for example but not limited to 1.1, 1.2, 1.3, 1.4, 1.5, 2, 2.5, 3 and 3.5.

As an example, the criteria for determining whether a locus is heterozygous can be a threshold of two alleles each appearing in at least a predetermined percentage (e.g., 30% or 40%) of reads aligned to the locus. If one nucleotide appears at a sufficient percentage (e.g., 70% or greater) then the locus can be determined to be homozygous in the particular tissue.

A similar technique can be performed for a subject with a tumor. The cell-free DNA molecules ending on the short-preferred ending position can be identified and analyzed. The base corresponding (e.g., aligned) to this position (or a nearby test position covered by the DNA fragments) can be determined for each cell-free DNA molecule of this set, and the percentages of the total bases can be computed for each base. For example, a percentage of Cs at the test position seen on the cell-free DNA molecules ending at the position can be determined. If C is not seen in the healthy tissue of the subject, then C can be identified as a mutation if a sufficient number of Cs are identified, e.g., above a threshold number, which can depend on the measured tumor DNA fraction in the sample

*D. Size-tagged preferred ends in healthy subjects vs. pregnant subjects*

The above analysis suggested that the Set S preferred end sites indeed reflect the fragmentation pattern of the fetally-derived DNA. However, these end sites were mined from a mixture of fetal and maternal DNA molecules. Hence, to test whether these preferred end sites only reflected the fetal-specific fragmentation pattern, we retrieved a dataset containing 32 healthy (non-pregnant) subjects from a previous study from our group (28) and searched for plasma DNA reads carrying the Set S preferred end sites in these samples. Interestingly, some plasma DNA reads with Set S preferred end sites were indeed present in plasma of healthy subjects and such plasma DNA molecules were also shorter than those covering Set L preferred end sites.

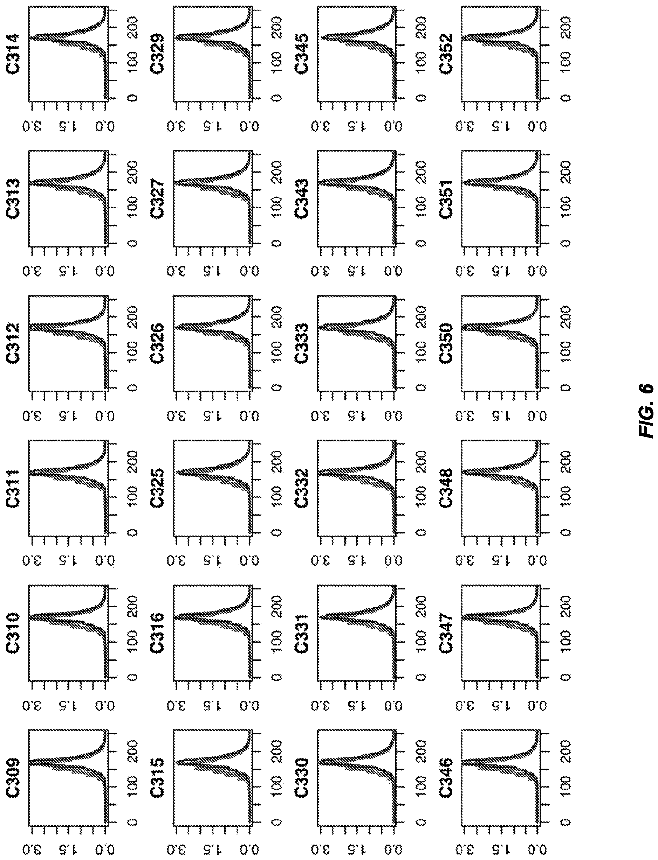

FIG. 6 shows size distributions of the plasma DNA reads covering Set S preferred end sites versus those covering Set L preferred end sites in 24 healthy subjects. Red and blue lines were reads covering Set S and Set L preferred end sites, respectively. The X-axis denotes the fragment size (bp) and Y-axis denotes the frequency (%). The cell-free DNA molecules ending on the set S preferred end sites were shorter on average than those ending on set L.

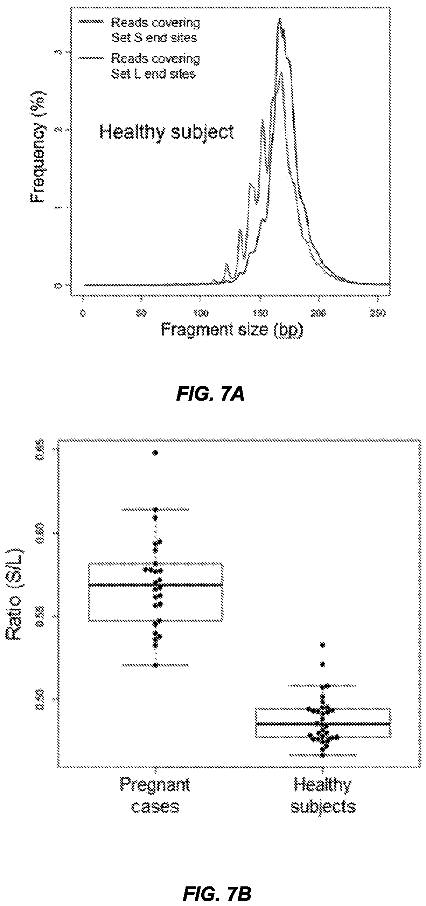

FIG. 7A shows size distribution of the plasma DNA reads covering Set S and Set L preferred end sites in a healthy subject according to embodiments of the present disclosure. FIG. 7A shows a case with a typical size distribution.

FIG. 7B shows a comparison of the relative abundance of plasma DNA reads with Set S versus Set L preferred end sites (S/L ratio) in pregnant women and healthy subjects according to embodiments of the present disclosure These healthy subjects showed a lower S/L ratio compared to the pregnant women. Thus, the reads ending at set S have an increased proportion of fetal DNA relative to other sets of ending positions, e.g., set L or the entire genome.

This shows that S/L is viable for use in a parameter value for increased accuracy in the detection of a sequence imbalance, e.g., when normalized to S/L for one or more reference regions. More generally, the set S of ending positions can be used as a filter to use only certain identified DNA molecules, resulting in an enrichment in fetal DNA. The DNA molecules ending at set S within a region (enriched for fetal DNA) can be used to detect if there is a sequence imbalance for the fetal DNA. As examples, the parameter value may include a ratio of S/L of a test region and S/L of one or more reference regions, or just a ratio of first number of DNA molecules ending on short-preferred ends in a test region and a second number of DNA molecules ending on short-preferred ends in one or more reference regions.

The data thus suggested that the size-tagged preferred end sites were general footprints of short and long DNA molecules in the plasma, irrespective of their origin (e.g. fetal versus maternal). Furthermore, fetal DNA molecules showed a higher proportion of molecules covering the Set S preferred end sites compared to maternal DNA. Accordingly, a ratio of an S/L value for a test region and one or more reference regions can be used as a parameter value that is compared to a reference value to discriminate between classifications of a sequence imbalance.

## IV. TUMOR USE OF SIZE-TAGGED PREFERRED END SITES

Similar measurements can be performed for samples including tumor DNA, as shown by the following data. For example, a proportional contribution of tumor DNA in a cell-free sample can be determined, or a sequence imbalance can be determined.

### A. Fragmentation of tumor DNA

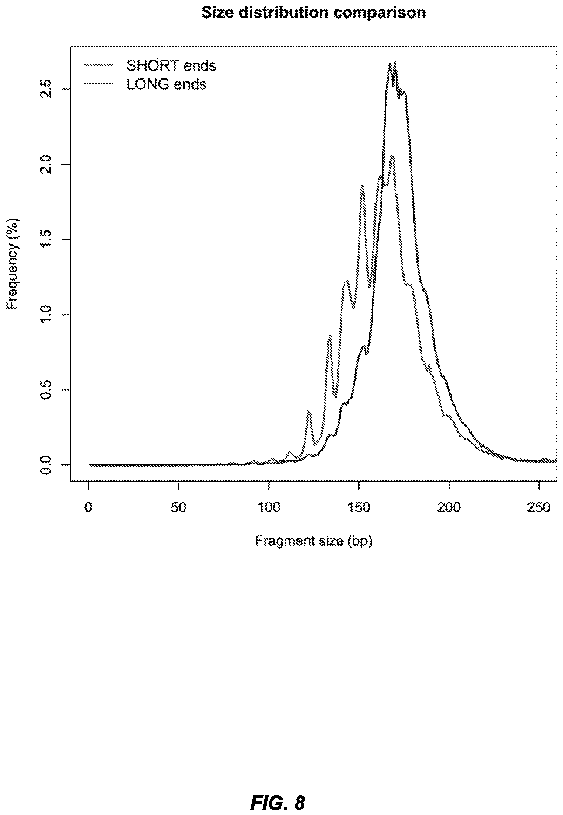

FIG. 8 shows a size distribution of the plasma DNA reads covering Set S and Set L preferred end sites in a hepatocellular carcinoma (HCC) patient according to embodiments of the present disclosure. The X-axis denotes the fragment size (bp) and Y-axis denotes the frequency (%). FIG. 8 shows a case with a typical size distribution. Although HCC is used as a test case, other cancers also exhibit short cell-free DNA fragments, and thus the technique is equally applicable to other types of cancer.

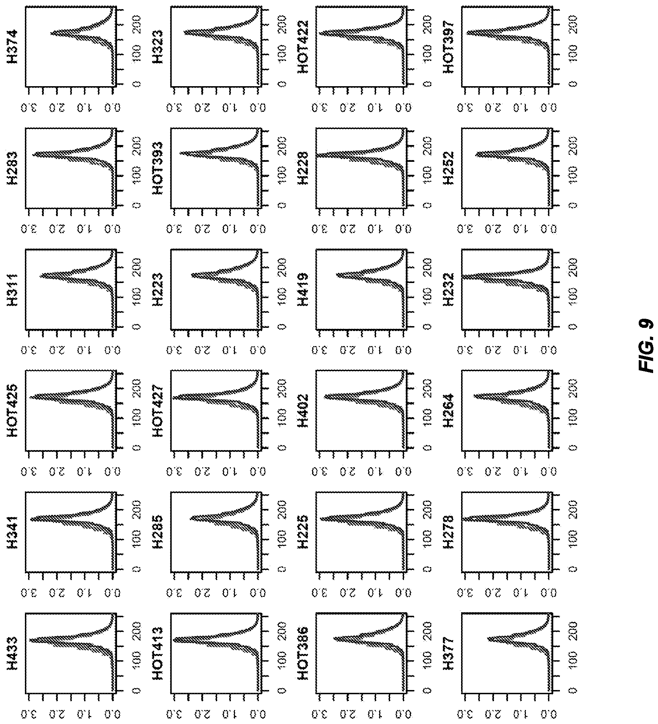

FIG. 9 shows size distributions of the plasma DNA reads covering Set S preferred end sites versus those covering Set L preferred end sites in a representative set of 24 hepatocellular carcinoma patients. Red and blue lines were reads covering Set S and Set L preferred end sites, respectively. The X-axis denotes the fragment size (bp) and Y-axis denotes the frequency (%). Overall, 90 HCC patients were analyzed, with the 90 patients having similar size distributions as shown in FIG. 9.

### B. Determining tumor fraction

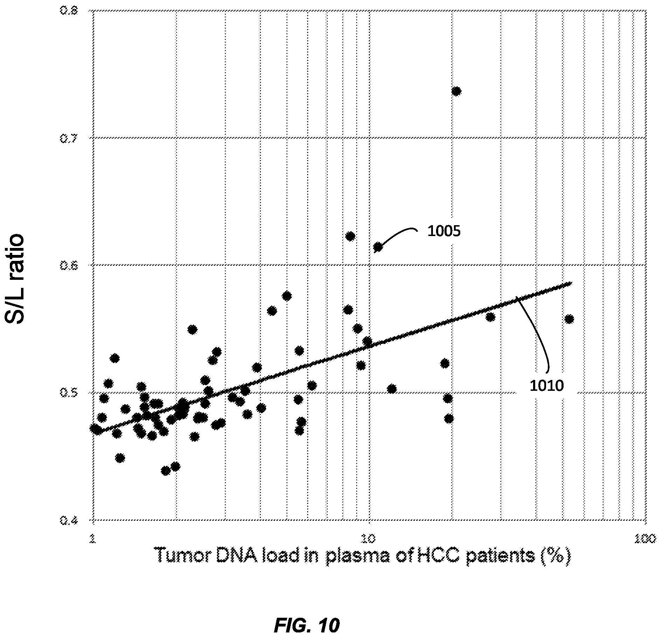

FIG. 10 shows the correlation between the relative abundance (S/L ratio) of plasma DNA molecules with size-tagged preferred end sites and tumor DNA fraction in 72 hepatocellular carcinoma patients with tumor DNA fraction higher than 1% in the plasma according to embodiments of the present disclosure. The same set S and set L sites as from FIG. 1 are used. A positive correlation was observed between the relative abundance of plasma DNA with Set S versus Set L preferred end sites [denoted as S/L ratio] and the tumor DNA fraction ($R = 0.58$, $P < 0.001$, Pearson correlation).

FIG. 10 shows similar behavior as FIG. 4A. For example, the relative abundance can be determined by counting a first number of cell-free DNA molecules that end at one of the set S sites and dividing by a second number of cell-free DNA molecules that end at one of the set L sites. Each calibration data point 1005 corresponds to a difference sample whose relative abundance and tumor DNA fraction was determined. The tumor DNA fraction can be determined using a tumor-specific marker, e.g., a tumor-specific allele, such as a loss of heterozygosity (LOH).

As with the fetal measurement, to determine a tumor DNA fraction for a new sample, a system can determine the relative abundance of cell-free DNA molecules ending at a set of short-preferred end positions compared to other cell-free DNA molecules (e.g., ones ending at a set of long-preferred end positions). Then, the newly measured relative abundance can be compared to one or more of the calibration data points 1005. For example, a calibration function 1010 can be fit to the calibration data points 1005, where the newly measured relative abundance can be used as an input to the calibration function 1010, which provides an output of a tumor DNA fraction.

The classification of the proportional contribution of a tissue type (e.g., tumor tissue) can correspond to values other than a percentage or range of percentages. For example, the classification can correspond to a detection of cancer, and more particularly to a tumor load.

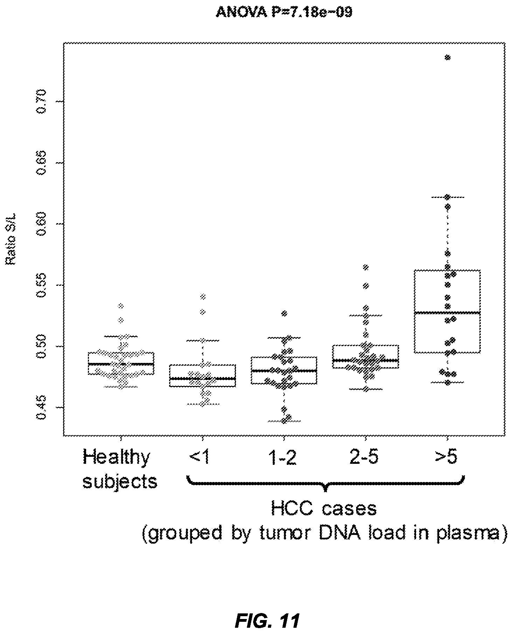

FIG. 11 shows the relative abundance (S/L ratio) of plasma DNA molecules with size-tagged preferred end sites among healthy subjects and hepatocellular carcinoma patients. The hepatocellular carcinoma patients are divided into 4 groups based on the tumor DNA fractions in the plasma. The higher the S/L ratio the higher the tumor load is. The 4 groups correspond to different ranges of percentages of tumor DNA fraction. The drop in the <1 group is due to a small tumor such that the longer DNA in surrounding necrotic tissue outweighs the short DNA from the tumor.

Accordingly, the classification can whether any tumor tissue is measured, or at least an appreciable amount (e.g., above a minimum threshold for detection). Thus, a classification of a proportional contribution can be that cancer is detected. Depending on the sensitivity or specificity, embodiments could use a detection threshold of about 0.5, 0.51, 0.52, or 0.53, as examples.

Other values for the relative abundance (besides ratio S/L) can be used, e.g., as described above for determining the fetal fraction. For instance, the normalization can use a total number of reads obtained, which would include reads ending at positions outside of any short-preferred windows. Such a total number is an example of a second number of reads that include reads not ending on a short-preferred position. Analyzing a same number of reads from one sample to another sample provides a same result as normalizing by a total number of reads or other second number, and thus is included by such normalization.

*C. Detecting sequence imbalance resulting from tumor*

A sequence imbalance can also be detected in a chromosomal region of tumor tissue. For example, amplifications and deletions typically occur in tumor tissue. Thus, a sequence imbalance would occur and cause one haplotype to be overrepresented relative to another haplotype. Such copy number aberrations can be tested in a plurality of regions (e.g., all the same size, such as 1 Mb) in differently sized regions, such as chromosomal arms.

In the examples below, for detection of a sequence imbalance in a cell-free sample from a subject with a tumor, chromosomal region 1p, 1q, 8p and 8q are investigated as they are known to frequently suffer from CNA in HCC. A first number of cell-free DNA molecules ending at short-preferred positions in one of these regions can be used as a parameter value for detecting a sequence imbalance in the region. A second number of cell-free DNA molecules ending at short-preferred positions in one or more reference regions may be used to normalize the first number, e.g., so that the size of the sample can be accounted for. The one or more second regions can be known or presumed to not have a sequence imbalance.

In the examples below, the one or more reference regions includes all of the autosomes, and thus all of the DNA fragments that end at a short-preferred sites in the autosomes. Accordingly, all autosomes are combined to serve as the control to normalize the count of reads that end at one of the set S positions. The normalized count of DNA molecules ending at a particular set of positions (e.g., set S) can be compared to a reference value (e.g., an expected value when no sequence imbalance exists), which may include comparing to a cutoff value to determine if a statistically significant deviation exists from the reference value.

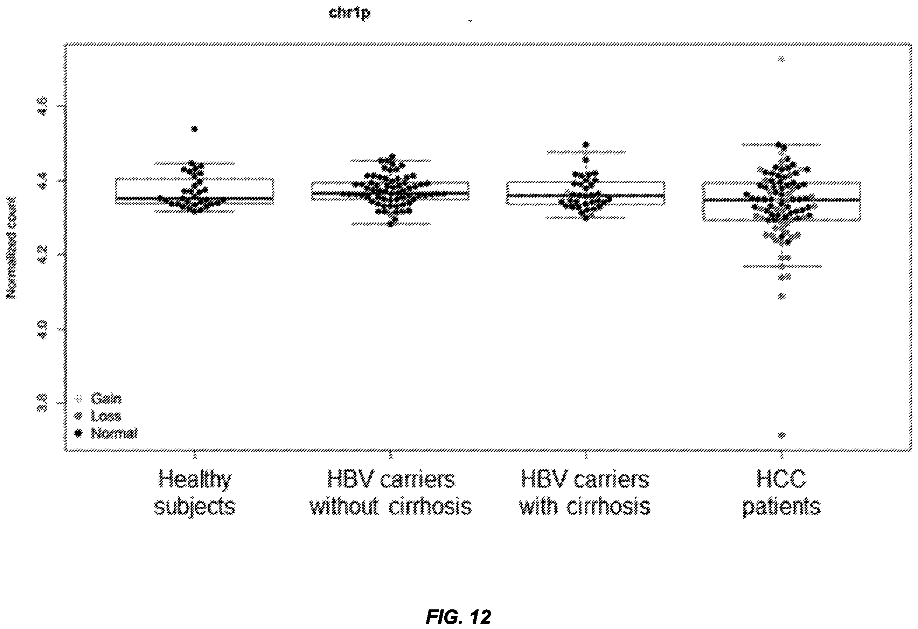

FIG. 12 shows the normalized read count covering the Set S ends on chr1p among healthy subjects, HBV carriers with or without cirrhosis, and HCC patients according to embodiments of the present disclosure. FIG. 12 shows box plots for each class of subject, with a median shown as a bar and the upper and lower quartiles as the whiskers. Each data point corresponds to the normalized read count for the chr1p region for a given sample, where the sample is in one of the four classes. The normalized read count can be determined as the number of reads having an end position at one of the set S ends in the chr1p region divided by a total number of reads having an end position at one of the set S ends.

The copy number aberration information is also incorporated, as certain samples are marked as exhibiting a gain (amplification), loss (deletion), or as normal. In general, one expects relatively few aberrations in non-cancer subjects, although there a few in the HBV subjects with cirrhosis, which may be a precursor for HCC. As shown, the regions with a copy number loss generally have values lower than the median. A sufficient deviation from the median or a particular percentage value away can be used as a threshold or reference value to determine a sequence imbalance exists for the region. The determination of gains and losses for the regions is determined using (28).

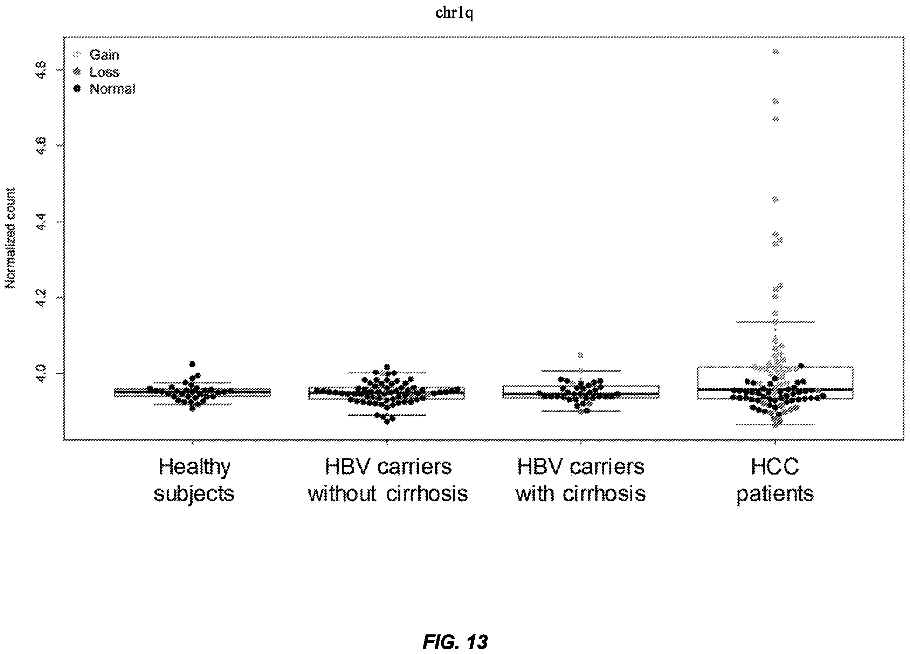

FIG. 13 shows the normalized read count covering the Set S ends on chr1q among healthy subjects, HBV carriers with or without cirrhosis, and HCC patients according to embodiments of the present disclosure. The copy number aberration information (gain, loss, or normal) is also incorporated. FIG. 13 shows similar plots as FIG. 12, but with a copy number gain being predominant aberration for chr1q, as opposed to a loss that is predominant for chr1p.

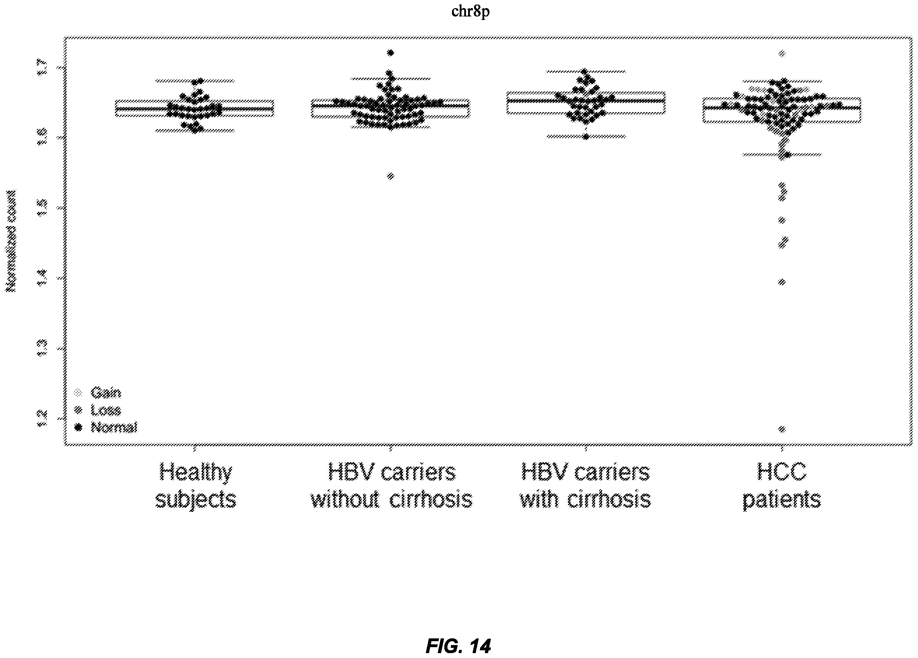

FIG. 14 shows the normalized read count covering the Set S ends on chr8p among healthy subjects, HBV carriers with or without cirrhosis, and HCC patients according to embodiments of the present disclosure. The copy number aberration information is also incorporated. FIG. 14 shows similar plots as FIG. 12, with a copy number loss being predominant aberration for chr8p.

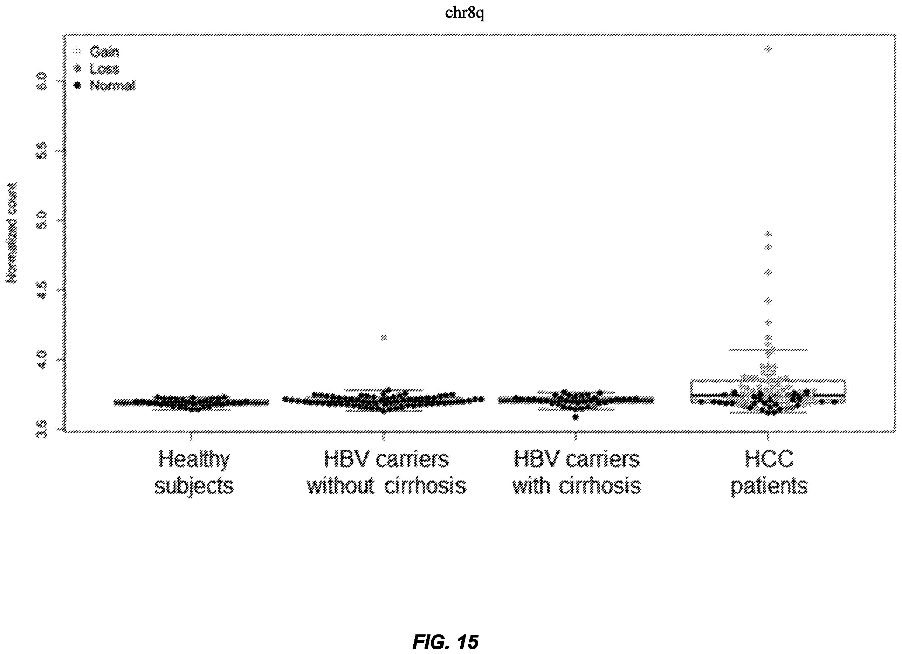

FIG. 15 shows the normalized read count covering the Set S ends on chr8q among healthy subjects, HBV carriers with or without cirrhosis, and HCC patients according to embodiments of the present disclosure. The copy number aberration information is also incorporated. FIG. 15 shows similar plots as FIG. 12, but with a copy number gain being predominant aberration for chr8q, as opposed to a loss that is predominant for chr1p.

As described in section III.C, the sequence imbalance may involve determining a genotype of the tissue. A group of DNA molecules ending on a short-preferred site can be identified, for example, as generally corresponding to tumor DNA fragments. The alleles at a given locus covered by the DNA fragments of the identified group can be analyzed to determine the genotype at the locus. For instance, a difference or ratio can be determined between a first number of DNA fragments in the group that have a first allele and a second number of DNA fragments in the group that have a second allele. The difference or ratio are examples of a value of the identified group of cell-free DNA molecules. The value can be compared to a reference value to determine whether a sequence imbalance exists, e.g., the genotype being heterozygous for the two alleles in the tumor tissue if a sequence imbalance does not exist and the genotype being homozygous for the predominant allele (possibly only allele in the group) when a sequence imbalance does exist.

## V. LOCATION OF ENDING SITES IN CHROMATIN

*A. Genomic annotation of the size-tagged preferred end sites.*

To explore how the size-tagged preferred end sites were generated in the genome, we investigated the separation (in bp) between any two closest preferred end sites in Set S and Set L, respectively.

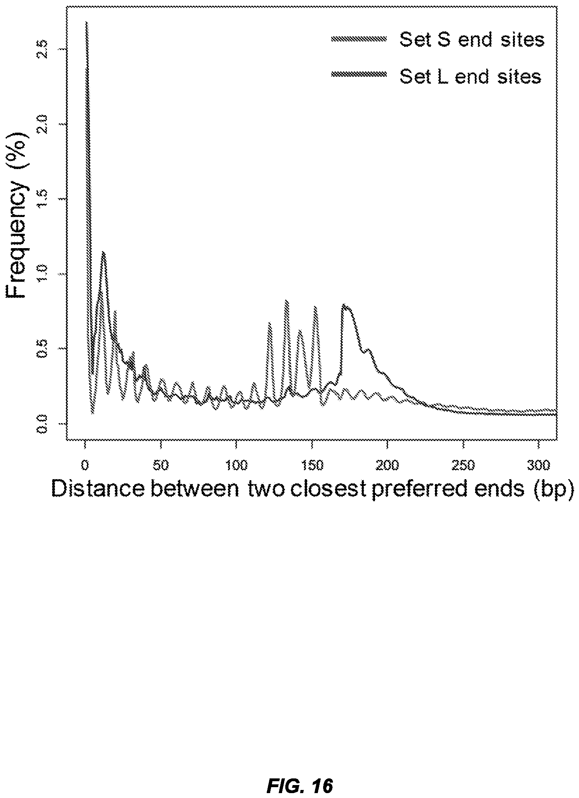

FIG. 16 shows a distribution of the distance between any two closest preferred end sites in Set S and Set L preferred end sites according to embodiments of the present disclosure. The distance is between the closest S sites for the set S data, and the distance between the closest set L sites for the set L data. For Set S preferred end sites, there was a strong 10-bp periodicity up to ~150 bp. On the other hand, for Set L preferred end sites, there was one peak at ~170 bp while no 10-bp periodicity was observed. This pattern of separation was thus highly consistent with the size characteristics of plasma DNA and the nucleosomal structure, suggesting that the Set S preferred end sites might be located within the nucleosome core while the Set L preferred ends might be located in the linker region.

To explore this hypothesis, we investigated the distribution of size-tagged preferred end sites around regions with well-positioned nucleosomes. Specifically, we investigated the preferred ends profile in chr12p11.1, a region known to have well-positioned nucleosomes in almost all tissue types (29, 30).

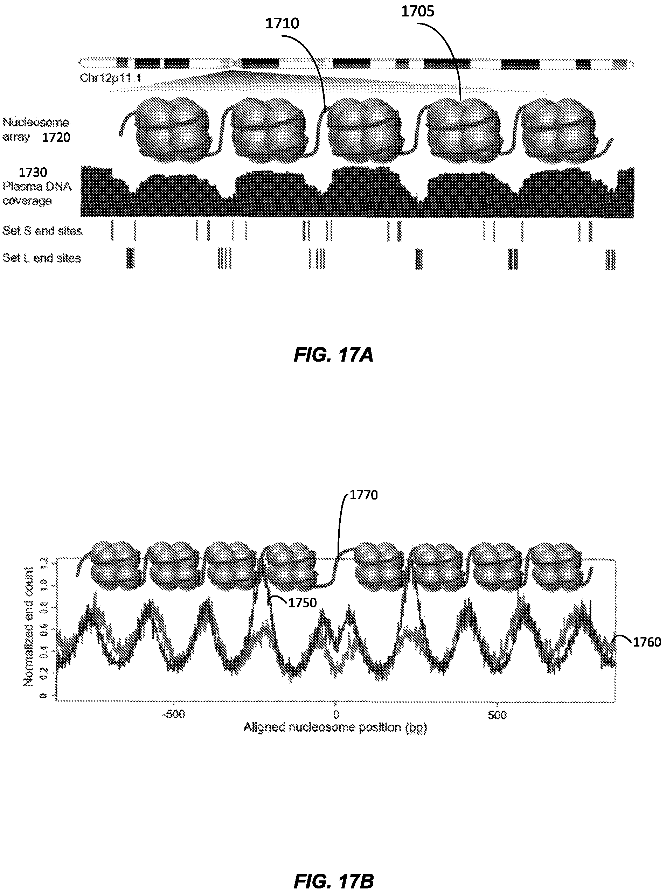

FIG. 17A shows a snapshot of the plasma DNA coverage, Set S, and Set L preferred end sites according to embodiments of the present disclosure. An illustration of the nucleosome arrays on chr12p11.1 region is shown. The nucleosome array 1720 is shown with nucleosome cores 1705 and linker regions 1710. The DNA coverage 1730 shows a number of reads covering each genomic position, with the horizontal axis corresponding to genomic position. As shown in FIG. 17A, the Set L preferred ends were mostly located in the linker regions 1710 while the Set S preferred ends were mostly located within the nucleosome core 1705, even if on the edges of the core.

In addition, since the nucleosomes around the open chromatin regions (e.g., promoters and enhancers) were also known to be well-positioned (30), we investigated the localizations of the preferred end sites around the open chromatin regions. Fetal and maternal DNA molecules in maternal plasma are known to be mostly originated from the placental tissue and the hematopoietic system, respectively (12, 31). To this end, we downloaded DNaseI hypersensitivity profiles for placental and selected hematopoietic tissues from the RoadMap Epigenomics project (32). Of note, DNaseI profiles for neutrophils are not available. We used the T-cell profile as being representative of other hematopoietic cells because the RoadMap project revealed that the epigenomic profiles were similar between several hematopoietic cell lineages (i.e., T-cells, B-cells, natural killer cells, monocytes, neutrophils and hematopoietic stem cells) (32). We determined the size-tagged preferred end sites around the open chromatin regions shared by the placenta and T-cells and termed these the common open chromatin regions.

FIG. 17B shows a distribution of the preferred end sites surrounding the common open chromatin regions shared by placental tissues and T-cells according to embodiments of the present disclosure. An illustration of the nucleosome positions is shown. As the data is for all common open chromatin regions, the number of preferred ending sites is much more than FIG. 17A and a distribution pattern can be seen.

The aligned nucleosome positions as plotted on the X-axis are in relation to the center of the common open chromatin regions represented as region 1770. The normalized end count for long-preferred sites is shown as 1750 and for short-preferred sites is shown as 1760. In FIG. 17B, the end count at a position is normalized by a total number of short and long preferred sites existing within the common open chromatin region, i.e., within the genomic coordinates shown in FIG. 17B. Thus, the two datasets 1750 and 1760 are normalized in the same manner.

As shown in FIG. 17B, a periodicity pattern of ~190 bp could be observed between the peaks of either dataset, which was consistent with the nucleosomal phasing pattern and represented the distance between nucleosomes (29). Moreover, the preferred end sites were less abundant in the center of the open chromatin regions. It has been reported that there is frequent occupancy of transcription factor binding in the open chromatin regions (33) and thus possibly preventing DNA cutting. In addition, the peaks for Set S and Set L preferred end sites were not located at the same position. These peaks were separated by ~25 bp that was about the size of the linker region. Together, these data suggested that the locations of size-tagged preferred end sites were closely related with the nucleosomal structure. Thus, the positions of the plasma DNA end sites are related with the nucleosomal structure. The high peaks just after the first nucleosome after the open chromatin region is due to the two nucleosomes surrounding the open chromatin regions being more strictly well-phased than the ones nearby, which makes the preferred ends more predictable in their linkers (i.e., the peaks are higher).

To further validate the relationship of the size-tagged preferred end sites and the nucleosome structure in a genomewide manner, we downloaded the annotated "nucleosome track" from Snyder et al. (24), which contained the location of ~13M nucleosome centers (i.e., the loci with maximum nucleosome protection) deduced using a computational approach for all tissues. For both Set S and Set L preferred end sites, we correlated each preferred end site to its nearest nucleosome center. We then profiled the distribution of the distances of the preferred end sites to the nucleosome centers.

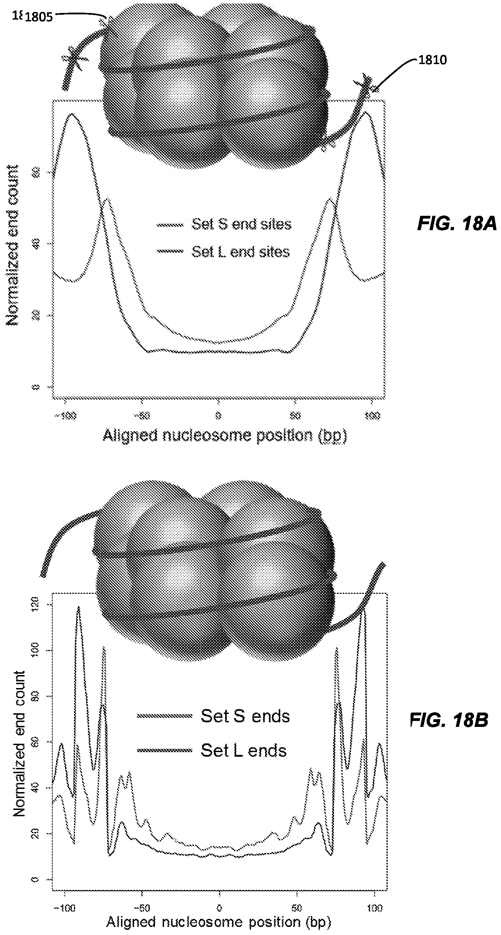

FIG. 18A shows a distribution of the size-tagged preferred end sites in pregnant plasma DNA relative to the nucleosome structure according to embodiments of the present disclosure. The horizontal axis is the genomic position relative to a nucleosome center, and the vertical axis is the normalized end count of the two categories of size-tagged preferred ends, where each set of values is normalized separately using their respective total numbers of size-preferred ending sites.

The red scissors 1805 and blue scissors 1810 represent cutting events that would generate Set S and Set L preferred end sites, respectively. As shown in FIG. 18A, the Set S and Set L preferred end sites showed major peaks at ±73 bp and ±95 bp, respectively, which fitted the size of DNA wrapping the nucleosome core and nucleosome spacing pattern in the genome. Annotation using another computationally deduced nucleosome track by Straver et al. (23) showed similar results.

FIG. 18B shows a distribution of the size-tagged preferred end sites relative to the nucleosome centers predicted by Straver et al (23) according to embodiments of the present disclosure. The aligned nucleosome positions as plotted on the X-axis are in relation to the nucleosome center. The data were consistent with FIG. 16 and demonstrated that the Set S preferred end sites were located within the nucleosome core while the Set L preferred end sites were located in the linker region. FIG. 18B differs from FIG. 18A in that another nucleosome positions from an independent group was used for confirmation of the result in FIG. 18A.

In addition, we also studied the fragment ends for all autosomes in the healthy subjects.

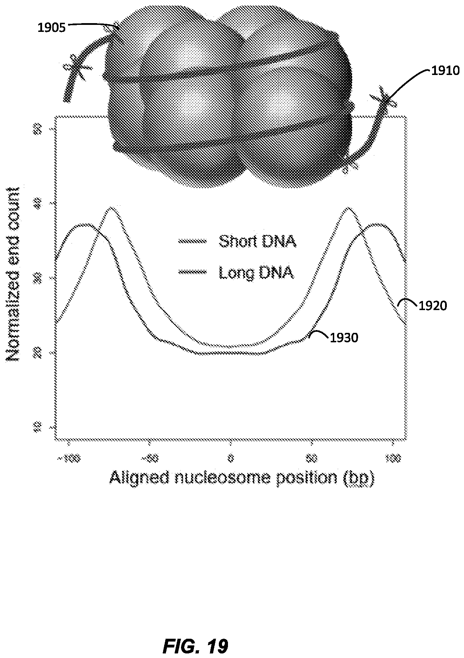

FIG. 19 shows the distribution of autosomal fragment ends for short and long DNA molecules in relation to the nucleosome structure in healthy non-pregnant subjects according to embodiments of the present disclosure. The red 1905 and blue 1910 scissors represent cutting events that would generate short and long fragments, respectively. The aligned nucleosome positions as plotted on the X-axis are in relation to the nucleosome center (23).

The normalized end count is the number of DNA fragments ending at a particular position, e.g., number of short DNA fragments 1920 and number of long DNA fragments 1930, divided by the overall read number of the corresponding size category. The peaks for short DNA occurred at ±73 bp and for long DNA occurred at ±95 bp, respectively. The short DNA fragments corresponded to 60-155 bases, and the long DNA fragments corresponded to 170-250 bases.

As shown in FIG. 19, the short DNA molecules showed a similar distribution to the Set S preferred ends and the long DNA molecules showed a similar distribution to the Set L preferred ends. The data thus suggested that in the healthy subjects, the short DNA molecules were mostly cut within the nucleosome core while the long DNA molecules were mostly cut within the linker region.

*B. Characteristics of fetal- and maternal-specific end sites.*

Considering that both Set S and Set L preferred end sites were mined from a mixture of fetal and maternal DNA, we further investigated the nucleosomal localization of fetal- and maternal-specific preferred end sites from our previous study (21). These preferred end sites were mined from DNA molecules in maternal plasma carrying fetal-specific and maternal-specific SNP alleles. Thus, an analysis of the fetal-specific, maternal-specific plasma DNA end sites and chrY fragment end sites was performed.

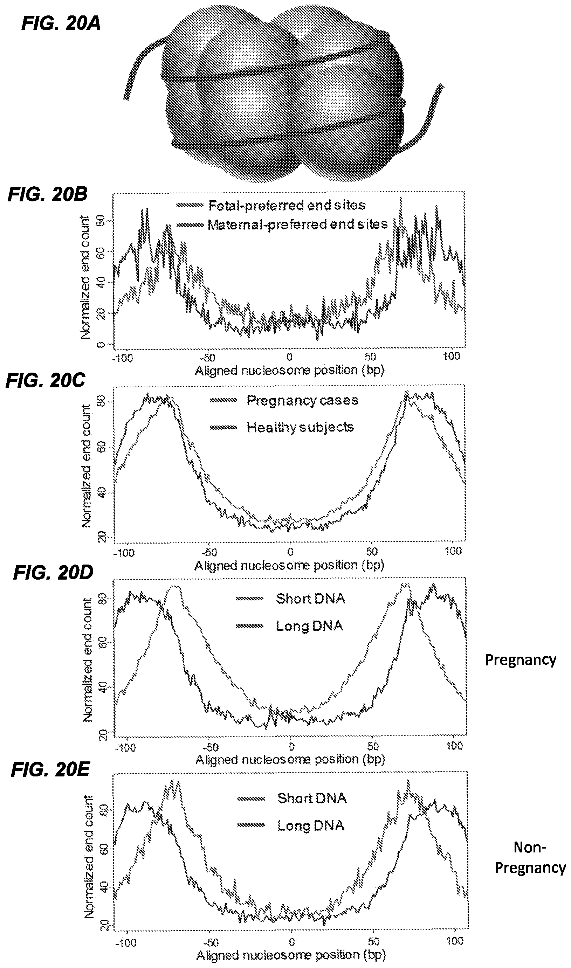

FIG. 20A shows an illustration of the nucleosomal structure. FIG. 20B shows a distribution of fetal- and maternal-specific preferred end sites in the nucleosome structure. FIG. 20C shows a distribution of the chrY fragment ends of pregnant cases and healthy male subjects in the nucleosome structure. FIG. 20D shows the distribution of chrY fragment ends for short and long DNA molecules in the nucleosome structure in pregnant cases. FIG. 20E shows the distribution of chrY fragment ends for short and long DNA molecules in the nucleosome structure in healthy subjects.

The aligned nucleosome positions as plotted on the X-axis are in relation to the nucleosome center (23). The vertical axis is the normalized end count. Each plot shows two sets of data, with the normalized end or read count provided for each dataset.

As shown in FIG. 20B , fetal-specific preferred end sites were mostly located within the nucleosome core while the maternal-specific end sites were mostly located in the linker region. These fetal- and maternal-specific preferred ends were mined in a previous study using fetal- and maternal-specific SNP sites (55). This is similar to short-preferred end sites mostly located within the nucleosome core (as shown in FIG. 18A) and long-preferred end sites located in the linker region. The normalized end count corresponds to the number of position divided by the total number of for a given group. Thus, the two groups (fetal-preferred and maternal-preferred) are normalized separately.

In the plasma of pregnant women carrying male fetuses, chrY reads were of fetal-origin. On the other hand, in healthy male subjects, chrY reads were mainly originated from the hematopoietic system. End sites for all the chrY reads were studied in the plasma of pregnant women carrying male fetuses and in the plasma of healthy males.

FIG. 20C shows the overall end site distribution. The normalized end count corresponds to a number of cell-free DNA fragments in a sample ending at a position relative to a nucleosome center, with the normalization being based on a total number of DNA fragments analyzed in the sample. Similar to the observations derived from FIG. 20B, chrY molecules in the pregnant samples showed more end sites locating within the nucleosomal cores while chrY molecules in the plasma of healthy male subjects showed more end sites beyond the nucleosome cores.

We further split the chrY reads in both pregnant women and healthy male subjects into short and long categories.

FIGS. 20D and 20E show the distributions of end sites in pregnant cases and healthy subjects, respectively. Interestingly, the short DNA molecules in both the pregnant and non-pregnant samples showed similar nucleosomal localization for their end sites. This observation suggested the possibility of similar mechanisms being operative in the generation of such short DNA molecules. Analogously, the long DNA molecules in both the pregnant and non-pregnant samples also showed similar nucleosomal localization for their end sites, and hence probably shared similar mechanisms in their production. On the other hand, the preference in generating short and long DNA molecules appeared to be different in fetal- and maternal-derived DNA.

In summary, in the context of pregnancy, fetal DNA was frequently cut within the nucleosome cores (i.e., Set S preferred end sites), and maternal DNA was mostly cut within the linker regions (i.e., Set L preferred end sites).

*C. Nucleosome accessibility in placental and hematopoietic cells.*

We wondered why the fetal DNA was frequently cut within the nucleosome cores. In somatic tissues, it was more difficult for endonuclease enzymes to cut DNA within the nucleosome cores than the linker regions as DNA within nucleosome cores was bound by histones (34). We therefore hypothesized that placental cells were different from somatic tissues in that the DNA within the nucleosome core was more accessible and hence could be cut more easily.

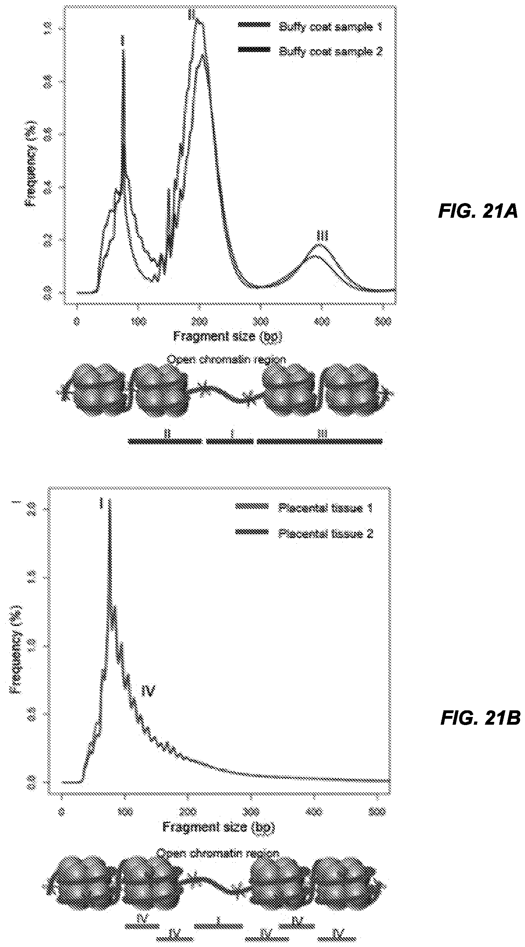

To test this hypothesis, ATAC-seq (Assay for Transposase-Accessible Chromatin using sequencing) experiments (35), which had been utilized to explore the nucleosome accessibility (36), were conducted on two placental tissue samples (one syncytiotrophoblast sample and one cytotrophoblast sample) and two maternal buffy coat samples. ATAC-seq experiments take advantage of the transposase enzyme that cuts nucleosome-free DNA to study the open chromatin regions and the nucleosome positioning nearby (35). The DNA insert size pattern in previously conducted ATAC-seq experiments (35, 37, 38) on somatic tissues showed a strong periodicity pattern of approximately 200 bp. This pattern suggested that the open chromatin regions were separated by 200-bp regions and likely to be bound by intact nucleosomes (35). The insert size distributions for our ATAC-seq experiments are shown in FIGS. 21A and 21B.

FIGS. 21A and 21B show fragment size distribution from ATAC-seq data of (A) buffy coat samples and (B) placental tissues. The size of the DNA fragments generated from transposase cutting are measured, and then a frequency histogram is determined. Different sections of the chromatin structure are labeled for each of FIGS. 21A and 21B.

In the buffy coat samples, the transposase enzyme mostly cut the non-nucleosome bound DNA (e.g., linker region). As a contrast, the transposase enzyme was able to cut within the nucleosomes in the placental tissues, indicating that the nucleosome packaging in the placental tissues was not as tight as that in the buffycoat samples. Blue and red scissors indicated possible cutting event in buffy coat samples and placental tissues, respectively.

The insert size distributions for buffy coat samples (FIG. 21A) were similar to those observed in previous studies (35, 37, 38). Peaks at ~200 and ~400 bp in the size profiles are DNA protected by integer multiples of nucleosomes (37), suggesting that the transpose enzyme mostly cut the non-nucleosomal bound DNA (e.g., linker region) in the buffy coat samples. On the other hand, placental tissue samples showed a drastically altered size distribution in that the peak around 200 bp was absent (FIG. 21B). Instead, the ATAC-seq insert distributions for the placental samples showed much shorter DNA distribution, suggesting that the transposase enzyme was able to cut within the nucleosomes thus indicating that the nucleosome packaging in the placental tissues was not as tight as that in the buffycoat samples. As a result, the data showed that placental DNA was associated with more accessible chromatin than the buffy coat DNA.

## VI. TECHNIQUES USING SIZE-TAGGED ENDING POSITIONS

As described above, various embodiments can use short-preferred ending positions to determine a proportional contribution of DNA fraction from a particular tissue type (e.g., tumor, transplant, or fetal tissue) that is associated with short cell-free DNA fragments. Various embodiments can also determine whether a sequence imbalance exists for the first tissue type. The first tissue type (e.g., tumor, transplant, or fetal tissue) can be identified based on the specific subject. For example, if the subject previously had liver cancer, then screening can be performed to check whether the liver cancer has returned, which would result in an increase in the proportional contribution from tumor tissue. As another example, if the subject is a pregnant female, then the first tissue type can be fetal tissue. Such a selection criteria applies to other methods described herein.

*A. Summary of example results for size-tagged preferred ends*

We performed integrative analysis of size profiling and preferred DNA end sites in plasma DNA. Compared to using genotype information to deduce fetal- and maternal-specific preferred end sites, the size-tagged approach described here allowed us to mine size-preferred end sites that enabled an improved estimation of fetal DNA fraction in plasma DNA. For estimating the fetal DNA fraction, such size-tagged preferred end sites also showed a better performance than using the size profiling alone (17), as shown in FIGS. 4A and 4B. Moreover, we showed that the reads covering the size-tagged preferred end sites provided an improved performance in noninvasive prenatal testing of trisomy 21 over using random reads (FIG. 5B). These data opened up the possibility for developing targeted approaches to specifically enrich for plasma DNA molecules with the size-tagged preferred end sites. Such an enrichment approach would potentially reduce the sequencing depth requirement for noninvasive fetal aneuploidy detection.

In addition, we correlated locations of the size-tagged preferred end sites in the context of nucleosomal structure, e.g., as shown in FIG. 17A. We found that the Set S preferred end sites were located within the nucleosome core while the Set L preferred end sites were located in the linker region. Interestingly, we found that for all the pregnant women and healthy non-pregnant subjects investigated, the reads covering Set S preferred end sites were shorter than those covering Set L preferred end sites, as shown in FIGS. 2, 3, 6, and 7A. This observation suggested that the Set S and Set L preferred end sites were associated with short and long plasma DNA molecules, irrespective of their tissue of origin, since the association also existed in healthy non-pregnant subjects.

Further analysis on chrY reads from plasma of pregnant women showed consistent results. Even though the relative shortness of fetal DNA in maternal plasma was first reported in 2004 (14), the mechanistic explanation to this phenomenon is still unsolved. Here, we have proposed a theory that the nucleosome accessibility in placental tissue is higher than the maternal somatic tissues (e.g., blood cells) thereby allowing the endonuclease enzymes to cut within the nucleosome cores during cell death processes (e.g. apoptosis). Our ATAC-seq experiments showed that indeed the nucleosome cores were more readily accessed by the transposase enzyme in placental cells compared to blood cells, as shown in FIGS. 21A and 21B. While the molecular basis of this accessibility is still unclear, we propose that DNA methylation could be one contributing factor. In the human genome, DNA methylation profile shows a 10-bp periodicity over the nucleosome-bound DNA, which coincides with the size pattern of the plasma DNA (39).

In fact, we and others had demonstrated that the fragment size of plasma DNA was positively correlated with DNA methylation level (40, 41). In addition, during pregnancy, the DNA methylation of the placental genome increases progressively and the fragment size of the fetally derived DNA in maternal plasma also increases with gestational age (42). All these studies suggested that DNA methylation may affect the fragmentation process and perhaps by altering chromatin accessibility. Compared to somatic tissues, placental tissues are known to exhibit genomewide hypomethylation (43). Previous studies had demonstrated that DNA methylation could induce a tighter wrapping of DNA around the accompanied histones (44) and increase the nucleosome compaction, rigidity and stability (45, 46). Furthermore, DNA methylation could also regulate histone modifications as well as heterochromatin formation (47, 48), which was correlated with nucleosome unwrapping, disassembly and stability (49). All these studies suggested that the higher nucleosome accessibility in placental tissues might be linked to its hypomethylation.

While we used circulating cell-free fetal DNA and DNA from placental tissues to gain mechanistic insights into fetal DNA fragmentation, the concept is applicable to cell-free DNA of non-fetal origin. The preferred end sites in short and long DNA molecules in plasma of non-pregnant individuals demonstrated the same localization patterns with respect to the nucleosome structure, e.g., as shown in FIGS. 20D and 20E. These data suggest that a similar set of mechanisms might contribute to the liberation of short or long DNA molecules into the plasma of pregnant and non-pregnant individuals. However, the ratio of short to long DNA molecules is higher in pregnant samples than in the plasma from non-pregnant individuals, as shown in FIG. 7B. Furthermore, there are notable similarities between the plasma DNA profiles of cancer patients and pregnant women. Hence, tumor-derived DNA molecules in plasma are shorter (28) and the tumoral genome also exhibit genomewide hypomethylation (50, 51). We therefore think that the shortness of tumor-derived DNA may be due to an analogous mechanism (52). Thus, size-tagged end sites might are useful for noninvasive cancer testing, as described herein.

We have incorporated size characteristics in mining preferred end sites in cell-free DNA, and demonstrated the utility of such size-tagged sites in noninvasive prenatal and cancer testing. We further showed that the preferred ends were highly correlated with the nucleosomal structure, thus shedding mechanistic insight on the production mechanism of cell-free DNA and the relative shortness of fetal DNA in maternal plasma.

Further, we use short size and fragment end characteristics to enrich for the clinically relevant DNA molecules. Here, embodiments use these characteristics to identify the subset of cell-free DNA molecules that are relevant. Broad and deep sequencing is not needed for a test sample, and the broad and deep sequencing may only be needed to identify these characteristics from historical samples. Such enriched samples for clinically-relevant DNA (e.g., fetal, tumor, and transplant) can be used to detect sequence imbalance with higher accuracy.

### B. Determining fraction of DNA from particular tissue type

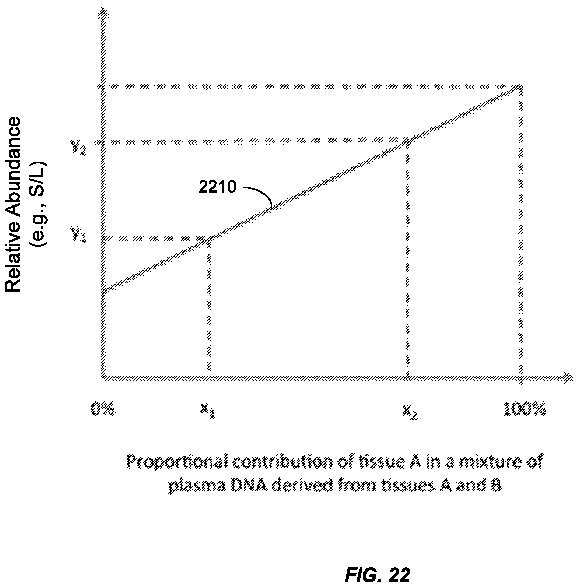

FIG. 22 shows the relationship between a relative abundance of cell-free DNA molecules ending on short-tagged ending positions (e.g., short/long) and the proportional contribution of tissue A to DNA in a mixture determined by analysis of two or more calibration samples with known proportional concentrations of DNA from tissue A. In the example shown, two samples with proportional contribution of tissue A of $x_1$ and $x_2$ are analyzed. The relative abundance values of the two samples were determined as $y_1$ and $y_2$, respectively. The relationship between relative abundance and the proportional contribution of A can be determined based on the values of $x_1$, $x_2$, $y_1$ and $y_2$. Various examples of a relative abundance for cell-free DNA molecules ending at short-tagged ending positions are described herein.

The values y1 and y2 are examples of calibration values. The data points (x1,y1) and (x2,y2) are examples of calibration data points. The calibration data points can be fit to a function to obtain a calibration curve (e.g., 1010, which may be linear. When a new relative abundance is measured for a new sample, the new relative abundance can be compared to at least one of the calibration values to determine a classification of the proportional contribution of the new sample. The comparison to the calibration value can be made in various ways. For example, the calibration curve can be used to find the proportional contribution x corresponding to the new relative abundance. As another example, the new relative abundance can be compared to calibration value y1 of a first calibration data point to determine whether the new sample as a proportional contribution greater or less than x1.

In other embodiments, a mixture containing more than two types of tissues can be analyzed similarly for the proportional contribution of tissues A as long as the relative abundance of other tissues is relatively constant. Such methods are practically useful for the analysis of different clinical scenarios, for example but not limited to cancer detection, transplantation monitoring, trauma monitoring, infection, and prenatal diagnosis.

For a fetal analysis, a goal may be to provide a quantitative value for the proportional contribution or confirm that a minimum percentage of fetal DNA is present. For example, methods can be used for the determination of fetal DNA concentration in maternal plasma. In maternal plasma, the DNA molecules carrying the fetal genotypes are generally derived from the placenta.

For cancer, other classifications may be desirable. For example, the relative abundance at short-preferred positions can be determined and compared with normal healthy subjects. Through the comparison with a calibration curve similar to FIG. 22, the contribution of the particular tissue (e.g., fetal, tumor, or transplant) can be determined. The value of relative abundance of the tested case can be compared with a range of the contribution of the liver in the healthy subjects.

Similarly, the contribution of the transplanted organ in a patient who has received organ transplantation can be determined by this method. In previous studies, it was shown that patients with rejection would lead to an increased release of DNA from the transplanted organ resulting in an elevated concentration of the DNA from the transplanted organ in plasma. The analysis of relative abundance of the transplanted organ would be a useful way for the detection and monitoring of organ rejection. The regions used for such analysis can vary depending on which organ is transplanted.

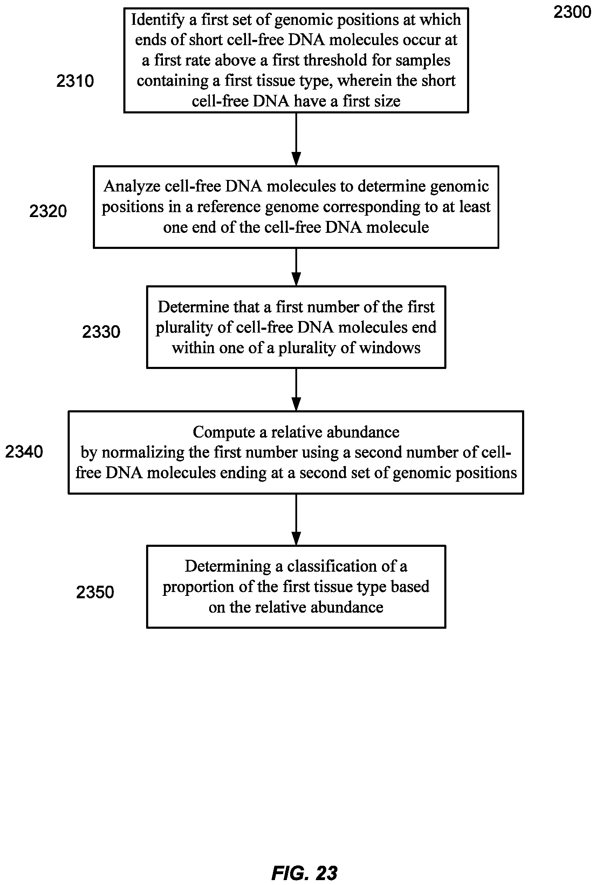

FIG. 23 is a flowchart of a method 2300 of analyzing a biological sample to determine a classification of a proportional contribution of the first tissue type in a mixture according to embodiments of the present disclosure. The biological sample includes a mixture of cell-free DNA molecules from a plurality of tissues types that includes a first tissue type. As with other methods described herein, method 2300 can use a computer system. Examples of the first tissue type include fetal tissue, transplant tissue, and tumor tissue.

At block 2310, a first set of genomic positions is identified at which ends of short cell-free DNA molecules occur at a first rate above a first threshold for samples containing the first tissue type. The short cell-free DNA can have a specified first size, e.g., 60-155 bases, other ranges described herein, or other ranges smaller than long cell-free DNA fragments. A range does not have to be contiguous, e.g., 60-120 and 125-155. As an example, long DNA fragments can be 170-250 bases and other ranges described herein. The higher rate can be determined in at least one additional sample (e.g., in calibration samples). Further details about block 2310 can be found in section II.B above and elsewhere in this disclosure.

In some embodiments, identifying the first set of genomic positions can include analyzing, a second plurality of cell-free DNA molecules from at least one additional sample to identify ending positions of the second plurality of cell-free DNA molecules. The at least one additional sample can be known to include the first tissue type and be of a same sample type as the biological sample. For example, the additional sample can be from a pregnant female, a subject having a transplanted organ, or a subject with a tumor. For each genomic window of a plurality of genomic windows, a corresponding number of the second plurality of cell-free DNA molecules ending on the genomic window can be computed and compared to a reference value to determine whether the rate of cell-free DNA molecules ending on one or more genomic positions within the genomic window is above the threshold.

At block 2320, a first plurality of cell-free DNA molecules from the biological sample of a subject is analyzed. The analyzing of a cell-free DNA molecule can include determining a genomic position (ending position) in a reference genome corresponding to at least one end of the cell-free DNA molecule. Thus, two ending positions can be determined, or just one ending position of the cell-free DNA molecule.

In some embodiments, the analyzing the first plurality of cell-free DNA molecules can include sequencing the first plurality of cell-free DNA molecules to obtain sequence reads and aligning the sequence reads to the reference genome to determine genomic positions of the first plurality of cell-free DNA molecules. In other embodiments, the analyzing the first plurality of cell-free DNA molecules can include hybridization capture or amplification of the first plurality of cell-free DNA molecules at the first set of genomic positions.

The ending positions can be determined in various ways, as described herein. For example, the cell-free DNA molecules can be sequenced to obtain sequence reads, and the sequence reads can be mapped (aligned) to the reference genome. If the organism was a human, then the reference genome would be a reference human genome, potentially from a particular subpopulation. As another example, the cell-free DNA molecules can be analyzed with different probes (e.g., following PCR or other amplification), where each probe corresponds to a genomic location, which may cover the at least one genomic region.

A statistically significant number of cell-free DNA molecules can be analyzed so as to provide an accurate determination the proportional contribution from the first tissue type. In some embodiments, at least 1,000 cell-free DNA molecules are analyzed. In other embodiments, at least 10,000 or 50,000 or 100,000 or 500,000 or 1,000,000 or 5,000,000 cell-free DNA molecules, or more, can be analyzed. As a further example, at least 10,000 or 50,000 or 100,000 or 500,000 or 1,000,000 or 5,000,000 sequence reads can be generated.

At block 2330, it is determined that a first number of the first plurality of cell-free DNA molecules end within one of a plurality of windows. The determination can be performed based on the analyzing of the first plurality of cell-free DNA molecules in block 2320. For example, the genomic positions of the end(s) of the cell-free DNA molecules can be known from the analysis (e.g., alignment or use of particular probes). Each window includes at least one of the first set of genomic positions. As described in section II.A, the first set of genomic positions can be identified from an initial set and then expanded to include windows around the initial set. Thus, a set of short-preferred ending positions can include an expanded set S of ending sites. As examples, the widths of the windows can be 1 bp, 2 bp, 3 bp, 4 bp, 5 bp, 6 bp, 7 bp, 8 bp, 9 bp, 10 bp, 15 bp, 20 bp, 25 bp and 30 bp. The windows may or may not have all the same widths. A reference to bp and bases may be considered as equivalent units for width or length.

At block 2340, a relative abundance of the first plurality of cell-free DNA molecules ending within one of the plurality of windows is computed. The relative abundance can be determined by normalizing the first number of the first plurality of cell-free DNA molecules using a second number of cell-free DNA molecules. The second number of cell-free DNA molecules can include cell-free DNA molecules ending at a second set of genomic positions outside of the plurality of windows including the first set of genomic positions. As an example, the relative abundance can includes a ratio of the first number and the second number.

In various embodiments, the second set of genomic positions can be ending positions preferred for long cell-free DNA fragments or any of the ending positions determining in the biological sample. The second set of genomic positions can be such that ends of long cell-free DNA molecules occur at a second rate above the threshold in the at least one additional sample. The long cell-free DNA would have a second size that is greater than the first size. The first size can have a first range of sizes, and the second size can have a second range of sizes. The first range of sizes can have less than the second range of sizes in that a first maximum of the first range of sizes being less than a second maximum of the second range of sizes. As described herein, the first range of sizes can overlap with the second range of sizes. In another implementation, the second set of genomic positions can include all genomic positions corresponding to an end of at least one of the first plurality of cell-free DNA molecules, thereby including various genomic positions potentially sampled in a random fashion.

Another example of a relative abundance value is a proportion of cell-free DNA molecules ending on a genomic window, e.g., measured as a proportion of sequenced DNA fragments ending on a preferred ending position. Thus, the second set of genomic positions can include all genomic positions corresponding to an end of at least one of the first plurality of cell-free DNA molecules. In another examples, the second set of genomic positions can correspond to windows that are larger than the windows used to define the first set of genomic positions, thereby including addition genomic positions not in the first set. The widths of the two sets of windows can be adjusted to achieve the desired performance. As examples, the widths of the second set of windows can be 20 bp, 25 bp, 30 bp, 40 bp, 50 bp, 60 bp, 70 bp, 80 bp, 100 bp, 120 bp, 140 bp, 160 bp, 180 bp and 200 bp

At block 2350, the classification of the proportional contribution of the first tissue type is determined by comparing the relative abundance to one or more calibration values determined from one or more calibration samples whose proportional contributions of the first tissue type are known. Examples are shown in FIGS. 4A and 4B for fetal tissue being the first tissue type and shown in FIGS. 10 and 11 for tumor DNA. As an example, the classification of the proportional contribution can correspond to a range above a specified percentage. As another example, the classification can correspond to a particular percentage within a specified accuracy range or to a specified precision. As further examples, the classification can be a textual classification corresponding to ranges, such as low, medium, and high.

As described above, the comparison to the calibration values can be performed via a calibration function that has been determined using calibration data points measured in calibration samples, whose proportional contribution is measured via other techniques, e.g., using a tissue-specific marker (e.g., for fetal, transplant, or tumor tissue), such as tissue-specific allele or tissue-specific epigenetic markers, such as hypomethylation or hypermethylation at a particular site of the particular tissue relative to other tissues. Accordingly, comparing the relative abundance to the one or more calibration values can use a calibration function fit to calibration points comprising proportional contributions of the first tissue type measured in a plurality of calibration samples and respective relative abundances determined in the plurality of calibration samples.

When the first tissue type is a tumor, the classification can be selected from a group consisting of: an amount of tumor tissue in the subject, a size of the tumor in the subject, a stage of the tumor in the subject, a tumor load in the subject, and presence of tumor metastasis in the subject.

For cancer, if the proportional contribution is high, further action can be performed, such as a therapeutic intervention or imaging of the subject (e.g., if the first tissue type corresponds to a tumor). For example, an investigation can use imaging modalities, e.g. computed tomography (CT) scan or magnetic resonance imaging (MRI), of the subject (entire subject or a specific part of the body (e.g. the thorax or abdomen), or specifically of the candidate organ) can be performed to confirm or rule out the presence of a tumor in the subject. If presence of a tumor is confirmed, treatment can be performed, e.g., surgery (by a knife or by radiation) or chemotherapy.

Treatment can be provided according to a determined level of cancer, the identified mutations, and/or the tissue of origin. For example, an identified mutation (e.g., for polymorphic implementations) can be targeted with a particular drug or chemotherapy. The tissue of origin can be used to guide a surgery or any other form of treatment. And, the level of cancer can be used to determine how aggressive to be with any type of treatment, which may also be determined based on the level of cancer.

*C. Determining sequence imbalance*

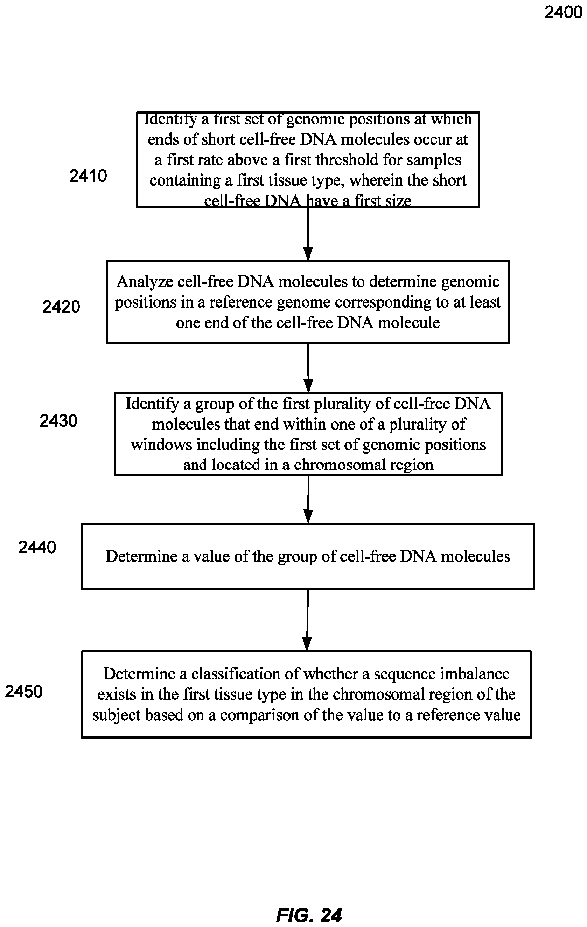

FIG. 24 is a flowchart of a method 2400 of analyzing a biological sample to determine whether the first tissue type exhibits a sequence imbalance in a chromosomal region in the mixture of cell-free DNA molecules according to embodiments of the present disclosure. The sequence imbalance can relate to various measurements in the chromosomal region, e.g., an aneuploidy, amplifications/deletions, or genotyping the first tissue type at a locus in the region. For example, the first tissue can have a different genotype from other tissue types of the plurality of tissues types. The chromosomal region may be an entire chromosome. Examples of the first tissue type include fetal tissue and tumor tissue.

At block 2410, a first set of genomic positions is identified at which ends of short cell-free DNA molecules occur at a first rate above a first threshold for samples containing the first tissue type. The short cell-free DNA can have a first size, which may be one or more ranges. Block 2410 can be performed in a similar manner as block 2310 of FIG. 23.

At block 2420, a first plurality of cell-free DNA molecules from the biological sample of a subject is analyzed. Analyzing a cell-free DNA molecule includes determining a genomic position in a reference genome corresponding to at least one end of the cell-free DNA molecule. Block 2420 can be performed in a similar manner as block 2320 of FIG. 23.

At block 2430, a group of cell-free DNA molecules that end within one of a plurality of windows is identified based on the analyzing of the first plurality of cell-free DNA molecules. Each window includes at least one of the set of genomic positions and is located in the chromosomal region. By selecting particular cell-free DNA molecules that end on this set of genomic positions preferred by short DNA fragments, this group of cell-free DNA molecules can effectively be enriched for the first tissue type, e.g., tumor DNA or fetal DNA. Further, DNA fragments in the cell-free mixture covering or ending on the set of genomic positions could be amplified or captured to provide further enrichment.

Block 2430 can be performed in a similar manner as block 2330 of FIG. 23, e.g., with respect to the identifying of DNA molecules that end at one of the set of genomic positions. By having the windows within the chromosomal region, the group of cell-free DNA molecules can act as a representative set for that chromosomal region. Thus, this group of cell-free DNA molecules (enriched for the first tissue type) can be analyzed using existing techniques for noninvasive analysis.

In various embodiments, the group can be selected for a particular haplotype. Another group of cell-free DNA molecules that end within one of a plurality of windows can correspond to the other haplotype. Or, a subgroup of the group can correspond to one haplotype and another subgroup of the group can correspond to the other haplotype. The DNA molecules corresponding to a haplotype can be determined based on alleles (e.g., determined by sequencing or probes) of the DNA molecules matching a particular allele of a particular haplotype. Later blocks of method 2400 can analyze the two groups to compare properties of the two haplotypes, e.g., to determine a sequence imbalance.

At block 2440, a value of the group of cell-free DNA molecules is determined. The value can be determined in various ways. For example, a number of cell-free DNA molecules in the group can be determined, e.g., as described in U.S. Patent Publication Nos. 2009/0087847, 2009/0029377, 2011/0105353, 2013/0040824, and 2016/0201142. As another example, the value could be a statistical value of a size distribution of the group of cell-free DNA molecules, e.g., as described in U.S. Patent Publication Nos. 2011/0276277, 2013/0040824, and 2016/0201142. As another example, the value could be a methylation density of the group of cell-free DNA molecules, e.g., at CpG sites covered by these cell-free DNA molecules. Accordingly, in various embodiments, the value of the group of cell-free DNA molecules can be an amount of the group of cell-free DNA molecules, a statistical value of a size distribution of the group of cell-free DNA molecules, or a methylation level of the group of cell-free DNA molecules. Further details about using methylation to detect a sequence imbalance can be found in PCT publication WO 2017/012544.

The value of the group of the group of cell-free DNA molecules can be normalized, e.g., to account for differing number of DNA molecules in difference samples. For example, the value of the group can be normalized by (e.g., divided by) a value from another group cell-free DNA molecules of one or more reference regions or a total number of cell-free DNA molecules in the sample. As another example, a same number of cell-free DNA molecules can be analyzed, which is a type of normalization by the total number of cell-free DNA molecules in the sample.

At block 2450, a classification of whether a sequence imbalance exists in the first tissue type in the chromosomal region of the subject is determined based on a comparison of the value to a reference value. The reference value can be determined in various ways, e.g., from healthy subjects, from subjects that have cancer or are pregnant, from one or more values determined from other regions in the sample that do not have an imbalance, or from another haplotype in the chromosomal region (e.g., to determine what the genotype is). The genotype can be determined by analyzing an imbalance in reads for different alleles at one locus or for haplotypes, e.g., as described for section III.C. The comparison can involve a determination of whether the value is statistically different than the reference value (e.g., exceeding a cutoff value, such as a specific number of standard deviations, as determined from a population).

As an example, a first number of cell-free DNA molecules ending in one of first windows in a first chromosomal region (clinically-relevant region being tested) can be compared to a second number of cell-free DNA molecules ending at one of second windows in one or more reference chromosomal regions, where the first and second windows include at least one of the set of genomic positions. Such a comparison can include determining a separation value (e.g., a difference or a ratio) using the first number and the second number, where the separation value can be compared to a reference value to detect the sequence imbalance. Similarly, the first and second numbers can be determined for first and second haplotypes.

As another example, a size distribution can be determined of the group of cell-free DNA molecules. A statistical value can be determined of the size distribution, e.g., an average or median size, or an amount of short DNA molecules to long DNA molecules. A separation value can be determined between a first statistical value of the chromosomal region and a second statistical value of the size distribution of one or more reference chromosomal regions, where the separation value can be compared to a reference value to detect the sequence imbalance. Similarly, the first and second statistical values can be determined for first and second haplotypes.

As yet another example, a methylation level can be determined using the methylation status (methylated or not methylated) at a plurality of sites covered by the group of cell-free DNA molecules. The methylation level for the group can be compared to another methylation level for another group corresponding to one or more reference chromosomal regions. A separation value can be determined between the two methylation levels, where the separation value can be compared to a reference value to detect the sequence imbalance. Similarly, the two methylation levels can be determined for first and second haplotypes. In another example, multiple methylation levels can be determined for different sites in a region, and a fractional contribution can be determined using a deconvolution technique as in WO 2017/012544. The fractional contribution would be an example of a value of the group determined in block 2440.

Accordingly, for haplotype analysis, the value of the group may be determined using a first subgroup corresponding to a first haplotype a second subgroup corresponding to a second haplotype in the chromosomal region. A separation value between a first haplotype value and a second haplotype value (examples are provided above) can be determined and compared to the reference value.

For a comparison among regions (as described above), the reference value can be determined by identifying a reference group of cell-free DNA molecules that end within one of a plurality of reference windows, each reference window including at least one of the set of genomic positions and are located in one or more reference chromosomal regions, which may be known or assumed to not have a sequence imbalance (e.g., an amplification or deletion). Then, the reference value can be determined from the reference group of cell-free DNA molecules. The reference value can be of the same type as the value (e.g., amount, statistical size value, or a methylation level). A separate value between the value and the reference value can then be compared to a cutoff value that separates classifications of a sequence imbalance existing and no sequence imbalance existing, e.g., as shown in FIG. 5A.

For examples when the sequence imbalance is the result of the different genotype of the first tissue type from other tissue types (e.g., as described for section III.C), the value of the group of cell-free DNA molecules can be a relative abundance between a first number of cell-free DNA molecules of the group that have a first allele at the locus and a second number of cell-free DNA molecules that have a second allele at the locus. When the other tissue types are heterozygous at the locus in the chromosomal region, the classification of the sequence imbalance can be an overabundance of the first allele indicating that the first tissue type is homozygous for the first allele. When the other tissue types are heterozygous at the locus in the chromosomal region, the classification can be that no imbalance exists indicating the first tissue type is heterozygous for the first allele and the second allele.

If a sequence imbalance is associated with cancer (amplifications or deletions), then a level of cancer can be determined (e.g., based on a number of regions having the sequence imbalance). Treatment can then be provided, e.g., as described herein, such as for method 2300.

## VII. ORIENTATION-AWARE PLASMA CELL-FREE DNA FRAGMENTATION ANALYSIS IN OPEN CHROMATIN REGIONS

Recent studies had demonstrated the clinical feasibility of cfDNA analysis for sensitive cancer screening (56, 57, 61). For future developments of this field, it would bebeneficial to develop a robust approach for localizing the site of the tumor following a positive liquid biopsy test. Exploiting the differences in DNA methylation patterns between tissues, we have previously demonstrated that circulating fetal-derived DNA in maternal plasma originated predominantly from the placenta (58). This work was based on the detection of unmethylated *SERPINB5* sequences as a placental marker in maternal plasma (58). More recently, an approach has been applied to the detection of cfDNA derived from the brain (78), cells of the erythroid lineage (75), the heart (109), and the liver (64, 77).

We have further developed a general DNA methylation-based approach for determining the contributions of multiple tissue types into the cfDNA pool, a method that we have named "plasma DNA tissue mapping" (102). This principle has also been utilized to predict the tissue-of-origin of tumors by other researchers (72, 79). These published approaches used whole genome bisulfite sequencing (BS-seq) (80, 54, 85). However, BS-seq has the disadvantage that bisulfite conversion is associated with degradation of input DNA (65) and also introduces GC content changes which may lead to biases in the sequencing data (89).

Besides DNA methylation, recent studies had demonstrated that cfDNA molecules retained signatures of their nucleosomal origin, showing a size distribution with a dominant peak at 166 bp and a ~10 bp periodicity (81). CfDNA has been shown to carry a non-random pattern of fragmentation that provides a window into epigenetic regulation across the genome (67). Considering that nucleosome positioning across the genome is highly related to the cell identity (92), such fragmentation patterns thus hold the potential of tracing back the tissue-of-origin of cfDNA molecules. Snyder et al. showed that the plasma DNA molecules carried nucleosomal footprints (98). The authors further constructed a "nucleosome track" and found that the nucleosome spacing pattern could be used to infer the tissue origin of cfDNA. They also demonstrated the potential of this approach in predicting the tumor origin in cancer patients. In another study, Ulz et al. reported that plasma DNA coverage in the promoters could be used to predict the expression of genes (106). Our group had demonstrated the existence of tissue-specific preferred ending sites in cfDNA which showed clinical utility in predicting the fetal DNA fractions in maternal plasma (55).

In this disclosure, we further explore the clinical potential of fragmentation patterns, especially in tracing the tissue-of-origin of cfDNA molecules. We first profiled the coverage and cfDNA fragment end signatures around known well-positioned nucleosome arrays and open chromatin regions. During the analysis, we separated the plasma DNA fragment ends into two groups where the orientation information was considered, namely ends on an upstream or downstream side of a plasma DNA fragment in relation to the reference genome. We showed that in these regions, plasma DNA showed characteristic fragmentation patterns including sequencing coverage imbalance and differences between the upstream and downstream fragment end signals. We then analyzed the plasma DNA fragmentation patterns in various tissue-specific open chromatin regions and further quantified the fragmentation patterns in various clinical scenarios to investigate the feasibility in inferring the tissue-of-origin of cfDNA, including predicting the tumor location in cancer patients.

### A. Conceptual framework and nomenclature

FIGS. 25A-25F shows the conceptual framework of our approach. FIG. 25A shows an illustration of nucleosome positioning in the genome. The nucleosomes 2505 are wrapped with DNA 2510 (yellow line). Other portions of DNA are also shown: linker DNA 2512 (brown line), and active regulatory elements 2514 (green line), which are in the open chromatin region. An abstraction of nucleosome positioning and illustration of cutting events (scissors) during apoptosis is also shown.

In eukaryotic chromatin, the nucleosome is the basic unit for DNA packaging, which consists of a DNA segment wrapped around histone proteins. Nucleosomes are generally connected to each other by a relatively short linker DNA, except in active regulatory elements (e.g., open chromatin regions) where nucleosomes are evicted and the nearby nucleosomes will be connected by a much longer stretch of DNA. It is believed that a significant proportion of cfDNA molecules are released following cell apoptosis (68, 81). During apoptotic DNA fragmentation, it is proposed that endonuclease enzymes prefer cutting internucleosomal DNA (94, 103).

FIG. 25B shows an illustration of cfDNA generated from apoptotic DNA fragmentation. DNA portions 2520 wrapped around the nucleosomes is preserved while very small DNA pieces 2522 in the linkers and open chromatin regions are cleaved into such small pieces (grey line), which cannot be sequenced efficiently. As a result, when the cfDNA molecules are subjected to sequencing, the DNA portions 2520 wrapped on the histones are preserved. On the other hand, DNA originating from the linkers and active regulatory elements, as they are relatively unprotected, will be cleaved into small DNA pieces 2522 (grey lines) and may not be efficiently sequenced (FIG. 25C) (69, 98, 106).

FIG. 25C is an illustration of the sequenced reads and extraction of the two ends. Red ends 2530 and blue ends 2532 represent the U (upstream) and D (downstream) plasma DNA ends, respectively. The DNA pieces 2522 are not shown as they are not sequenced. Therefore, the genomic coverage of cfDNA would be high in the nucleosomes, and low in the linkers and open chromatin regions (FIG. 25D).

FIG. 25D shows the genomic coverage. The horizontal axis corresponds to the genomic coordinate. The vertical axis corresponds to the number of reads covering each coordinate (position). In this idealized depiction, the coverage is zero (or near zero) in the linker and open chromatin regions, but substantial and uniform in the nucleosome regions.

FIG. 25E shows U and D fragment end profiles of cfDNA in relation to the genomic coordinate. We took advantage of the orientation information of the cfDNA fragment ends and defined those cfDNA fragment ends based on their alignment to the reference genome. An upstream (U) end 2530 represented one that had a lower value in the genome coordinate, while a downstream (D) end 2532 represented one that had a higher value in the genome coordinate. Hence, DNA wrapped on the nucleosomes will result in a pair of U and D ends at the upstream and downstream borders of the nucleosomes, respectively.

Example locations of the upstream ends 2530 and downstream ends 2532 of the DNA are shown in FIG. 25E. The upstream U signals 2550 are located at ending positions of the upstream ends 2530. The downstream D signals 2552 are located at ending positions of the downstream ends 2532. The U signals 2550 and the D signals 2552 are bunched together showing some stochastic processes, as not every fragment will be cut at the same position. Such a window of positions can correspond to the windows described above for size-preferred ending sites.

The linker and open chromatin regions can be identified based on the U signals 2550 and the D signals 2552. For the linker or open chromatin regions, there would be D ends flanking their upstream boundaries, and U ends flanking their downstream boundaries. In this regard, the U and D end signals could be used to infer the positioning of the nucleosomes, linkers, and the open chromatin regions (FIG. 25F).

FIG. 25F shows smoothed plasma DNA end signals and deduced nucleosome positioning. Such smoothed end signals illustrate realistic data as the ends of DNA fragments will show a distribution due to the stochastic processes involved in cutting the DNA. Upstream distributions 2560 are centered around the U signals 2550 in FIG. 25E. Downstream distributions 2562 are centered around the D signals 2552 in FIG. 25E.

The different regions are identified under the smoothed plasma DNA end signals. Purple lines 2575 represent the nucleosomes. Brown lines 2572 represent the linker regions. Green lines 2574 represent open chromatin regions.

*B. Results showing differential phasing*

The hypothesis from the conceptual framework was tested by analyzing various parts of the genome, e.g., active promoters of housekeeping genes, inactive promoters, and tissue-specific open chromatin regions.

**1. Differentially phased plasma DNA fragment ends in a nucleosome array**

To illustrate the above concept in a human genomic region, we first examined chr12p11.1, a region known to have well-positioned nucleosomes in almost all human tissue types (107, 63, 98). To do this, we pooled plasma DNA data from 32 healthy non-pregnant subjects from our previous study (70) and profiled the coverage and fragment ends in this region.

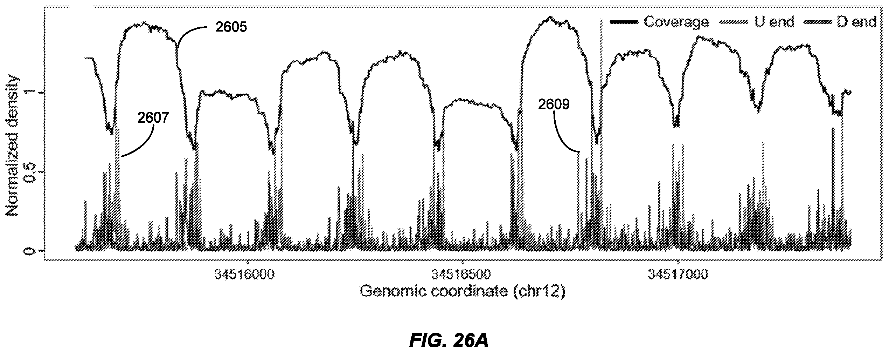

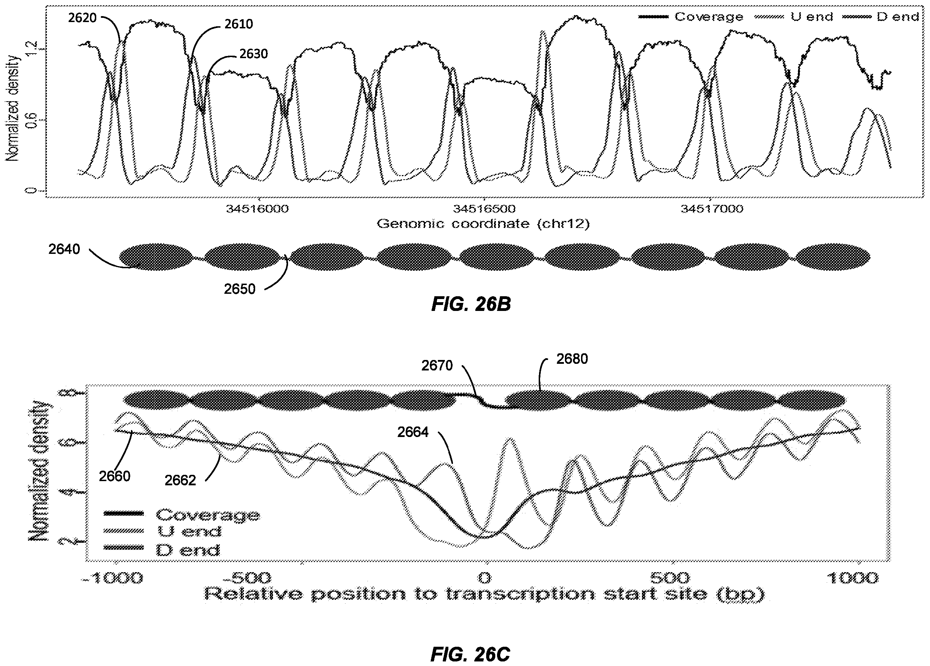

FIGS. 26A and 26B show plasma DNA fragmentation pattern in the chr12p11.1 region in pooled healthy non-pregnant subjects according to embodiments of the present invention. FIG. 26A shows the raw signal for genomic coverage 2605, upstream U ending locations 2607, and downstream D ending locations 2609. The X-axis is the genomic coordinates. The Y-axis is a normalized density for the genomic coverage, so that the average value at any coordinate is 1. The genomic coverage 2605 corresponds to the number of reads aligned to each genomic. The data for the upstream ending locations 2607 and the downstream ending locations 2609 are normalized counts for the number of DNA fragments ending at those positions. Since we are only interested in the relative counts of the ends across different positions, the raw counts are normalized in a way to fit the Y-axis in this figure.

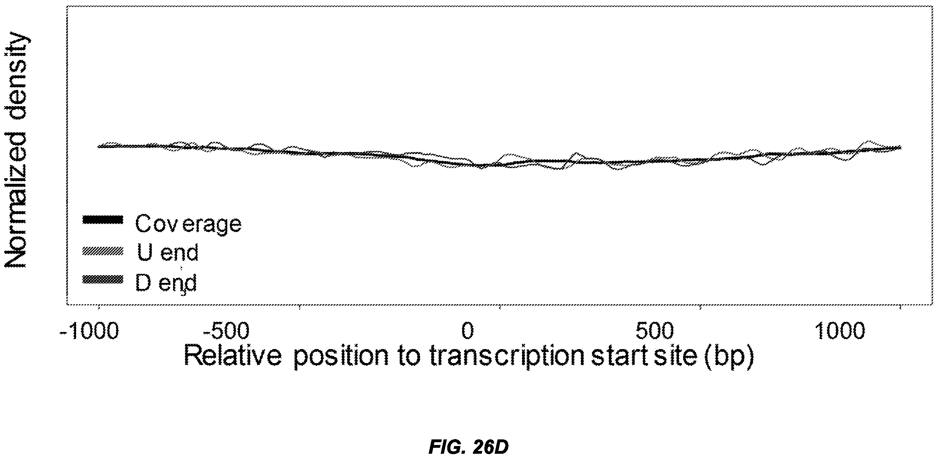

As shown in FIG. 26A, plasma DNA coverage 2605 showed a strong periodicity pattern of ~190 bp and the regions with higher and lower coverages corresponded to the nucleosomes and linkers, respectively (98). The U ending locations 2607 and D ending locations 2609 showed a similar periodicity pattern and both were enriched in the linkers, i.e., there were more U and D ends in the linker regions than in the nucleosomes. The coverage signal was normalized by dividing the raw signal by the average signal in this region; the end signals were linearly adjusted to fit into the figure. These non-universal normalization procedures between coverage and end signals in FIG. 26A, 26B, 26C, and 26D were for illustration of the fragmentation pattern purpose only.

FIG. 26B shows the smoothed signal and the deduced nucleosome positioning. The U and D end signals were then smoothed using the LOWESS (locally weighted scatterplot smoothing) algorithm (60) for further analyses. As shown in FIG. 26B, the distance between any D end peak (e.g., 2610) to its nearest upstream U end peak (e.g., 2620) was ~170 bp, which was roughly the size of a nucleosome (101). The distance between any D end peak (e.g., 2610) to its nearest downstream U end peak (e.g., 2630) was ~20 bp, which was roughly the size of a linker (101). Below the plot, the nucleosomes 2640 and the linkers 2650 are shown at the positions corresponding to the data in the plot.

The data thus were highly concordant with our conceptual framework (FIGS. 25A-25F) and showed that differentially phased plasma DNA fragment ends indeed reflected the nucleosome positioning in this region. Notably, with the separation of U and D ends, we were able to resolve the positioning of both the nucleosomes and linkers, which presents an advance over previous studies that mostly focused on predicting the positions of the nucleosome centers (i.e., the loci with maximum nucleosome protection) (63, 90, 98).

Besides chr12p11.1 region, nucleosomes around active promoters are also known to be well positioned (69). To explore the fragmentation pattern around active promoters, a list of human housekeeping genes were obtained from the literature (62).

FIG. 26C shows the plasma DNA coverage and end signals around the active promoters of housekeeping genes. Plasma DNA coverage 2660, U ending signal 2662, and D ending signal 2664 for the housekeeping genes located on the Watson strand are shown. The X-axis is the genomic coordinate relative to a transcription start site (TSS) of the housekeeping genes. The Y-axis is the normalized density of the Plasma DNA coverage 2660, U ending signal 2662, and D ending signal 2664. The TSS is shown at the center of an open chromatin region 2670 that is between two sets of nucleosome arrays.

The housekeeping genes located on the Crick strand showed an almost identically mirrored pattern. Plasma DNA coverage 2660 showed a "V" shape pattern around the promoters. However, the end profiles 2662 and 2664 showed a strong periodicity and phased difference between U and D ends, which was consistent with a nucleosome-depleted region around the transcription start site (TSS) and well-positioned nucleosome arrays nearby. In addition, a ~60 bp distance between the TSS and the +1 nucleosome 2680 (i.e., the first nucleosome downstream of the TSS) could be observed, which was consistent with the canonical gene structure in a human (69).

Furthermore, we also mined a list of genes that were not expressed in major human somatic tissues from the Expression Atlas (73) to investigate the fragmentation pattern around inactive promoters where there were no such nucleosome-depletion patterns.

FIG. 26D shows the plasma DNA coverage and end signals around inactive promoters. Around the inactive promoters, plasma DNA ends were found to be evenly distributed and did not show any specific nucleosome positioning pattern around the promoters of these unexpressed genes. Accordingly, promoters of non-expressed genes of a particular type of cell are inactive and do not have a structure indicating open chromatin regions. These results were consistent with previous studies on nucleosome positioning in which DNA fragment ends following micrococcal nuclease or transposase digestion were studied (96, 95). Taken in aggregate, our results suggested that differentially phased plasma DNA fragment ends could indeed inform the nucleosome positioning pattern in active promoters.

## 2. Differentially phased plasma DNA fragment ends in tissue-specific open chromatin regions

Open chromatin regions are regulatory elements that are known to have a paucity of nucleosomes in the center and are flanked by well-phased nucleosome arrays (63, 95). Therefore, we hypothesized that cfDNA derived from such regions might also exhibit differentially phased fragment end signals. Hence, we first investigated the common open chromatin regions shared by T-cells and the liver, considering that these tissues are important contributors to the plasma DNA pool in various clinical scenarios. Hence, DNA derived from the T-cells was one example of plasma DNA released from the hematopoietic system (103), which is the major source of plasma DNA in healthy individuals (84). The liver is another major source of plasma DNA in healthy individuals as well as liver transplantation recipients and liver cancer patients (83, 64, 77).

We obtained the open chromatin data for T-cells and the liver from the RoadMap Epigenomics project (93) and the ENCODE project (104) (see Materials and Methods). We identified the open chromatin regions that were shared by T-cells and liver as the common open chromatin regions. We then performed fragmentation analysis on these regions in the pooled plasma DNA data.

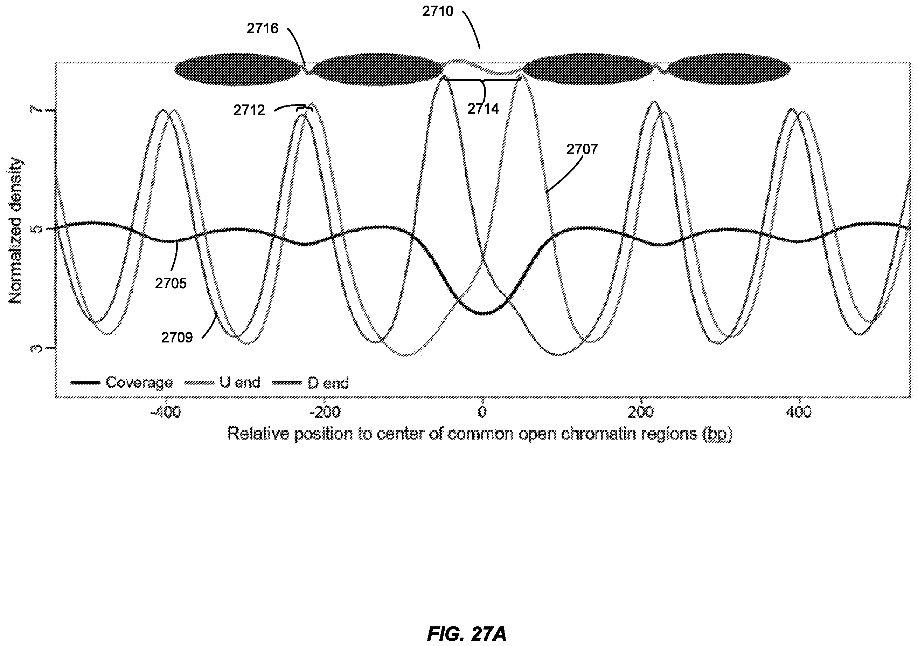

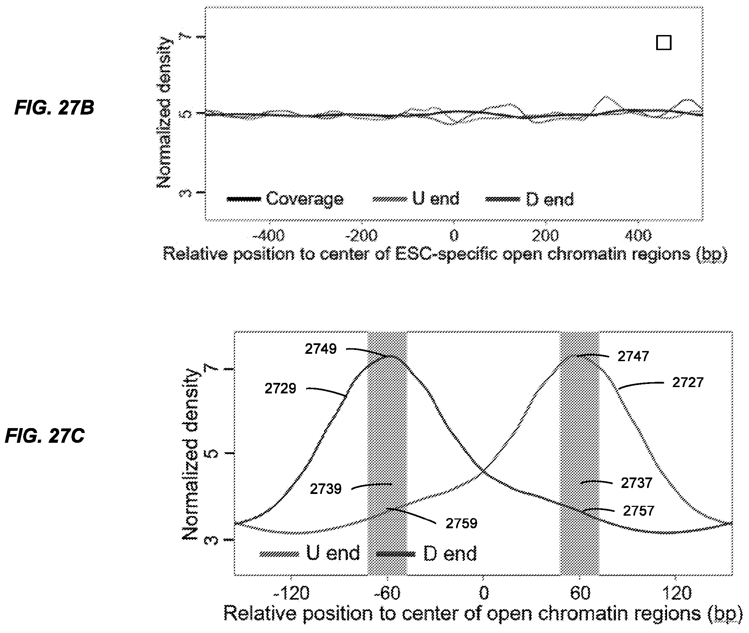

FIGS. 27A, 27B, and 27C show plasma DNA fragmentation pattern in pooled healthy non-pregnant subjects according to embodiments of the present invention. The DNA fragmentation in and near open chromatin regions was analyzed using upstream and downstream ending signals along with genomic coverage.

FIG. 27A shows the pattern in common open chromatin regions shared by T-cells and liver cells (deduced nucleosome positioning was also plotted). The X-axis is the relative position to a center of the common open chromatin regions. The Y-axis is the normalized density of the genomic coverage 2705, the upstream ending signal 2707, and the downstream ending signal 2709. The open chromatin region 2710 is shown above with two nucleosomes on either side. Both the coverage and end signals were normalized by dividing by their corresponding total signals then amplified by a constant numeric factor of 1000, such that the mean values of the coverage and end signals were uniformly adjusted to 5. This normalization was applied to all the figures showing the coverage and end signals around open chromatin regions (i.e., FIG. 27 to 29).

The downstream peaks coincide with a downstream end of the nucleosomes, and the upstream peaks coincide with the upstream ends of the nucleosomes. The extent of the difference between the two peaks indicates whether a linker exists between the two nucleosomes or an open chromatin region exists.

As shown in FIG. 27A, a characteristic fragmentation pattern of plasma DNA, including coverage imbalance and differentially phased fragment ends, could be observed. The coverage imbalance is illustrated by the coverage dip at coordinate 0, i.e., the center of the common open chromatin regions. The differentially phased fragment ends are shown as small separations (e.g., 2712) between the peaks for linker regions 2716, and larger separations (e.g., 2714) for the open chromatin region 2710. These results are a consequence of a nucleosome-depleted region in the center of the open chromatin regions and the presence of neighboring well-phased nucleosomes. These results thus showed that differentially phased plasma DNA fragment ends could inform the nucleosome positioning pattern in the open chromatin regions.

FIG. 27B shows the pattern in embryonic stem cell (ESC)-specific open chromatin regions. As a negative control, we used the same dataset to analyze the plasma DNA fragmentation pattern around the open chromatin regions that were specific to embryonic stem cells (ESC). We reasoned that no plasma DNA would come from ESC in healthy adults. Indeed, we found that the nucleosome positioning pattern (e.g., nucleosome-depletion in the center of the open chromatin regions) could not be seen in the ESC-specific open chromatin regions.

We further hypothesized that cfDNA would only show the fragmentation pattern at the open chromatin regions where the corresponding tissues contributed DNA into the plasma. To test this hypothesis, besides T-cells and the liver, we mined tissue-specific open chromatin regions for 5 additional major human tissues (i.e.,

the placenta, lungs, ovary, breast and small intestines; see Materials and Methods section below). The selection of these tissues was based on data availability and previous knowledge that they would contribute DNA into the plasma in selected clinical scenarios. In previous work, researchers have shown that the placenta-, lung-, ovary- and breast-derived DNA could be found in the plasma of pregnant women, lung cancer, ovarian cancer, and breast cancer patients, respectively (82, 58, 59, 66, 88). In addition, colonic DNA could be found in the plasma of colorectal cancer patients (99). As there was no publicly accessible open chromatin data for colonic tissues, we used the data from the small intestines in the present work to represent the gastrointestinal system and considered small intestine-specific open chromatin regions as a surrogate for colonic ones. These open chromatin regions were mentioned as "intestine-specific" thereafter. We believed that our decision was justified because the epigenomic profiles of the small intestines and the colon shared much similarity (93).

In total, ~26,000 tissue-specific open chromatin regions were obtained for each tissue type (ranges: 7,540-55,537). The tissue-specific open chromatin regions may be identified as described in a later section. We then investigated the plasma DNA fragmentation pattern in these tissue-specific open chromatin regions in the plasma of healthy individuals.

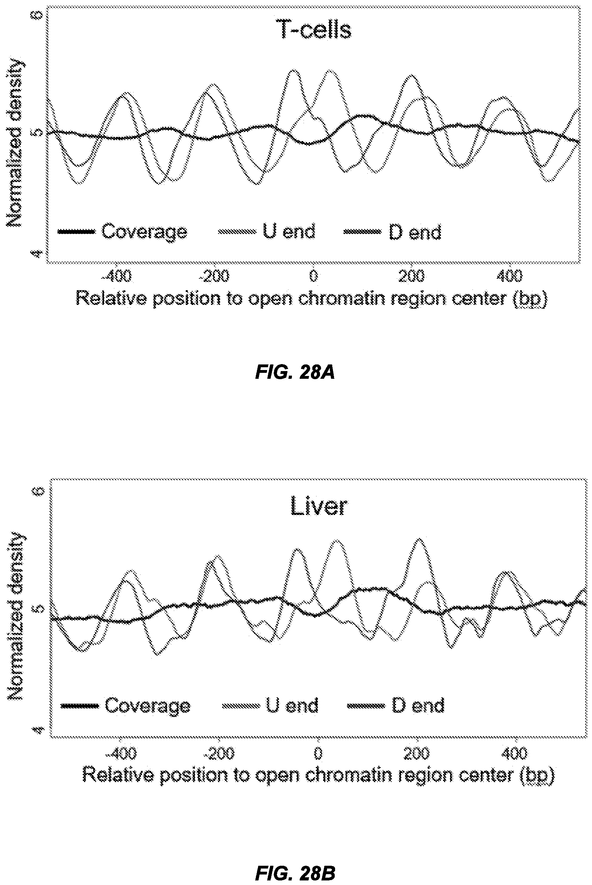

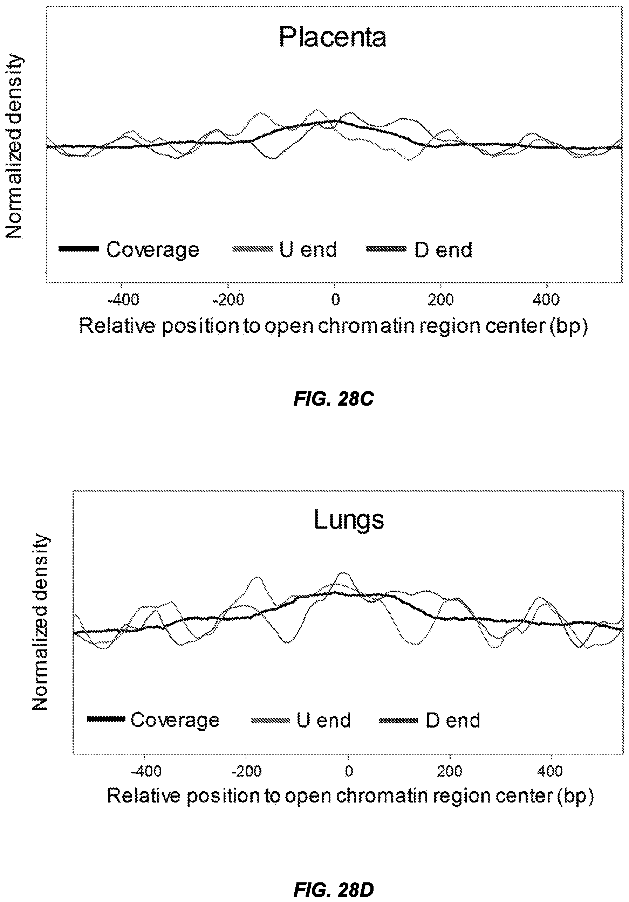

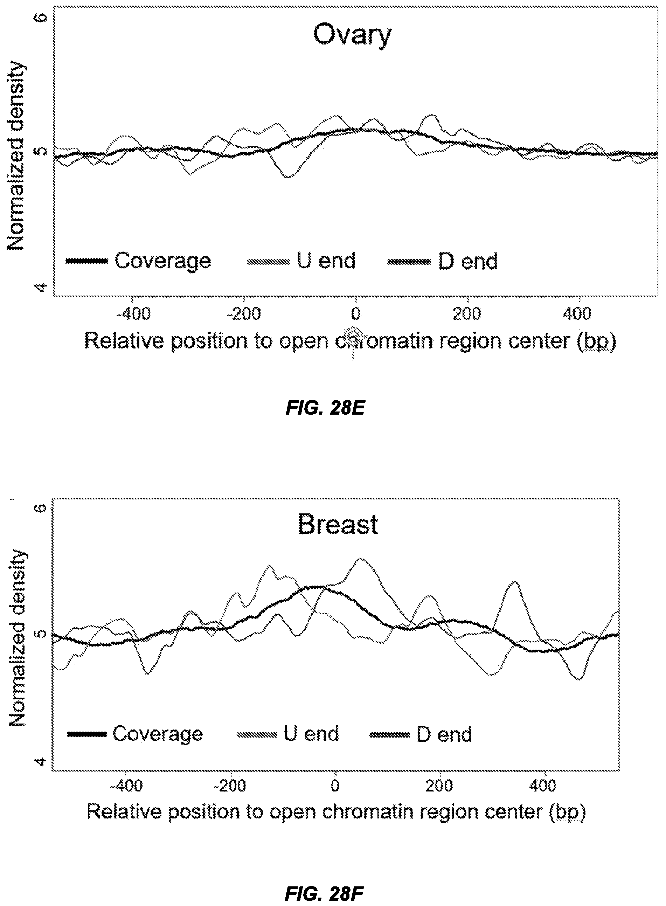

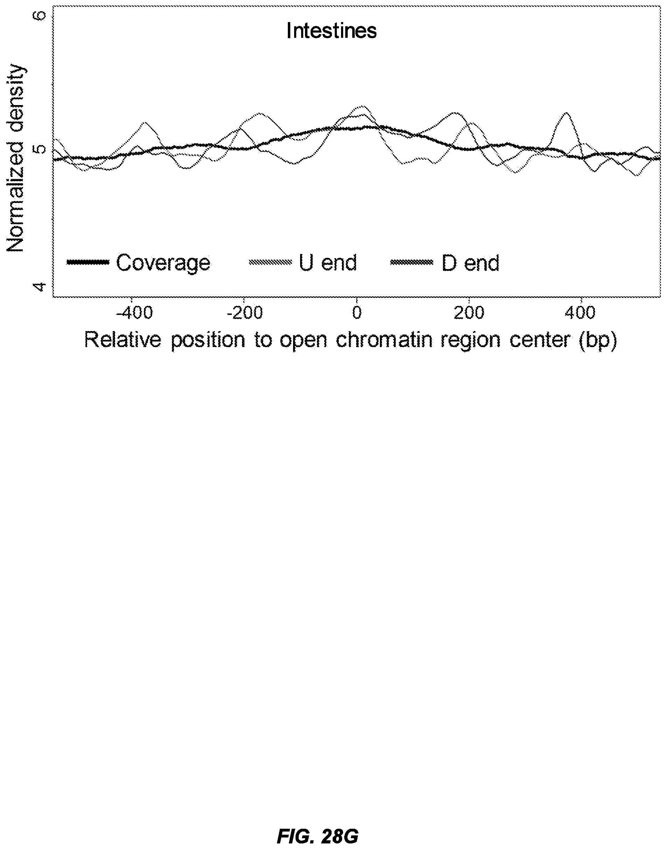

FIGS. 28A-28F show plasma DNA fragmentation pattern in tissue-specific open chromatin regions in a healthy subject according to embodiments of the present disclosure. Each figure shows the result from tissue-specific open chromatin regions corresponding to one tissue type: FIG. 28A T-cells; FIG. 28B the liver; FIG. 28C the placenta; FIG. 28D lungs; FIG. 28E the ovary; FIG. 28F breasts; FIG. 28G intestines. The X-axis show the position relative to a corresponding center of an open chromatin region. The Y-axis is a normalized density for the genomic coverage, U ends, and D ends.

As expected, plasma DNA showed nucleosome-depletion and well-phased nucleosome arrays in the T-cell- and liver-specific open chromatin regions, but not in other tissue-specific open chromatin regions. Well-phased nucleosome arrays can refer to regions in the genome where the locations of the nucleosomes are very reproducible and predictable in nearly all cells of the same tissue type. These results were consistent with the fact that the hematopoietic system and the liver were the major contributors of plasma DNA in healthy individuals (84, 102, 78).

*C. Quantification of plasma DNA fragmentation pattern*

The quantification of plasma DNA fragmentation pattern around an open chromatin region was explored. To quantify the plasma DNA fragmentation pattern around the tissue-specific open chromatin regions, we focused on the nucleosome-depletion signal at the center as it was one of the key characteristics of this pattern (69). In this nucleosome-depletion signal, upstream (U) and downstream (D) ends exhibited the highest read densities at offsets (e.g., 60 bp) in different directions away from the center of the open chromatin regions (FIG. 27C).

FIG. 27C is an illustration of the concept of OCF (Orientation-aware cfDNA fragmentation) value. The X-axis is the relative position to the center of the open chromatin region. The Y-axis shows the normalized density for the upstream ending signal 2727 and the downstream ending signal 2729. The analysis focuses on the U and D ends in the center of the open chromatin regions and measures a separation value (e.g., a differences or a ratio) between U and D signals 2727 and 2729 in the shadowed regions 2737 and 2739 as the OCF value in the tissue-specific open chromatin regions.

As one can see, the D end peak is on the left-hand side while U end peak is on the right-hand side. As can be seen in FIGS. 28A-28G and others, the presence of a tissue type is related to a phasing difference between the upstream and downstream signals. This phasing difference can be measured using information about a difference in position of the peaks, which can provide particular genomic positions for measuring U and D ends. Such a difference in position will result in more upstream ends occurring at one position or window of positions (e.g., in region 2737) than the downstream positions. For example, in region 2737, upstream peak 2747 corresponds to more U ends in that region than the D end signal 2757. Similarly, in region 2739, downstream peak 2749 corresponds to more D ends in that region than the U end signal 2759. Given that most of the tissue-specific open chromatin regions are about the similar size, the regions can be selected at symmetrical positions relative to the center for various tissues.

In some examples, the phasing difference is quantified by the differences of the read densities of the U and D ends in two windows (e.g., 20 bp) around the peaks as follows:

$$OCF = \sum_{-peak-bin}^{-peak+bin} (D-U) + \sum_{peak-bin}^{peak+bin} (U-D)$$

The peak is the distance from the center of the open chromatin region, and the bins is a width of the region. As shown in FIG. 27C, the peaks are 60 bases from the center, and are about 10 bases wide.

This class of parameters is referred to as OCF (Orientation-aware CfDNA Fragmentation) value. In various embodiments, one or both terms may be present, and different values for the peak offset may be used. In some implementations, we used (but not limited to) 60 bp as the peak and 10 bp as the bin size for the quantification. Other example values for the peak offset are 40, 45, 50, 55, 65, 70, and 75 bp. Other example values for the window are 2, 3, 4, 5, 6, 7, 8, 9, 15, 20, 25, and 30 bp. One peak can be identified as a downstream peak, where more downstream ending positions are expected. Another peak can be identified as an upstream peak, where more upstream ending positions are expected. For each case, OCF values were calculated for the 7 tissue types investigated in this study using their tissue-specific open chromatin regions separately.

*D. Applications*

These above results show that differentially phased plasma DNA fragment ends may be used in inferring the tissue origin of cfDNA. And, such results show that the cfDNA fragmentation profile has a relationship with nucleosome positioning in the open chromatin regions. Further results show that quantitative measurements of the differentially phased plasma DNA fragment ends for a particular tissue-specific open chromatin region can be used to detect a pathology in the tissue type. Other cell-free samples besides plasma may also be used.

**1. Quantification of differentially phased plasma DNA fragment ends**

To explore the potential in inferring the relative contributions of various tissues in plasma DNA pool, we developed a novel approach to measure the differential phasing of upstream (U) and downstream (D) fragment ends in tissue-specific open chromatin regions. We generally call this strategy <u>O</u>rientation-aware <u>C</u>fDNA <u>F</u>ragmentation (OCF) analysis, where various OCF values may be used. The OCF values can be based on the differences in U and D end signals at offset positions relative to the center of the relevant open chromatin regions, which occur in the tissue of interest. The more DNA from the tissue of interest, the larger the difference will be, e.g., the difference between the downstream peak 2749 and U end signal 2759 in one or more offset regions.

As shown in FIG. 27A, for tissues that contributed DNA into plasma, one would expect much plasma DNA fragmentation to have occurred at the nucleosome-depleted region in the center of the corresponding tissue-specific open chromatin regions. In such a region, U and D ends exhibited the highest read densities (i.e., peaks) at ~60 bp from the center with the peaks for U and D ends located on the right-hand and left-hand side, respectively. In some examples, we measured the differences of U and D end signals in 20 bp windows around the peaks (e.g., shadowed regions in FIG. 27C) in the tissue-specific open chromatin regions as the OCF value for the corresponding tissue. Conversely, this pattern would not be expected for tissue-specific open chromatin regions where the corresponding tissue did not contribute DNA into the plasma (e.g., ESC in FIG. 27B).

As a result, for tissues that contributed DNA into the plasma, positive OCF values for the corresponding tissue-specific open chromatin regions would be expected. Otherwise, the OCF values should be zero or negative. Of course, a different definition of an OCF value can have the opposite relationship (i.e., negative values being expected if the tested tissue was present). Using the definition with positive values being an indicator, negative values can result from end signals that are noisy, which can relate to sequencing bias (e.g., GC bias), resulting in slightly more DNA in these regions when they do not have the open chromatin structure.

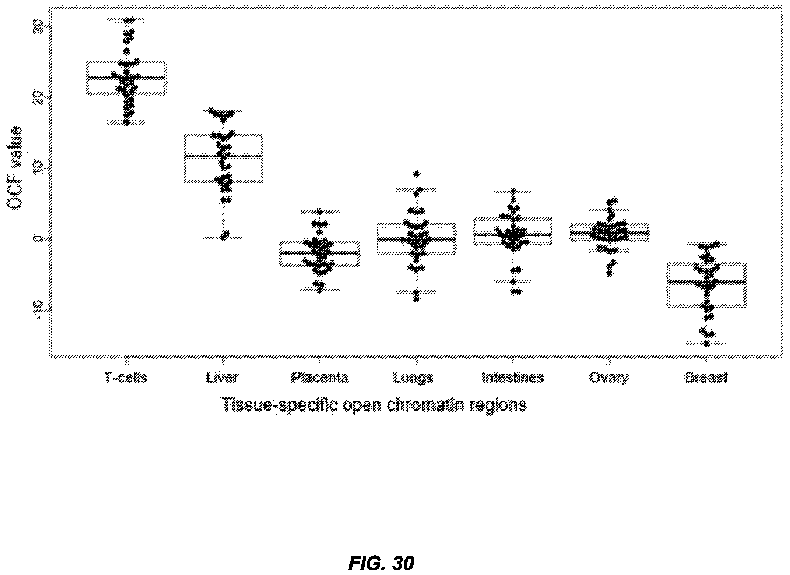

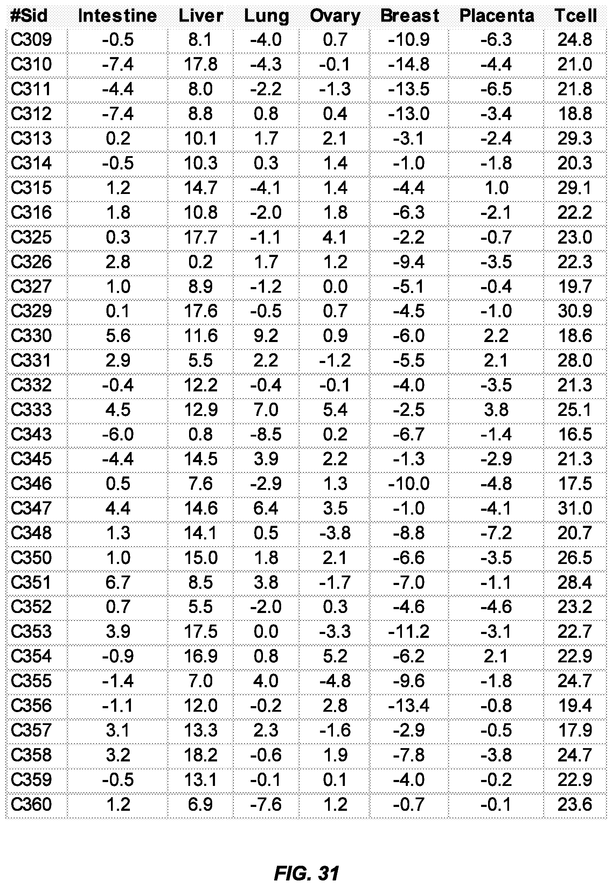

FIG. 30 shows the quantification of plasma DNA fragmentation pattern (OCF values) among various tissues in the healthy non-pregnant subject cohort according to embodiments of the present invention. FIG. 31 shows a table of OCF values for tissue types in healthy individuals according to embodiments of the present invention.

OCF values for the 7 tissue types in the 32 healthy individuals are shown in FIG. 30 and FIG. 31. All subjects showed positive OCF values for T-cells and the liver; in addition, OCF values for T-cells were higher than those for the liver in all cases ($P<0.001$, Wilcoxon signed-rank test). OCF values for other tissue types were much lower and were close to or below zero. These results were consistent with previous data showing that in healthy individuals, the majority of plasma DNA originated from the hematopoietic system and the liver, with the former being the most dominant source (84, 102). Our results thus showed the utility of the OCF values in measuring the relative contributions of different tissues into the cfDNA pool.

## 2. Application in noninvasive prenatal testing

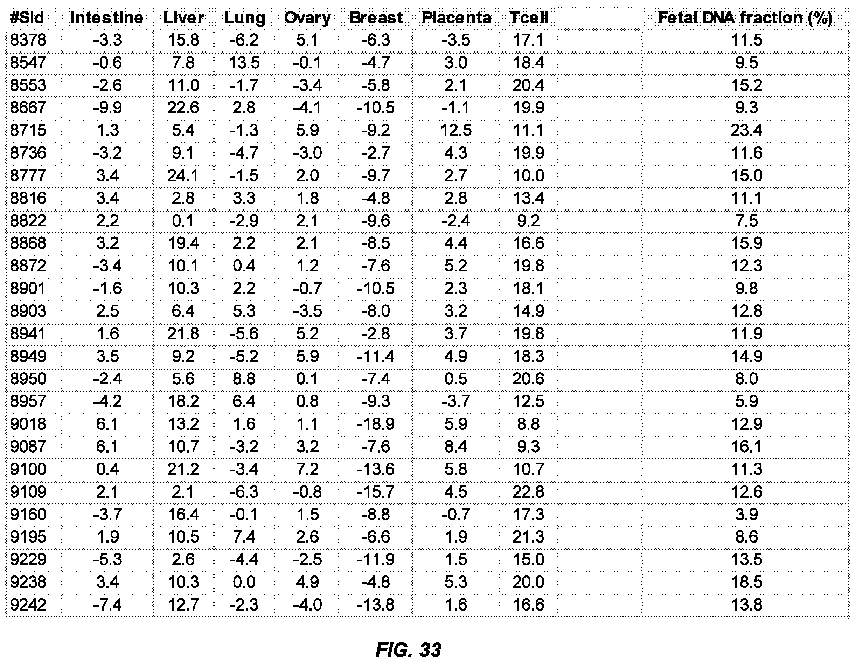

To demonstrate the utility of our approach in noninvasive prenatal testing, we retrieved maternal plasma DNA sequencing data from a previous study (55). As previously discussed, circulating fetal DNA in the plasma of pregnant women mostly originated from the placenta (58). FIGS. 32A-32D show the application of the plasma DNA fragmentation pattern analysis in noninvasive prenatal testing according to embodiments of the present invention. FIG. 33 shows a table of OCF values tissue types in pregnant subjects according to embodiments of the present invention.

FIG. 32A shows plasma DNA fragmentation pattern in the placenta-specific open chromatin regions in a third-trimester pregnant case. The axes are similar to similar plots. A strong nucleosome-positioning pattern similar to that of common open chromatin regions in healthy non-pregnant individuals (FIG. 27A) could be observed. These observations suggested that plasma DNA fragmentation pattern analysis could indeed detect the presence of placental DNA in maternal plasma.

We further investigated the plasma DNA fragmentation pattern using the previously published data from a cohort of 26 first-trimester pregnant cases (55). Each case in this cohort was carrying a male fetus. Hence, the fetal DNA fraction in the plasma DNA could be determined by analyzing the reads aligned to the Y chromosome. We analyzed the plasma DNA fragmentation for placenta (higher in pregnancy cases) and T-cells, which should reduce in pregnancy as percentage from mother decreases.

FIG. 32B shows a comparison of OCF values for T-cells between healthy non-pregnant subjects and pregnant women. FIG. 32C shows a comparison of the OCF values for the placenta between healthy non-pregnant subjects and pregnant women. A total of 25,223 open chromatin regions were used for the T-cells, and 55,537 for placenta When compared to results from non-pregnant healthy individuals, OCF values for the T-cells were significantly decreased in the pregnant samples, and only OCF values for the placenta showed significant elevation (FIGS. 32B and 32C; $P<0.001$, Mann-Whitney rank-sum test; FIG. 33). Only the OCF values for the placenta showed significant elevation (FIG. 32C; $P<0.001$, Mann-Whitney rank-sum test). Thus, a correlation between OCF values and placenta DNA indicates that OCF values can be used to measure a fetal DNA fraction in the cell-free sample.

FIG. 32D shows a correlation between OCF values for placenta and fetal DNA fractions in a cohort of 26 pregnant women. A strong positive correlation between OCF values for the placenta and the fetal DNA fractions was observed (FIG. 32D; $R=0.77$, $P<0.001$, Pearson correlation). Notably, this R value was higher than that obtained by our previous fetal-specific preferred end sites approach (which was 0.66) (55). The fetal DNA fraction is one of the most important parameters governing the performance of noninvasive prenatal testing. These results thus demonstrated the potential utility of differentially phased plasma DNA fragment ends in noninvasive prenatal testing.

## 3. Application in liver transplantation and hepatocellular carcinoma patients

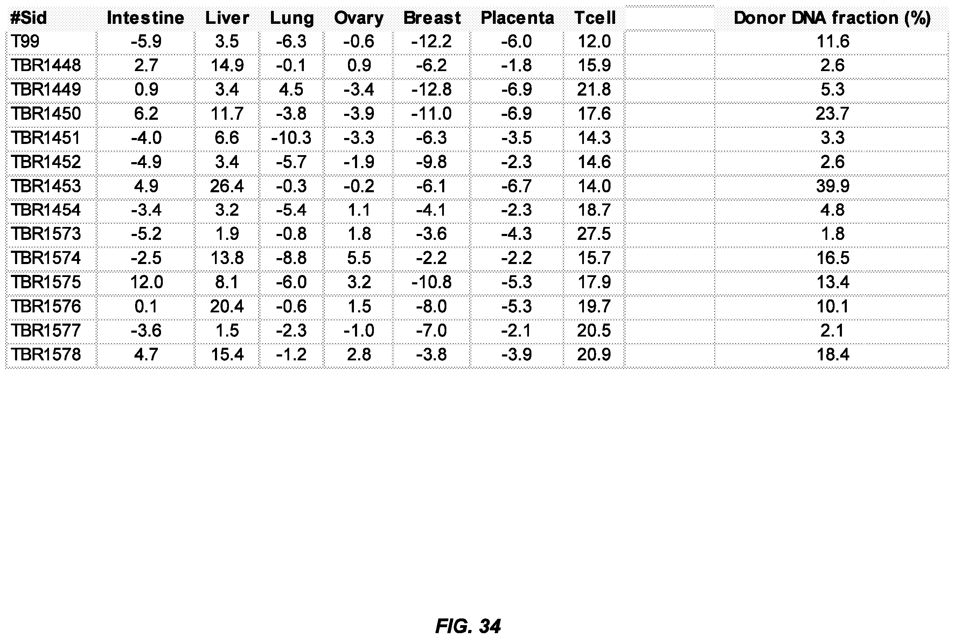

To investigate the performance of plasma DNA fragmentation pattern analysis in predicting the contribution of liver tissue, plasma DNA sequencing results from a previously reported cohort of 14 liver transplantation patients were retrieved (64). For each case, both the donor and recipient were genotyped such that donor-specific informative SNP sites could be identified to deduce the donor-DNA fraction in plasma (64). A donor-specific informative SNP site has an allele that is specific to the donor and not in the recipient. FIG. 34 shows a table of OCF values tissue types in liver transplantation patients according to embodiments of the present invention. The last column shows the donor DNA fraction as determined using donor-specific informative SNP sites. A correlation exists between the OCF values for the liver and the donor DNA fractions.

FIG. 35A shows the correlation between OCF values for the liver and donor DNA fractions in liver transplantation patients. When the plasma DNA fragmentation pattern analysis was performed on this dataset, a positive correlation between OCF values for the liver and donor-DNA fractions could be observed (; R=0.74, P=0.0022, Pearson correlation).

In addition, we also retrieved the plasma DNA sequencing data from a previously published cohort of hepatocellular carcinoma (HCC) patients (70). For these HCC patients, the tumor DNA fractions in plasma DNA were estimated by copy number aberration analyses (70), although other techniques could be used, such as a tumor specific allele. Through such analyses, 74 HCC plasma samples showed evidence of the presence of tumor DNA in the plasma. Notably, in these HCC patients, the tumor-derived cfDNA molecules were considered to have originated from the liver since they only had tumors in the liver (102, 64).

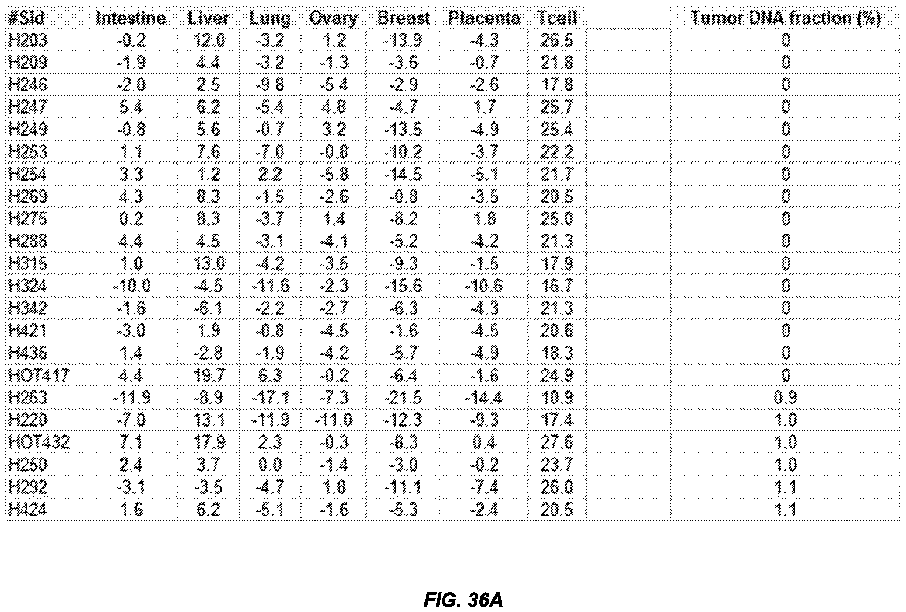

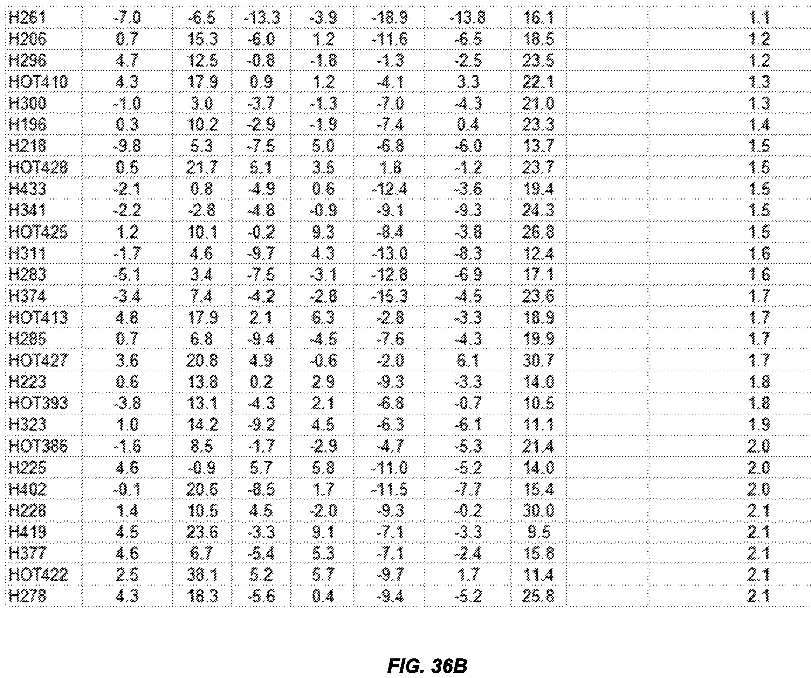

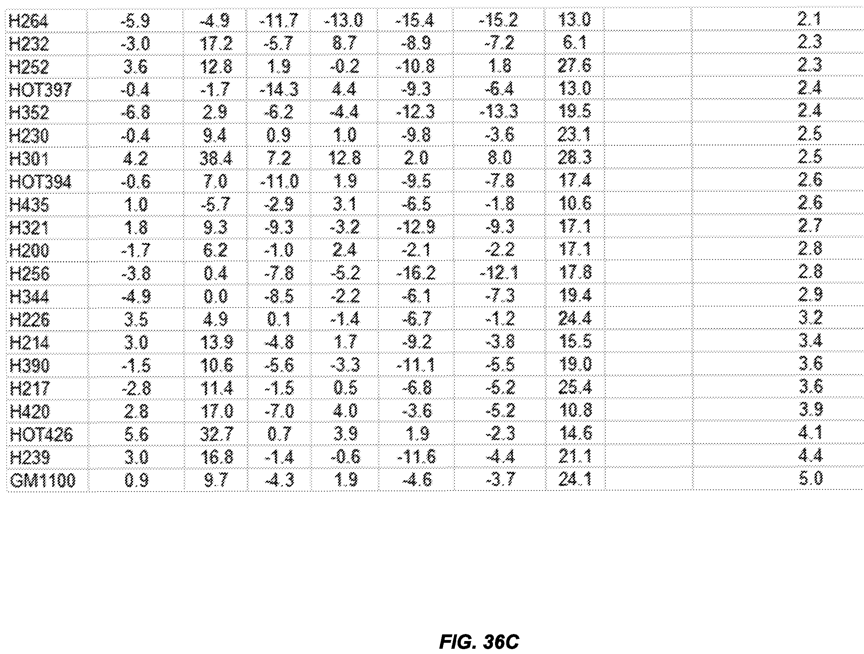

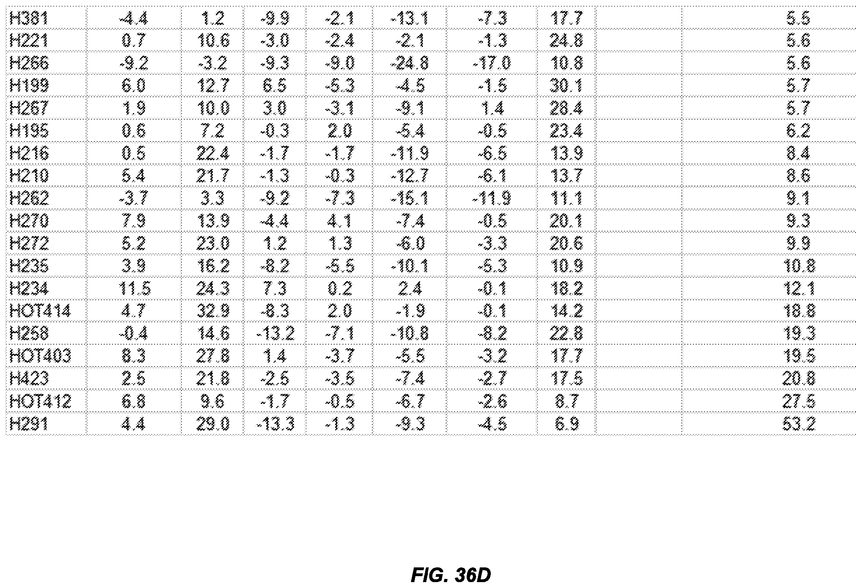

FIG. 35B shows the tumor DNA fraction in HCC cases. FIGS. 36A-36D shows a table of OCF values tissue types in hepatocellular carcinoma patients according to embodiments of the present invention. A positive correlation between OCF values for the liver and the tumor DNA fractions was observed (; R=0.36, P=0.0017, Pearson correlation).

Furthermore, we separated the HCC patients into two subgroups based on the tumor DNA fraction: "low tumor DNA load" group contained those with tumor DNA load lower than 10% and "high tumor DNA load" group for the rest cases. This separation was based on the knowledge that liver contributes -10% plasma DNA in healthy subjects (102).

FIG. 35C shows the comparison of OCF values for T-cells among healthy subjects and HCC cases (separated into 2 groups based on the tumor DNA load in plasma). As shown in FIG. 35C, when compared to the healthy subjects, OCF values for T-cells were significantly decreased for both HCC patient groups (P=0.0035 and P<0.001 for low and high tumor DNA load group, respectively, Mann-Whitney rank-sum test). As explained herein, the contribution by the T-cells would go down when a significant change in contribution occurs from other tissue; liver in this case.

FIG. 35D shows the comparison of OCF values for the liver among healthy subjects and HCC cases (separated into 2 groups based on the tumor DNA load in plasma). OCF values for the liver in FIG. 35D showed no statistical difference in low tumor DNA load group patients (P=0.080, Mann-Whitney rank-sum test) while were significantly elevated in high tumor DNA load group patients (P<0.001, Mann-Whitney rank-sum test). Taken together, these results shows that present techniques have application in liver transplantation monitoring and cancer testing.

**4. Application in colorectal cancer and lung cancer patients**

A cohort of 11 colorectal cancer (CRC) patients was newly recruited in this study. For each case, the plasma DNA was bisulfite sequenced (see Materials and Methods section) such that the colonic contribution could be determined using the plasma DNA tissue mapping approach (102). These results allowed us to explore the use of cfDNA fragmentation pattern analysis in BS-seq data. In the plasma DNA of such individuals, we observed characteristic fragmentation patterns in the intestine-specific open chromatin regions, which corresponded to nucleosome-depletion in the center and well-phased nucleosome arrays nearby.

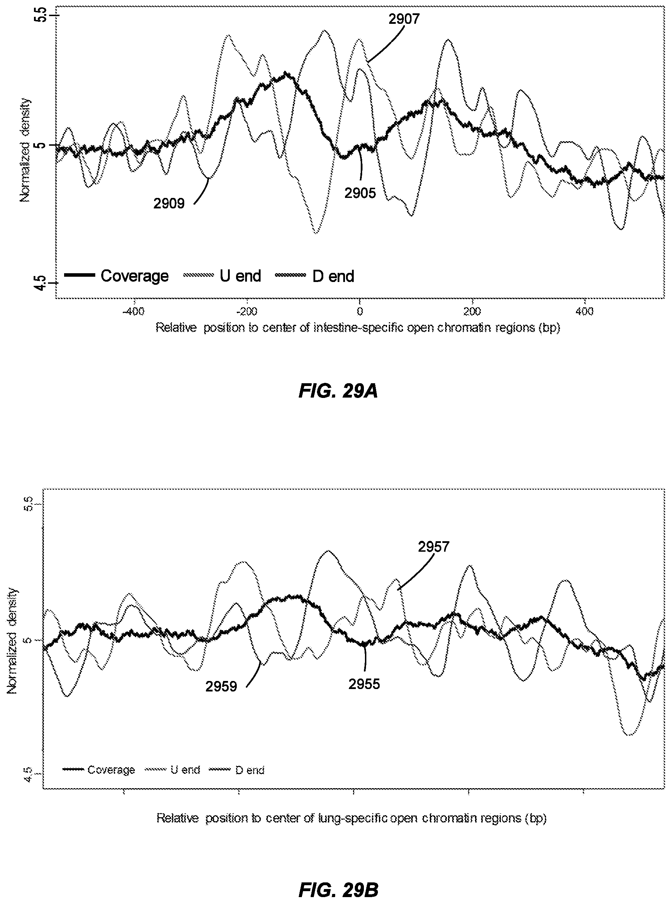

FIG. 29A shows the plasma DNA fragmentation pattern in intestine-specific open chromatin regions in one CRC patient according to embodiments of the present invention. The genomic coverage 2905 shows a dip at the center of the open chromatin region in a similar manner as in FIGS. 27A, 28A, and 28B when tissue exists having the tested open chromatin regions. Further, the U ending signals 2907 and the D ending signals 2909 show a phasing difference that would result in a positive OCF value.

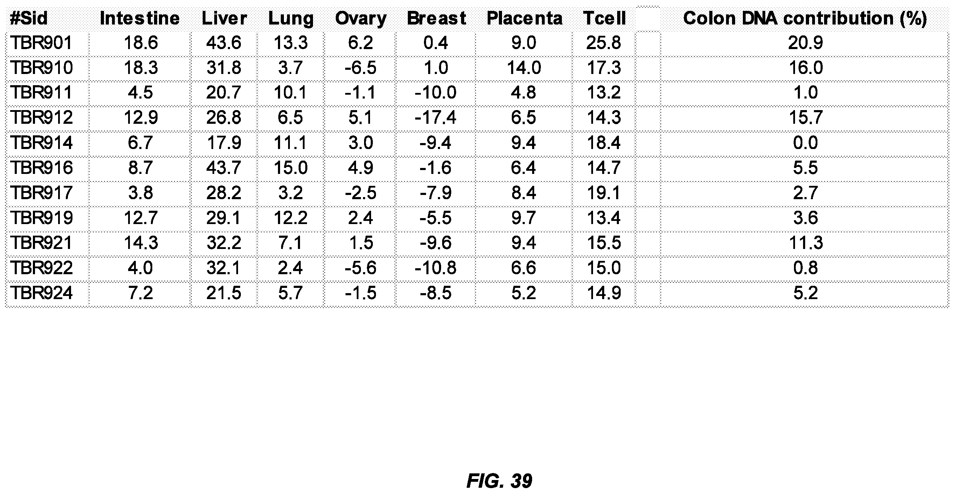

FIG. 37A shows a comparison of OCF values for T-cells between healthy subjects and CRC patients. FIG. 37B shows a comparison of OCF values for intestines between healthy subjects and CRC patients. FIG. 39 shows a table of OCF values tissue types in colorectal cancer patients according to embodiments of the present invention. The colonic DNA contribution is also provided in FIG. 39.

The OCF values for the T-cells are reduced for the CRC patients, as would be expected when there is an increase in the contribution from another tissue. FIG. 37B shows the corresponding increase in the OCF values for the intestine open chromatin regions (28,456 were used). Accordingly, when compared to the healthy subjects, OCF values for the T-cells were significantly decreased while OCF values for the intestines were significantly elevated in the CRC patients (FIG. 37A and 37B; both P<0.001, Mann-Whitney rank-sum test).

FIG. 37C shows the correlation between OCF values for intestines and colonic DNA fractions (deduced by plasma DNA tissue mapping method) in CRC patients. The colonic contribution was determined using the plasma DNA tissue mapping approach (102). A positive correlation between OCF values for the intestines and colonic contributions (as measured using the plasma DNA tissue mapping approach (102)) could be observed (FIG. 37C; R=0.89, P<0.001, Pearson correlation).

In addition, plasma DNA sequencing data for 9 lung cancer patients were retrieved from the dataset generated by Snyder et al (98). We found that plasma DNA showed the characteristic fragmentation, i.e., differentially phased end signatures of central nucleosome-depletion regions, flanked by well-phased nucleosome arrays in the lung-specific open chromatin regions in these patients.

FIG. 29B shows the plasma DNA fragmentation pattern in lung-specific open chromatin regions in one lung cancer patient according to embodiments of the present invention. The genomic coverage 2955 shows a dip at the center of the open chromatin region in a similar manner as in FIGS. 27A, 28A, and 28B when tissue exists having the tested open chromatin regions. Further, the U ending signals 2957 and the D ending signals 2959 show a phasing difference that would result in a positive OCF value.

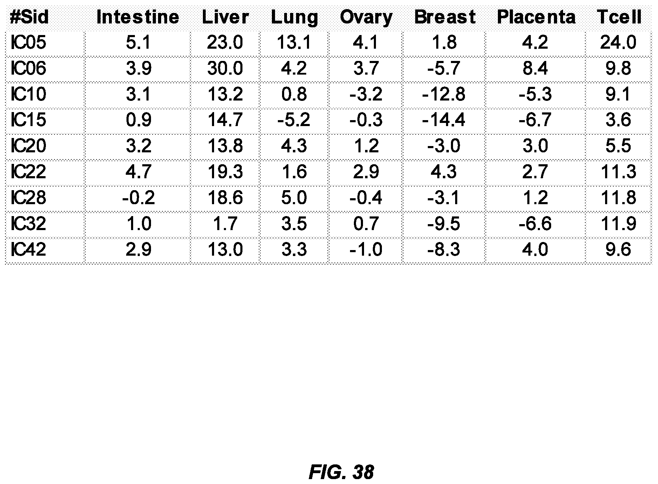

FIG. 37D shows a comparison of OCF values for T-cells between healthy subjects and lung cancer patients. FIG. 37E shows a comparison of OCF values for lungs between healthy subjects and lung cancer patients. FIG. 38 shows a table of OCF values tissue types in lung cancer patients according to embodiments of the present invention.

The OCF values for the T-cells are reduced for the lung cancer patients, as would be expected when there is an increase in the contribution from another tissue. FIG. 37E shows the corresponding increase in the OCF values for the lung open chromatin regions (19,701 were used). Accordingly, OCF values for the T-cells were decreased while OCF values for the lungs were elevated compared to the healthy individuals ( $P<0.001$ and 0.025 for T-cells and lungs, respectively, Mann-Whitney rank-sum test).

*E. Orientations-Aware techniques*

As described above, techniques for nucleosome positioning profiling using an orientation-aware analysis of open chromatin regions are provided, as well as quantitative determination of the relative contributions of various tissues in plasma DNA by such fragmentation pattern analyses. We also demonstrated the diagnostic ability of using orientation-aware analysis of tissue-specific open chromatin region(s) in noninvasive prenatal testing, organ transplantation monitoring, as well as cancer testing. We showed that plasma DNA fragmentation pattern analysis bore characteristic profiles in the nucleosome-depleted region and well-phased nucleosome arrays around the open chromatin regions..

**1. Summary of example results orientation-Aware analysis**

The ability to trace the tissue-of-origin of cfDNA is of great interest in liquid biopsy, especially in predicting the tumor-of-origin in cancer patients. We showed that by quantifying the plasma DNA fragmentation patterns in cancer patients, OCF values for T-cells would decrease while OCF values for the tissue-of-origin of the tumor would increase (e.g., FIGS. 32B, 32C, 35C, 35D, 37A, 37B, 37D, and 37E). These observations were consistent with the fact that, in these patients, the tumor tissues (and peri-tumoral tissues) release DNA into the plasma which: (i) would increase the contribution from that tissue of origin of the cancer, and (ii) would dilute the contribution of the hematopoietic system. In addition, the results on the CRC cases (FIG. 37C) showed that our approach was highly concordant with the plasma DNA tissue mapping method (102).

It is interesting to note that the plasma DNA fragmentation patterns were preserved among the bisulfite-converted DNA. This is likely to be partly related to our library preparation protocol whereby sequencing adaptors were first ligated to plasma DNA molecules before bisulfite treatment (85). Some embodiments may provide additive value by using both OCF measurement and methylation-based tissue mapping in a synergistic manner to further enhance the performance of the tissue-of-origin analysis. Here, we demonstrated that OCF analysis is an approach that provides tissue-of-origin information without reliance of methylation analysis. This can provide cost savings. Compared to bisulfite sequencing (BS-seq), standard DNA sequencing experiments are cheaper and involve simpler protocols.

As to a further efficiency improvement, Ulz et al. had demonstrated the potential of plasma DNA coverage pattern analysis in inferring the expression of genes thus revealing the tissue-of-origin of tumors in cancer patients (105). However, the authors estimated that a 75% tumor DNA fraction in the plasma might be required for this purpose (105), which was difficult to achieve in most clinical cases. In contrast, present techniques can work on cases with a much lower fraction of DNA from the tissue of interest. For instance, in CRC cases, higher OCF values for intestines than that in healthy individuals were already apparent when the colon contribution was only 5%, as can be seen in FIGS. 37A, 37B, and 39. Thus, these results suggest that these techniques can work on relatively early cancer cases where the tumor DNA load in the plasma might not be high.

Embodiments could be integrated with targeted massively parallel sequencing technology (87) to analyze plasma DNA. Since the tissue-specific open chromatin regions only accounted for a very small proportion of the human genome, through designing hybridization probes to capture these regions, the cost could be largely reduced.

Embodiments may include treating the disease or condition in the patient after determining the level of the disease or condition in the patient. Treatment may include any suitable therapy, drug, chemotherapy, radiation, or surgery, including any treatment described in a reference mentioned herein.

**2. Determining Proportional Contribution of tissue type**

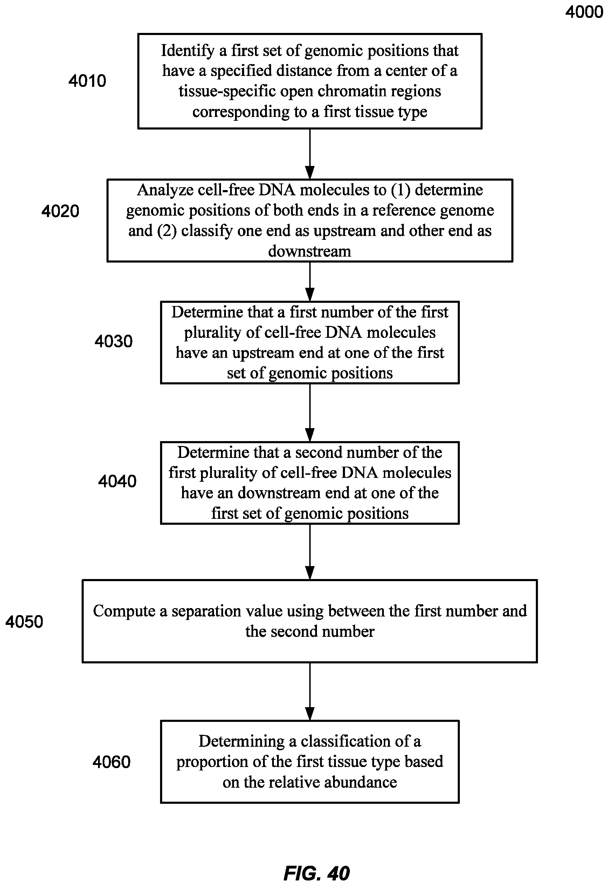

FIG. 40 is a flowchart of a method 4000 of analyzing a biological sample to determine a classification of a proportional contribution of the first tissue type in a mixture according to embodiments of the present disclosure. The biological sample includes a mixture of cell-free DNA molecules from a plurality of tissues types that includes a first tissue type. As with other methods described herein, method 4000 can use a computer system. Examples of the first tissue type include fetal tissue, tumor tissue, and tissue from a transplanted organ. Aspects of method 4000 may be performed in a similar manner as methods 2300 and 2400.

At block 4010, a first set of genomic positions are identified that have a specified distance from a center of one or more tissue-specific open chromatin regions corresponding to the first tissue type. The tissue-specific open chromatin regions can be identified by analyzing tissue samples of the first tissue type, e.g., liver, T-cells, colon, ovaries, breast, etc.. The set of genomic positions can be specified as a range of distances. As examples, the number of tissue-specific open chromatin regions can be at least 500, 1000, 2000, 5000, 10,000, 20,000, 30,000, 40,000, 50,000, or more.

As examples, the specified distance can be +/- X base pairs from the center, including a range (window) of values, as described herein. Accordingly, the specified distance can include a first range of distances before the center and includes a second range of distances after the center. Such a set can be define by an offset from the center, and a window around the offset. Example values for the offset are 40, 45, 50, 55, 60, 65, 70, and 75 bp. Other example values for the window are 2, 3, 4, 5, 6, 7, 8, 9, 10, 15, 20, 25, and 30 bp. The ranges may be asymmetric or symmetric.

At block 4020, a first plurality of cell-free DNA molecules from the biological sample of a subject is analyzed. The analyzing of a cell-free DNA molecule can include determining a genomic position (ending position) in a reference genome corresponding to both ends of the cell-free DNA molecule. The analyzing can also include classifying one end as an upstream end and another end as a downstream end based on which end has a lower value for the genomic position, e.g., as defined in the reference genome. Various alignment/mapping procedures can be used to determine the genomic positions of the ends. Aspects of block 4020 can be performed in a similar manner as block 2320 of method 2300.

At block 4030, it is determined that a first number of the first plurality of cell-free DNA molecules have an upstream end at one of the first set of genomic positions. The determination is performed based on the analyzing of the first plurality of cell-free DNA molecules. Given the first set of positions can be defined as specific genomic coordinates in a reference genome, once a sequence read(s) of a DNA fragment are aligned, the upstream end positions can be compared to the first set to determine whether that end position falls within the first set.

At block 4040, it is determined that a second number of the first plurality of cell-free DNA molecules have a downstream end at one of the first set of genomic positions. The determination is performed based on the analyzing of the first plurality of cell-free DNA molecules. Given the first set of positions can be defined as specific genomic coordinates in a reference genome, once a sequence read(s) of a DNA fragment are aligned, the downstream end positions can be compared to the first set to determine whether that end position falls within the first set.

At block 4050, a separation value is computed using the first number and the second number. The separation value can be determined in a variety of ways and may include a ratio and/or a difference. The separation value may be composed of multiple contributions. In embodiments where two ranges are used (e.g., on either side of the center of a tissue-specific open chromatin region corresponding to the first tissue type), the separation value can have a first contribution to the separation value determined in a first manner (e.g., a first formula) for the first range, and a second contribution to the separation value determined in a second manner (e.g., a second formula) for the second range.

In one example, the separation value can be an OCF value, e.g., as defined by

$$OCF = \sum_{-peak-bin}^{-peak+bin}(D-U) + \sum_{peak-bin}^{peak+bin}(U-D),$$

where D is a number downstream and U is a number upstream. A peak position can corresponds to an offset from the center and a bin value corresponds to a window size around the peak. Such a sum can be performed over each position. Such a sum can be performed in any order, e.g., determining a total for D for one peak and a total U for that peak. Contributions can be determined for one or two peaks around each center. One peak can be identified as a downstream peak, where more downstream ending positions are expected. Another peak can be identified as an upstream peak, where more upstream ending positions are expected. When two peaks are used, two downstream and two upstream numbers can be determined and used, e.g., as in the formula above. As a further example, a separation value can be determined for each position, with a specified formula used for that position, e.g., depending on which peak the position is associated a different formula may be used for that position. Thus, each position of the first set may have a contribution defined by a formula including a first number of cell-free DNA fragments having an upstream end at that position and a second number of cell-free DNA fragments having an downstream end at that position.

In a particular embodiment, the first range is between 50 and 70 bases less than the center and the second range is between 50 and 70 bases, and wherein the separation value includes:

$$OCF = \sum_{-60-10}^{-60+10}(D-U) + \sum_{60-10}^{60+10}(U-D),$$

where U is a first number and D is a second number.

The first number can be a value U at one of the positions in the first set (e.g., a partiocular position in a first range or a second range) and the second number can be a value D at that same position. As another example, the first number can be a sum of the numbers of cell-free DNA having an upstream end in a first range (e.g., corresponding to an upstream or a downstream peak), and the second number can be a sum of the numbers of cell-free DNA in the same first range. The separation value can be determined using pairs of numbers from each of the ranges. For example, a third number of cell-free DNA having an upstream end at a position in a second range (e.g., second sumamtion contribution in OCF formual above) can be determined, and a fourth numebr of cell-free DNA having a downstream end at a position in the second range can be determined. A second contribution to the separation value can be determined using the third and fourth numbers, e.g., as provided above.

Other example separation values can include ratios of sums instead of differences. For exmaple, a sum of D ends in a peak region divided by a sume of U ends for the peak region, or other ratio of the two numbers, such as the numerator or the denominator being a total amount of reads having either end in the peak region). For instance, the separation value can includes a ratio of the first number and the second number. When more than one peak is used, a ratio (or other function) can be determined differently for each peak.

At block 4060, the classification of the proportional contribution of the first tissue type is determined by comparing the separation value to one or more calibration values determined from one or more calibration samples whose proportional contributions of the first tissue type are known. Examples are shown in FIG. 32D for fetal tissue being the first tissue type, FIG. 35A for donor DNA from a transplanted liver organ, and FIG. 35B for tumor DNA from a liver being the first tissue type. As an example, the classification of the proportional contribution can correspond to a range above a specified percentage. Another example can correspond to an existence of cancer as well as other examples are provided herein, e.g., for block 2350, as well as further actions such as treatment, as described herein. Aspects of block 4060 may be performed in a similar manner as block 2350, e.g., relating to values for the classification and the comparison to the calibration values, as well as later treatments steps.

### 3. Determining Pathology

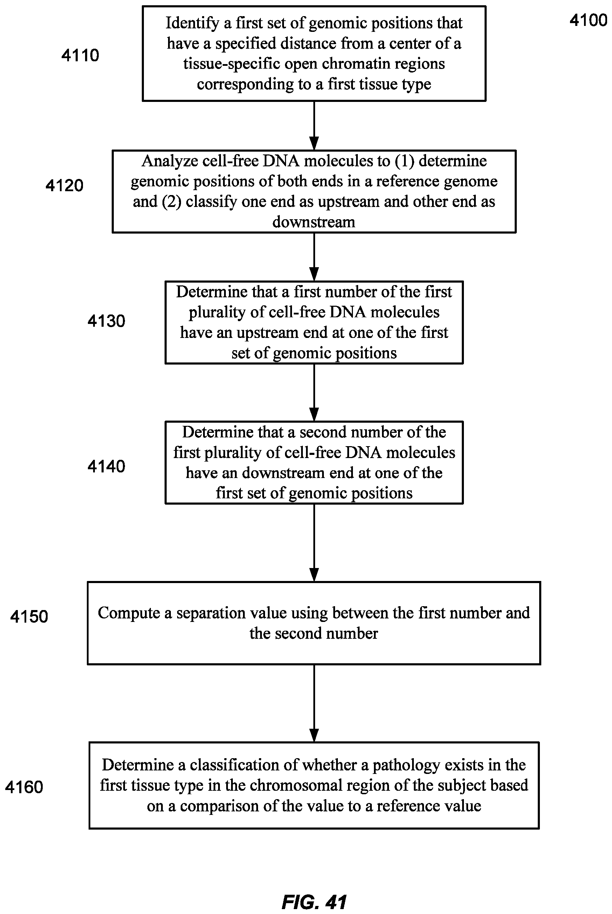

FIG. 41 is a flowchart of a method 4100 of analyzing a biological sample to determine a classification of whether a pathology exists for the first tissue type in the mixture according to embodiments of the present disclosure. The biological sample includes a mixture of cell-free DNA molecules from a plurality of tissues types that includes a first tissue type. As with other methods described herein, method 4100 can use a computer system. Examples of the first tissue type include tumor tissue, and tissue from a transplanted organ. Aspects of method 4100 may be performed in a similar manner as methods 2300, 2400, and 4100.

At block 4110, a first set of genomic positions are identified that have a specified distance from a center of one or more tissue-specific open chromatin regions corresponding to the first tissue type. Block 4110 may be performed in a similar manner as block 4010 of FIG. 40.

At block 4120, a first plurality of cell-free DNA molecules from the biological sample of a subject is analyzed. The analyzing of a cell-free DNA molecule can include determining a genomic position (ending position) in a reference genome corresponding to both ends of the cell-free DNA molecule. The analyzing can also include classifying one end as an upstream end and another end as a downstream end based on which end has a lower value for the genomic position, e.g., as defined in the reference genome. Block 4120 may be performed in a similar manner as block 4020 of FIG. 40.

At block 4130, it is determined that a first number of the first plurality of cell-free DNA molecules have an upstream end at one of the first set of genomic positions. Block 4130 may be performed in a similar manner as block 4030 of FIG. 40.

At block 4140, it is determined that a second number of the first plurality of cell-free DNA molecules have a downstream end at one of the first set of genomic positions. Block 4140 may be performed in a similar manner as block 4040 of FIG. 40.

At block 4150, a separation value is computed using the first number and the second number. Block 4150 may be performed in a similar manner as block 4050 of FIG. 40.

At block 4160, a classification of whether a pathology exists for the first tissue type of the subject is determined based on a comparison of the separation value to a reference value. As examples, block 4160 may use a reference value determined using training samples having a known classification, whose separation values (e.g., OCF) have been measured. FIGS. 37B and 37E provides an example set of training samples, where the pathology is cancer from a particular tissue, namely lungs. Thus, a pathology may be cancer of the first tissue type. A level of cancer may also be more specifically determined, e.g., as shown in FIGS. 35C or 35D.

Accordingly, the reference value may be determined from one or more control samples that do not have the pathology, and/or from one or more control samples that do have the pathology.

Another example of pathology is a rejection of a transplanted organ. If a transplanted organ is rejected, the fractional concentration of DNA from that organ will increase to abnormal levels. Another example of a pathology is an abnormally high fractional concentration of cell-free DNA from the first tissue type. Other example pathologies can include autoimmune attack ( e.g., lupus nephritis damaging the kidney), inflammatory diseases (e.g., hepatitis), and ischemic tissue damage (e.g., myocardial infarction). A heathy state of a subject can be considered a classification of no pathology.

## VIII. MATERIALS AND METHODS

### A. Sample processing.

Peripheral blood was collected in EDTA-containing tubes and centrifuged at 1,600 x g for 10 min at 4 °C. The plasma portion was recentrifuged at 16,000 x g for 10 min at 4 °C to obtain cell-free plasma and stored at -80 °C. The white and red blood cells portions were treated with ACK Lysing Buffer (Gibco) in a 1:10 ratio for 5 min at room temperature to remove the red blood cells. The mixture was centrifuged at 300 x g for 10 min at 4 °C. Supernatants with lysed red blood cells were discarded and white cell pellet was washed with phosphate buffered saline (Gibco). The white blood cell portion was recentrifuged at 300 x g for 10 min at 4 °C to remove residual red blood cells. Approximately 50,000 cells were used for downstream ATAC-seq library preparation.

Tissues from a placenta were collected and washed with phosphate buffered saline (Gibco) and then disaggregated into a single cell solution by Medimachine (BD Biosciences). Positive selection of syncytiotrophoblasts and cytotrophoblasts from the placental tissue were processed with an antibody towards CD105 (Miltenyi Biotec) and an antibody towards HAI-I (Abcam), respectively. Homogenized placental cells were resuspended in 80 $\mu$L of 0.5% bovine serum albumin buffer by diluting the MACS BSA Stock Solution (Miltenyi Biotec) with phosphate buffered saline (Gibco). To isolate syncytiotrophoblasts, 20 $\mu$L of CD105 MicroBeads (Miltenyi Biotec) was added and incubated for 15 min at 4 °C. After binding of syncytiotrophoblasts onto antibody-coated beads, we washed the cells by adding 2 mL of buffer and centrifuged at 200 x g for 10 minutes. Labeled cells were resuspended in 500 $\mu$L of buffer for the isolation step. To isolate cytotrophoblasts, 20 $\mu$L of the HAI-I antibody (Abcam) and 80 $\mu$L of buffer were added to homogenized placenta tissues and incubated for 15 minutes at 4 °C. After incubation, 2 mL of buffer was added to wash away excess primary antibody by centrifuging at 200 x g for 10 minutes. Cells were resuspended in 80 $\mu$L of buffer and 20 $\mu$L of secondary anti-mouse IgG MicroBeads (Miltenyi Biotec) was added and incubated for 15 minutes at 4 °C. Similar to the first antibody, 2 mL of buffer was added to wash away excess primary antibody by centrifuging at 200 x g for 10 minutes. Labeled cells were resuspended in 500 $\mu$L of buffer for the isolation step. Each sample for each cell type used one MS column (Miltenyi Biotec). We rinsed the column 500 $\mu$L of buffer before we applied the labeled cells. By applying the cells into the column, the labeled cells were attached onto the magnetic beads in the column and unlabeled cells were left in the flow-through. We washed the column 3 times with 500 $\mu$L buffer each time. The sorted syncytiotrophoblasts and cytotrophoblasts were eluted in 1 mL of buffer and counted by a hemocytometer to aliquot 50,000 cells per sample for ATAC-seq.

### B. ATAC-seq libraries preparation and sequencing.

ATAC-seq was performed as described (35). Briefly, 50,000 cells were spun at 500 x g for 5 minutes at 4 °C and followed by a cell lysis using cold lysis buffer (10 mM Tris-HCl, pH 7.4 (Ambion), 10 mM NaCl (Ambion), 3 mM MgCl$_2$ (Ambion) and 0.1% IGEPAL CA-630 (Sigma)). The mixture was immediately centrifuged at 500 x g for 10 minutes at 4 °C. The nuclei were resuspended in a transposase reaction mixture which contained 25 $\mu$L 2x TD buffer, 2.5 $\mu$L transposase from Nextera DNA Library Preparation Kit (Illumina) and 22.5 $\mu$L nuclease-free water. Transposition and tagmentation were carried out at 37 °C for 30 minutes. The sample was purified with Qiagen MinElute Kit (Qiagen) immediately after transposition following manufacturer's instruction. Purified DNA fragments were mixed with 1x NEBnext PCR master mix (New England BioLabs) and 1.25 $\mu$M of Nextera PCR primers 1 and 2 (IDT) for PCR amplification using the following conditions: 72 °C for 5 minutes; 98 °C for 30 s; thermocycling for 15 cycles at 98 °C for 10 s, 63 °C for 30 s and 72 °C for 1 minute. The libraries were purified with Qiagen PCR cleanup kit (Qiagen). The libraries were analyzed by a 2100 Bioanalyzer (Agilent) and quantified by the KAPA Library Quantification Kit (Kapa Biosystems) before sequencing. 2 x 75 paired-end sequencing was performed on Hi-Seq 2500 (Illumina).

### C. Alignment of sequencing data.

In examples, the paired-end reads were mapped to the reference human genome (NCBI37/hg19) using the SOAP2 aligner (53) in paired-end mode, allowing two mismatches for the alignment for each end. Only paired-end reads with both ends aligned to the same chromosome with the correct orientation, spanning an insert size of $\leq$ 600 bp were used for downstream analysis. Other alignment techniques (software) may be used, such as BLAST,BLAT, BWA, Bowtie, STAR, etc. If the entire DNA fragment is sequenced, then a paired-end mode is not needed. Further, the number of mismatches can be varied depending on a desired accuracy.

### D. Plasma DNA data collection and availability

Plasma data for healthy individuals, HCC patients and pregnant cases were retrieved from the European Genome-Phenome Archive (EGA; accession no. EGAS00001001024 and EGAS00001001882) (70, 55). Plasma DNA sequencing data for the liver transplantation patients as described in our previous work (64) had been deposited at the EGA (accession no. EGAS00001003116). Plasma DNA sequencing data for the lung cancer cases were obtained from Gene Expression Omnibus (GEO; accession no. GSE71378) (98).

Colorectal cancer patients were newly recruited in this study. Peripheral blood samples were collected into EDTA-containing tubes. Blood samples were centrifuged at 1,600 $\times$ g for 10 min at 4 °C. The plasma portion was harvested and recentrifuged at 16,000 $\times$ g for 10 min at 4 °C to remove the blood cells. Bisulfite conversion was performed as previously described (85). DNA libraries were prepared using the KAPA HTP Library Preparation Kit (Kapa Biosystems) according to the manufacturer's instructions (56) and sequenced on a HiSeq 2000 system (Illumina) in 75 $\times$ 2 (paired-end mode) cycles mode with the TruSeq SBS Kit v3 (Illumina). Analysis of the BS-seq data, including quality control, sequence alignment, methylation status determination and colon contribution inference were performed as previously described (71, 102). The median sequencing depth was 3.2x (range: 0.6-6.4x; FIG. 39) haploid human genome coverage for these samples.

*E. Tissue-specific open chromatin regions*

Open chromatin regions are important regulatory elements in the genome and are highly tissue-specific. Active promoter is one type of open chromatin regions. Other types include enhancers and insulators. The open chromatin regions may be determined using public Dnase-seq data for the tissues of interest. Dnase-seq is an experimental procedure that uses the DNaseI endonuclease enzyme to treat the cellular genomic DNA, which prefers cutting the non-nucleosome bound DNA. As a result, the DNA in the open chromatin regions are cut and gathered for sequencing. Therefore, we could identify these DNA coordinates as open chromatin regions, e.g., as shown in FIG. 25D. For each region, the genomic coordinates for its start and end are obtained, and a middle coordinate (i.e., (start +end)/2) can be used as the center.

After obtaining the open chromatin regions from Dnase-seq data for each tissue type, the open chromatin regions can be compared with each other and only those unique to one tissue type may be kept and defined as "tissue-specific" ones for further analysis, as described herein. For these tissue-specific open chromatin regions, the nucleosomes are only well-positioned in the corresponding tissue type, thus allowing the determination of the proportional contribution in the plasma DNA. Besides Dnase-seq, other example methods to identify the open chromatin regions include FAIRE-seq, ATAC-seq, MNASE-seq, and ChIP-seq on CTCF transcription factor.

In some embodiments, we used the publicly available DNase-seq (DNase I hypersensitive sites sequencing) data to mine the open chromatin regions. DNase-seq data for T-cells, placenta, lungs, ovary, breast and small intestines were obtained from the Road-Map Epigenomics project (93). DNase-seq data for liver and ESC were obtained from the ENCODE project (104).

For each tissue type, the raw sequencing data were downloaded and aligned to the reference human genome (UCSC hg19) using the bowtie alignment software (version 1.1.1) (76). Then, the open chromatin regions were determined using the MACS (Model-based Analysis for ChIP-Seq) software (version 2.0.9) (110, 74). Other reference genomes and alignment software may be used.

For such analyses, the ChIP-seq (chromatin immunoprecipitation followed by massively parallel DNA sequencing) input data were used as negative controls and a Q-value (i.e., adjusted P-value that reflects the false discovery rate) of 0.01 was used as the threshold to call peaks. For the lungs, DNase-seq data for IMR90 (human fetal lung) and HLF (human lung fibroblast) cell lines were both analyzed and only the peaks that existed in both samples were identified. Then, for each tissue type, we compared its peaks with all the other tissues and only kept those unique to this tissue type and within a size range of 50 - 200 bp as the final tissue-specific open chromatin regions.

## IX. EXAMPLE SYSTEMS

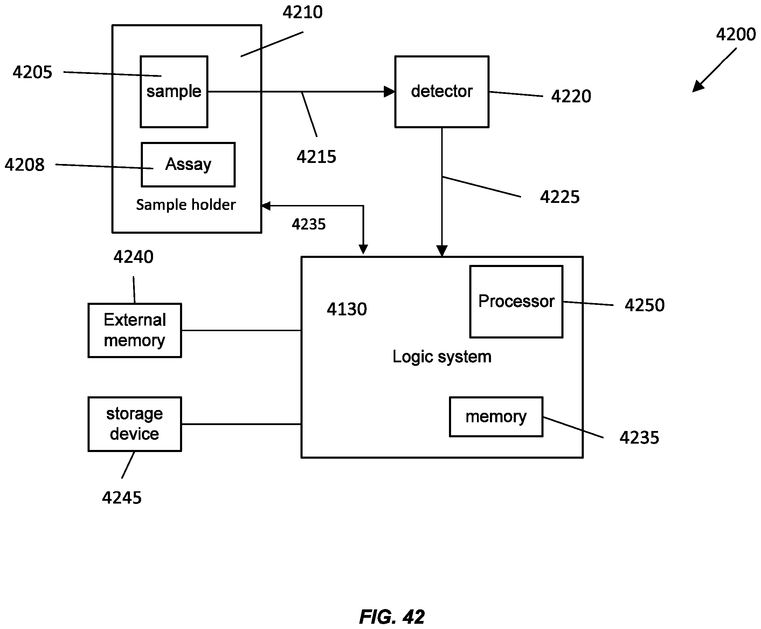

FIG. 42 illustrates a measurement system 4200 according to an embodiment of the present disclosure. The system as shown includes a sample 4205, such as cell-free DNA molecules within a sample holder 4210, where sample 4205 can be contacted with an assay 4208 to provide a signal of a physical characteristic 4215. An example of a sample holder can be a flow cell that includes probes and/or primers of an assay or a tube through which a droplet moves (with the droplet including the assay). Physical characteristic 4215 (e.g., a fluorescence intensity, a voltage, or a current), from the sample is detected by detector 4220. Detector can take a measurement at intervals (e.g., periodic intervals) to obtain data points that make up a data signal. In one embodiment, an analog to digital converter converts an analog signal from the detector into digital form at a plurality of times. Sample holder 4210 and detector 4220 can form an assay device, e.g., a sequencing device that performs sequencing according to embodiments described herein. A data signal 4225 is sent from detector 4220 to logic system 4230. Data signal 4225 may be stored in a local memory 4235, an external memory 4240, or a storage device 4245.

Logic system 4230 may be, or may include, a computer system, ASIC, microprocessor, etc. It may also include or be coupled with a display (e.g., monitor, LED display, etc.) and a user input device (e.g., mouse, keyboard, buttons, etc.). Logic system 4230 and the other components may be part of a stand-alone or network connected computer system, or they may be directly attached to or incorporated in a device (e.g., a sequencing device) that includes detector 4220 and/or sample holder 4210. Logic system 4230 may also include software that executes in a processor 4250. Logic system 4230 may include a computer readable medium storing instructions for controlling system 4200 to perform any of the methods described herein. For example, logic system 4230 can provide commands to a system that includes sample holder 4210 such that sequencing or other physical operations are performed. Such physical operations can be performed in a particular order, e.g., with reagents being added and removed in a particular order. Such physical operations may be performed by a robotics system, e.g., including a robotic arm, as may be used to obtain a sample and perform an assay.

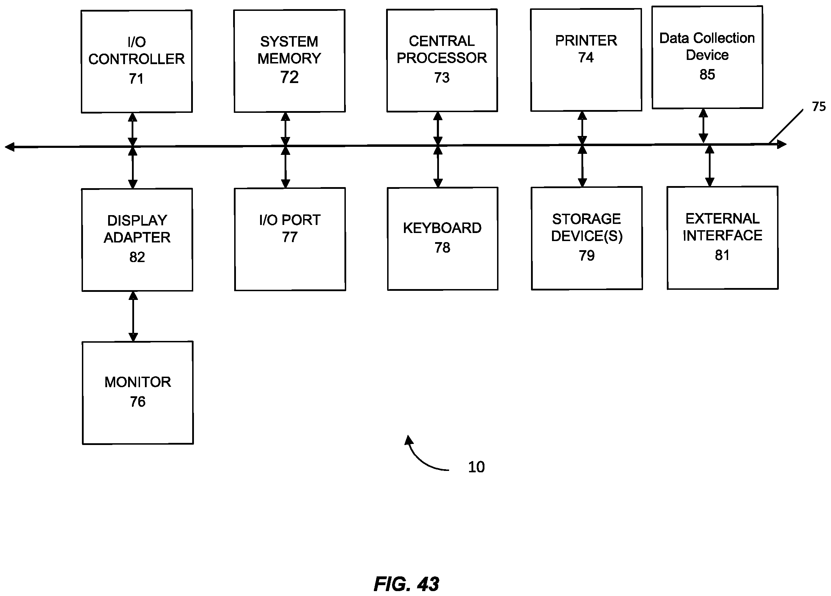

Any of the computer systems (e.g., logic system 4230) mentioned herein may utilize any suitable number of subsystems. Examples of such subsystems are shown in FIG. 43 in computer system 10. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

The subsystems shown in FIG. 43 are interconnected via a system bus 75. Additional subsystems such as a printer 74, keyboard 78, storage device(s) 79, monitor 76, which is coupled to display adapter 82, and others are shown. Peripherals and input/output (I/O) devices, which couple to I/O controller 71, can be connected to the computer system by any number of means known in the art such as input/output (I/O) port 77 (e.g., USB, FireWire®). For example, I/O port 77 or external interface 81 (e.g. Ethernet, Wi-Fi, etc.) can be used to connect computer system 10 to a wide area network such as the Internet, a mouse input device, or a scanner. The interconnection via system bus 75 allows the central processor 73 to communicate with each subsystem and to control the execution of a plurality of instructions from system memory 72 or the storage device(s) 79 (e.g., a fixed disk, such as a hard drive, or optical disk), as well as the exchange of information between subsystems. The system memory 72 and/or the storage device(s) 79 may embody a computer readable medium. Another subsystem is a data collection device 85, such as a camera, microphone, accelerometer, and the like. Any of the data mentioned herein can be output from one component to another component and can be output to the user.

A computer system can include a plurality of the same components or subsystems, e.g., connected together by external interface 81, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g.

an application specific integrated circuit or field programmable gate array) and/or using computer software with a generally programmable processor in a modular or integrated manner. As used herein, a processor can include a single-core processor, multi-core processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present disclosure using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a hard-drive or a floppy disk, or an optical medium such as a compact disk (CD) or DVD (digital versatile disk), flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order. Additionally, portions of these steps may be used with portions of other steps from other methods. Also, all or portions of a step may be optional. Additionally, any of the steps of any of the methods can be performed with modules, units, circuits, or other means of a system for performing these steps.

A recitation of "a", "an" or "the" is intended to mean "one or more" unless specifically indicated to the contrary. The use of "or" is intended to mean an "inclusive or," and not an "exclusive or" unless specifically indicated to the contrary. Reference to a "first" component does not necessarily require that a second component be provided. Moreover reference to a "first" or a "second" component does not limit the referenced component to a particular location unless expressly stated. The term "based on" is intended to mean "based at least in part on."

None of the patents, patent applications, publications, and descriptions mentioned herein are admitted to be prior art.

## X. REFERENCES

1. Lo YMD, et al. (1997) Presence of fetal DNA in maternal plasma and serum. Lancet 350(9076):485-487.

2. Lo YMD, et al. (1998) Presence of donor-specific DNA in plasma of kidney and liver-transplant recipients. Lancet 351(9112):1329-1330.

3. Ulz P, Heitzer E, Geigl JB, & Speicher MR (2017) Patient monitoring through liquid biopsies using circulating tumor DNA. Int J Cancer 141(5):887-896.

4. Cohen JD, et al. (2018) Detection and localization of surgically resectable cancers with a multi-analyte blood test. Science 359(6378):926-930.

5. Schutz E, et al. (2017) Graft-derived cell-free DNA, a noninvasive early rejection and graft damage marker in liver transplantation: A prospective, observational, multicenter cohort study. PLoS Med 14(4):e1002286.

6. Chan KCA, et al. (2017) Analysis of plasma Epstein-Barr virus DNA to screen for nasopharyngeal cancer. N Engl J Med 377(6):513-522.

7. Lehmann-Werman R, et al. (2016) Identification of tissue-specific cell death using methylation patterns of circulating DNA. Proc Natl Acad Sci U S A 113(13):E1826-1834.

8. van Opstal D, et al. (2017) Origin and clinical relevance of chromosomal aberrations other than the common trisomies detected by genome-wide NIPS: results of the TRIDENT study. Genet Med Oct 2. doi: 10.1038/gim.2017.132.

9. Lo YMD, et al. (2010) Maternal plasma DNA sequencing reveals the genome-wide genetic and mutational profile of the fetus. Sci Transl Med 2(61):61ra91.

10. Struhl K & Segal E (2013) Determinants of nucleosome positioning. Nat Struct Mol Biol 20(3):267-273.

11. Chim SSC, et al. (2005) Detection of the placental epigenetic signature of the maspin gene in maternal plasma. Proc Natl Acad Sci U S A 102(41):14753-14758.

12. Sun K, et al. (2015) Plasma DNA tissue mapping by genome-wide methylation sequencing for noninvasive prenatal, cancer, and transplantation assessments. Proc Natl Acad Sci U S A 112(40):E5503-5512.

13. Lui YYN, et al. (2002) Predominant hematopoietic origin of cell-free DNA in plasma and serum after sex-mismatched bone marrow transplantation. Clin Chem 48(3):421-427.

14. Chan KCA, et al. (2004) Size distributions of maternal and fetal DNA in maternal plasma. Clin Chem 50(1):88-92.

15. Sun K, et al. (2018) Noninvasive reconstruction of placental methylome from maternal plasma DNA: potential for prenatal testing and monitoring. Prenat Diagn 38(3):196-203.

16. Sun K, et al. (2017) COFFEE: control-free noninvasive fetal chromosomal examination using maternal plasma DNA. Prenat Diagn 37(4):336-340.

17. Yu SCY, et al. (2014) Size-based molecular diagnostics using plasma DNA for noninvasive prenatal testing. Proc Natl Acad Sci U S A 111(23):8583-8588.

18. Cirigliano V, Ordonez E, Rueda L, Syngelaki A, & Nicolaides KH (2017) Performance of the neoBona test: a new paired-end massively parallel shotgun sequencing approach for cell-free DNA-based aneuploidy screening. Ultrasound Obstet Gynecol 49(4):460-464.

19. Zhang L, Zhu Q, Wang H, & Liu S (2017) Count-based size-correction analysis of maternal plasma DNA for improved noninvasive prenatal detection of fetal trisomies 13, 18, and 21. Am J Transl Res 9(7):3469-3473.

20. Yu SCY, et al. (2013) High-resolution profiling of fetal DNA clearance from maternal plasma by massively parallel sequencing. Clin Chem 59(8):1228-1237.

21. Chan KCA, et al. (2016) Second generation noninvasive fetal genome analysis reveals de novo mutations, single-base parental inheritance, and preferred DNA ends. Proc Natl Acad Sci U S A 113(50):E8159-E8168.

22. Jahr S, et al. (2001) DNA fragments in the blood plasma of cancer patients: quantitations and evidence for their origin from apoptotic and necrotic cells. Cancer Res 61(4):1659-1665.

23. Straver R, Oudejans CB, Sistermans EA, & Reinders MJ (2016) Calculating the fetal fraction for noninvasive prenatal testing based on genome-wide nucleosome profiles. Prenat Diagn 36(7):614-621.

24. Snyder MW, Kircher M, Hill AJ, Daza RM, & Shendure J (2016) Cell-free DNA comprises an in vivo nucleosome footprint that informs its tissues-of-origin. Cell 164(1-2):57-68.

25. Ivanov M, Baranova A, Butler T, Spellman P, & Mileyko V (2015) Non-random fragmentation patterns in circulating cell-free DNA reflect epigenetic regulation. BMC Genomics 16 Suppl 13:S1.

26. Chiu RWK, et al. (2008) Noninvasive prenatal diagnosis of fetal chromosomal aneuploidy by massively parallel genomic sequencing of DNA in maternal plasma. Proc Natl Acad Sci U S A 105(51):20458-20463.

27. DeLong ER, DeLong DM, & Clarke-Pearson DL (1988) Comparing the areas under two or more correlated receiver operating characteristic curves: a nonparametric approach. Biometrics 44(3):837-845.

28. Jiang P, et al. (2015) Lengthening and shortening of plasma DNA in hepatocellular carcinoma patients. Proc Natl Acad Sci U S A 112(11):E1317-1325.

29. Valouev A, et al. (2011) Determinants of nucleosome organization in primary human cells. Nature 474(7352):516-520.

30. Gaffney DJ, et al. (2012) Controls of nucleosome positioning in the human genome. PLoS Genet 8(11):e1003036.

31. Lam WKJ, et al. (2017) DNA of erythroid origin is present in human plasma and informs the types of anemia. Clin Chem 63(10):1614-1623.

32. Roadmap Epigenomics Consortium, et al. (2015) Integrative analysis of 111 reference human epigenomes. Nature 518(7539):317-330.

33. Jiang C & Pugh BF (2009) Nucleosome positioning and gene regulation: advances through genomics. Nat Rev Genet 10(3):161-172.

34. Horlbeck MA, et al. (2016) Nucleosomes impede Cas9 access to DNA in vivo and in vitro. Elife 5:e12677.

35. Buenrostro JD, Giresi PG, Zaba LC, Chang HY, & Greenleaf WJ (2013) Transposition of native chromatin for fast and sensitive epigenomic profiling of open chromatin, DNA-binding proteins and nucleosome position. Nat Methods 10(12):1213-1218.

36. Mueller B, et al. (2017) Widespread changes in nucleosome accessibility without changes in nucleosome occupancy during a rapid transcriptional induction. Genes Dev 31 (5):451-462.

37. Buenrostro JD, Wu B, Chang HY, & Greenleaf WJ (2015) ATAC-seq: a method for assaying chromatin accessibility genome-wide. Curr Protoc Mol Biol 109:21.29.1-9.

38. Schep AN, et al. (2015) Structured nucleosome fingerprints enable high-resolution mapping of chromatin architecture within regulatory regions. Genome Res 25(11):1757-1770.

39. Chodavarapu RK, et al. (2010) Relationship between nucleosome positioning and DNA methylation. Nature 466(7304):388-392.

40. Jensen TJ, et al. (2015) Whole genome bisulfite sequencing of cell-free DNA and its cellular contributors uncovers placenta hypomethylated domains.

Genome Biol 16:78.

41. Lun FMF, et al. (2013) Noninvasive prenatal methylomic analysis by genomewide bisulfite sequencing of maternal plasma DNA. Clin Chem 59(11):1583-1594.

42. Jiang P, et al. (2017) Gestational age assessment by methylation and size profiling of maternal plasma DNA: a feasibility study. Clin Chem 63(2):606-608.

43. Schroeder DI, et al. (2013) The human placenta methylome. Proc Natl Acad Sci U S A 110(15):6037-6042.

44. Lee JY & Lee TH (2012) Effects of DNA methylation on the structure of nucleosomes. J Am Chem Soc 134(1):173-175.

45. Choy JS, et al. (2010) DNA methylation increases nucleosome compaction and rigidity. J Am Chem Soc 132(6):1782-1783.

46. Collings CK, Waddell PJ, & Anderson JN (2013) Effects of DNA methylation on nucleosome stability. Nucleic Acids Res 41(5):2918-2931.

47. Rose NR & Klose RJ (2014) Understanding the relationship between DNA methylation and histone lysine methylation. Biochim Biophys Acta 1839(12):1362-1372.

48. Soppe WJ, et al. (2002) DNA methylation controls histone H3 lysine 9 methylation and heterochromatin assembly in Arabidopsis. EMBO J 21(23):6549-6559.

49. Simon M, et al. (2011) Histone fold modifications control nucleosome unwrapping and disassembly. Proc Natl Acad Sci U S A 108(31):12711-12716.

50. Ehrlich M (2009) DNA hypomethylation in cancer cells. Epigenomics 1(2):239-259.

51. Chan KCA, et al. (2013) Noninvasive detection of cancer-associated genome-wide hypomethylation and copy number aberrations by plasma DNA bisulfite sequencing. Proc Natl Acad Sci U S A 110(47):18761-18768.

52. Holtan SG, Creedon DJ, Haluska P, & Markovic SN (2009) Cancer and pregnancy: parallels in growth, invasion, and immune modulation and implications for cancer therapeutic agents. Mayo Clin Proc 84(11):985-1000.

53. Li R, et al. (2009) SOAP2: an improved ultrafast tool for short read alignment. Bioinformatics 25(15):1966-1967.

54. Chan KCA, Jiang P, Chan CW, Sun K, Wong J, Hui EP, Chan SL, Chan WC, Hui DS, Ng SS et al. 2013a. Noninvasive detection of cancer-associated genome-wide hypomethylation and copy number aberrations by plasma DNA bisulfite sequencing. Proc Natl Acad Sci U S A 110(47): 18761-18768.

55. Chan KCA, Jiang P, Sun K, Cheng YK, Tong YK, Cheng SH, Wong AI, Hudecova I, Leung TY, Chiu RWK et al. 2016. Second generation noninvasive fetal genome analysis reveals de novo mutations, single-base parental inheritance, and preferred DNA ends. Proc Natl Acad Sci U S A 113(50): E8159-E8168.

56. Chan KCA, Jiang P, Zheng YW, Liao GJ, Sun H, Wong J, Siu SS, Chan WC, Chan SL, Chan AT et al. 2013b. Cancer genome scanning in plasma: detection of tumor-associated copy number aberrations, single-nucleotide variants, and tumoral heterogeneity by massively parallel sequencing. Clin Chem 59(1): 211-224.

57. Chan KCA, Woo JKS, King A, Zee BCY, Lam WKJ, Chan SL, Chu SWI, Mak C, Tse IOL, Leung SYM et al. 2017. Analysis of plasma Epstein-Barr virus DNA to screen for nasopharyngeal cancer. N Engl J Med 377(6): 513-522.

58. Chim SSC, Tong YK, Chiu RW, Lau TK, Leung TN, Chan LY, Oudejans CB, Ding C, Lo YM. 2005. Detection of the placental epigenetic signature of the maspin gene in maternal plasma. Proc Natl Acad Sci U S A 102(41): 14753-14758.

59. Christie EL, Fereday S, Doig K, Pattnaik S, Dawson SJ, Bowtell DDL. 2017. Reversion of BRCA1/2 germline mutations detected in circulating tumor DNA from patients with high-grade serous ovarian cancer. J Clin Oncol 35(12): 1274-1280.

60. Cleveland WS. 1979. Robust locally weighted regression and smoothing scatterplots. Journal of the American Statistical Association 74(368): 829-836.

61. Cohen JD, Li L, Wang Y, Thoburn C, Afsari B, Danilova L, Douville C, Javed AA, Wong F, Mattox A et al. 2018. Detection and localization of surgically resectable cancers with a multi-analyte blood test. Science 359(6378): 926-930.

62. Eisenberg E, Levanon EY. 2013. Human housekeeping genes, revisited. Trends Genet 29(10): 569-574.

63. Gaffney DJ, McVicker G, Pai AA, Fondufe-Mittendorf YN, Lewellen N, Michelini K, Widom J, Gilad Y, Pritchard JK. 2012. Controls of nucleosome positioning in the human genome. PLoS Genet 8(11): e1003036.

64. Gai W, Ji L, Lam WKJ, Sun K, Jiang P, Chan AWH, Wong J, Lai PBS, Ng SSM, Ma BBY et al. 2018. Liver- and colon-specific DNA methylation markers in plasma for investigation of colorectal cancers with or without liver metastases. Clin Chem (doi: 10.1373/clinchem.2018.290304).

65. Grunau C, Clark SJ, Rosenthal A. 2001. Bisulfite genomic sequencing: systematic investigation of critical experimental parameters. Nucleic Acids Res 29(13): E65-65.

66. Hulbert A, Jusue-Torres I, Stark A, Chen C, Rodgers K, Lee B, Griffin C, Yang A, Huang P, Wrangle J et al. 2017. Early detection of lung cancer using DNA promoter hypermethylation in plasma and sputum. Clin Cancer Res 23(8): 1998-2005.

67. Ivanov M, Baranova A, Butler T, Spellman P, Mileyko V. 2015. Non-random fragmentation patterns in circulating cell-free DNA reflect epigenetic regulation. BMC Genomics 16 Suppl 13: S1.

68. Jahr S, Hentze H, Englisch S, Hardt D, Fackelmayer FO, Hesch RD, Knippers R. 2001. DNA fragments in the blood plasma of cancer patients: quantitations and evidence for their origin from apoptotic and necrotic cells. Cancer Res 61(4): 1659-1665.

69. Jiang C, Pugh BF. 2009. Nucleosome positioning and gene regulation: advances through genomics. Nat Rev Genet 10(3): 161-172.

70. Jiang P, Chan CW, Chan KC, Cheng SH, Wong J, Wong VW, Wong GL, Chan SL, Mok TS, Chan HL et al. 2015. Lengthening and shortening of plasma DNA in hepatocellular carcinoma patients. Proc Natl Acad Sci U S A 112(11): E1317-1325.

71. Jiang P, Sun K, Lun FMF, Guo AM, Wang H, Chan KCA, Chiu RWK, Lo YMD, Sun H. 2014. Methy-pipe: an integrated bioinformatics pipeline for whole genome bisulfite sequencing data analysis. PLoS One 9(6): e100360.

72. Kang S, Li Q, Chen Q, Zhou Y, Park S, Lee G, Grimes B, Krysan K, Yu M, Wang W et al. 2017. CancerLocator: non-invasive cancer diagnosis and tissue-of-origin prediction using methylation profiles of cell-free DNA. Genome Biol 18(1): 53.

73. Kapushesky M, Emam I, Holloway E, Kurnosov P, Zorin A, Malone J, Rustici G, Williams E, Parkinson H, Brazma A. 2010. Gene expression atlas at the European bioinformatics institute. Nucleic Acids Res 38(Database issue): D690-698.

74. Koohy H, Down TA, Spivakov M, Hubbard T. 2014. A comparison of peak callers used for DNase-Seq data. PLoS One 9(5): e96303.

75. Lam WKJ, Gai W, Sun K, Wong RSM, Chan RWY, Jiang P, Chan NPH, Hui WWI, Chan AWH, Szeto CC et al. 2017. DNA of erythroid origin is present in human plasma and informs the types of anemia. Clin Chem 63(10): 1614-1623.

76. Langmead B, Trapnell C, Pop M, Salzberg SL. 2009. Ultrafast and memory-efficient alignment of short DNA sequences to the human genome. Genome Biol 10(3): R25.

77. Lehmann-Werman R, Magenheim J, Moss J, Neiman D, Abraham O, Piyanzin S, Zemmour H, Fox I, Dor T, Grompe M et al. 2018. Monitoring liver damage using hepatocyte-specific methylation markers in cell-free circulating DNA. JCI Insight 3(12).

78. Lehmann-Werman R, Neiman D, Zemmour H, Moss J, Magenheim J, Vaknin-Dembinsky A, Rubertsson S, Nellgard B, Blennow K, Zetterberg H et al. 2016. Identification of tissue-specific cell death using methylation patterns of circulating DNA. Proc Natl Acad Sci U S A 113(13): E1826-1834.

79. Li W, Li Q, Kang S, Same M, Zhou Y, Sun C, Liu CC, Matsuoka L, Sher L, Wong WH et al. 2018. CancerDetector: ultrasensitive and non-invasive cancer detection at the resolution of individual reads using cell-free DNA methylation sequencing data. Nucleic Acids Res (doi: 10.1093/nar/gky423).

80. Lister R, O'Malley RC, Tonti-Filippini J, Gregory BD, Berry CC, Millar AH, Ecker JR. 2008. Highly integrated single-base resolution maps of the epigenome in Arabidopsis. Cell 133(3): 523-536.

81. Lo YMD, Chan KCA, Sun H, Chen EZ, Jiang P, Lun FM, Zheng YW, Leung TY, Lau TK, Cantor CR et al. 2010. Maternal plasma DNA sequencing reveals the genome-wide genetic and mutational profile of the fetus. Sci Transl Med 2(61): 61ra91.

82. Lo YMD, Corbetta N, Chamberlain PF, Rai V, Sargent IL, Redman CW, Wainscoat JS. 1997. Presence of fetal DNA in maternal plasma and serum. Lancet 350(9076): 485-487.

83. Lo YMD, Tein MS, Pang CC, Yeung CK, Tong KL, Hjelm NM. 1998. Presence of donor-specific DNA in plasma of kidney and liver-transplant recipients. Lancet 351(9112): 1329-1330.

84. Lui YYN, Chik KW, Chiu RW, Ho CY, Lam CW, Lo YM. 2002. Predominant hematopoietic origin of cell-free DNA in plasma and serum after sex-mismatched bone marrow transplantation. Clin Chem 48(3): 421-427.

85. Lun FMF, Chiu RWK, Sun K, Leung TY, Jiang P, Chan KC, Sun H, Lo YM. 2013. Noninvasive prenatal methylomic analysis by genomewide bisulfite sequencing of maternal plasma DNA. Clin Chem 59(11): 1583-1594.

86. Mandel P, Metais P. 1948. Les acides nucléiques du plasma sanguin chez l'homme. C R Seances Soc Biol Fil 142(3-4): 241-243.

87. Mertes F, Elsharawy A, Sauer S, van Helvoort JM, van der Zaag PJ, Franke A, Nilsson M, Lehrach H, Brookes AJ. 2011. Targeted enrichment of genomic DNA regions for next-generation sequencing. Brief Funct Genomics 10(6): 374-386.

88. O'Leary B, Hrebien S, Morden JP, Beaney M, Fribbens C, Huang X, Liu Y, Bartlett CH, Koehler M, Cristofanilli M et al. 2018. Early circulating tumor DNA dynamics and clonal selection with palbociclib and fulvestrant for breast cancer. Nat Commun 9(1): 896.

89. Olova N, Krueger F, Andrews S, Oxley D, Berrens RV, Branco MR, Reik W. 2018. Comparison of whole-genome bisulfite sequencing library preparation strategies identifies sources of biases affecting DNA methylation data. Genome Biol 19(1): 33.

90. Pedersen JS, Valen E, Velazquez AM, Parker BJ, Rasmussen M, Lindgreen S, Lilje B, Tobin DJ, Kelly TK, Vang S et al. 2014. Genome-wide nucleosome map and cytosine methylation levels of an ancient human genome. Genome Res 24(3): 454-466.

91. Phallen J, Sausen M, Adleff V, Leal A, Hruban C, White J, Anagnostou V, Fiksel J, Cristiano S, Papp E et al. 2017. Direct detection of early-stage cancers using circulating tumor DNA. Sci Transl Med 9(403).

92. Radman-Livaja M, Rando OJ. 2010. Nucleosome positioning: how is it established, and why does it matter? Dev Biol 339(2): 258-266.

93. Roadmap Epigenomics Consortium, Kundaje A, Meuleman W, Ernst J, Bilenky M, Yen A, Heravi-Moussavi A, Kheradpour P, Zhang Z, Wang J et al. 2015. Integrative analysis of 111 reference human epigenomes. Nature 518(7539): 317-330.

94. Samejima K, Earnshaw WC. 2005. Trashing the genome: the role of nucleases during apoptosis. Nat Rev Mol Cell Biol 6(9): 677-688.

95. Schep AN, Buenrostro JD, Denny SK, Schwartz K, Sherlock G, Greenleaf WJ. 2015. Structured nucleosome fingerprints enable high-resolution mapping of chromatin architecture within regulatory regions. Genome Res 25(11): 1757-1770.

96. Schones DE, Cui K, Cuddapah S, Roh TY, Barski A, Wang Z, Wei G, Zhao K. 2008. Dynamic regulation of nucleosome positioning in the human genome. Cell 132(5): 887-898.

97. Schutz E, Fischer A, Beck J, Harden M, Koch M, Wuensch T, Stockmann M, Nashan B, Kollmar O, Matthaei J et al. 2017. Graft-derived cell-free DNA, a noninvasive early rejection and graft damage marker in liver transplantation: A prospective, observational, multicenter cohort study. PLoS Med 14(4): e1002286.

98. Snyder MW, Kircher M, Hill AJ, Daza RM, Shendure J. 2016. Cell-free DNA comprises an in vivo nucleosome footprint that informs its tissues-of-origin. Cell 164(1-2): 57-68.

99. Strickler JH, Loree JM, Ahronian LG, Parikh AR, Niedzwiecki D, Pereira AAL, McKinney M, Korn WM, Atreya CE, Banks KC et al. 2018. Genomic landscape of cell-free DNA in patients with colorectal cancer. Cancer Discov 8(2): 164-173.

100. Stroun M, Anker P, Maurice P, Lyautey J, Lederrey C, Beljanski M. 1989. Neoplastic characteristics of the DNA found in the plasma of cancer patients. Oncology 46(5): 318-322.

101. Struhl K, Segal E. 2013. Determinants of nucleosome positioning. Nat Struct Mol Biol 20(3): 267-273.

102. Sun K, Jiang P, Chan KCA, Wong J, Cheng YK, Liang RH, Chan WK, Ma ES, Chan SL, Cheng SH et al. 2015. Plasma DNA tissue mapping by genome-wide methylation sequencing for noninvasive prenatal, cancer, and transplantation assessments. Proc Natl Acad Sci U S A 112(40): E5503-5512.

103. Sun K, Jiang P, Wong AIC, Cheng YKY, Cheng SH, Zhang H, Chan KCA, Leung TY, Chiu RWK, Lo YMD. 2018. Size-tagged preferred ends in maternal plasma DNA shed light on the production mechanism and show utility in noninvasive prenatal testing. Proc Natl Acad Sci U S A 115(22): E5106-E5114.

104. The ENCODE Project Consortium. 2012. An integrated encyclopedia of DNA elements in the human genome. Nature 489(7414): 57-74.

105. Ulz P, Heitzer E, Geigl JB, Speicher MR. 2017.

Patient monitoring through liquid biopsies using circulating tumor DNA. Int J Cancer 141(5): 887-896.

106. Ulz P, Thallinger GG, Auer M, Graf R, Kashofer K, Jahn SW, Abete L, Pristauz G, Petru E, Geigl JB et al. 2016. Inferring expressed genes by whole-genome sequencing of plasma DNA. Nat Genet 48(10): 1273-1278.

107. Valouev A, Johnson SM, Boyd SD, Smith CL, Fire AZ, Sidow A. 2011. Determinants of nucleosome organization in primary human cells. Nature 474(7352): 516-520.

108. van Opstal D, van Maarle MC, Lichtenbelt K, Weiss MM, Schuring-Blom H, Bhola SL, Hoffer MJV, Huijsdens-van Amsterdam K, Macville MV, Kooper AJA et al. 2017. Origin and clinical relevance of chromosomal aberrations other than the common trisomies detected by genome-wide NIPS: results of the TRIDENT study. Genet Med 20(5): 480-485.

109. Zemmour H, Planer D, Magenheim J, Moss J, Neiman D, Gilon D, Korach A, Glaser B, Shemer R, Landesberg G et al. 2018. Non-invasive detection of human cardiomyocyte death using methylation patterns of circulating DNA. Nat Commun 9(1): 1443.

110. Zhang Y, Liu T, Meyer CA, Eeckhoute J, Johnson DS, Bernstein BE, Nusbaum C, Myers RM, Brown M, Li W et al. 2008. Model-based analysis of ChIP-Seq (MACS). Genome Biol 9(9): R137.

**Claims**

1. A computer-implemented method of analyzing a biological sample, including a mixture of cell-free DNA molecules from a plurality of tissues types that includes a first tissue type, to determine a classification of a proportional contribution of the first tissue type in the mixture, the method comprising:

   identifying a first set of genomic positions at which ends of short cell-free DNA molecules occur at a first rate above a first threshold for samples containing the first tissue type, wherein the short cell-free DNA molecules have a first size;
   analyzing a first plurality of cell-free DNA molecules from the biological sample of a subject, wherein analyzing a cell-free DNA molecule includes:
   determining a genomic position in a reference genome corresponding to at least one end of the cell-free DNA molecule;
   based on the analyzing of the first plurality of cell-free DNA molecules, determining that a first number of the first plurality of cell-free DNA molecules end within one of a plurality of windows, each window including at least one of the first set of genomic positions;
   computing a relative abundance of the first plurality of cell-free DNA molecules ending within one of the plurality of windows by normalizing the first number of the first plurality of cell-free DNA molecules using a second number of cell-free DNA molecules, wherein the second number of cell-free DNA molecules includes cell-free DNA molecules ending at a second set of genomic positions outside of the plurality of windows including the first set of genomic positions; and
   determining the classification of the proportional contribution of the first tissue type by comparing the relative abundance to one or more calibration values determined from one or more calibration samples whose proportional contributions of the first tissue type are known.

2. The method of claim 1, wherein the relative abundance includes a ratio of the first number and the second number.

3. The method of claim 1 or claim 2, wherein the classification of the proportional contribution corresponds to a range above a specified percentage.

4. The method of any one of the preceding claims, wherein the first tissue type is a tumor, and wherein the classification is selected from a group consisting of: an amount of tumor tissue in the subject, a size of the tumor in the subject, a stage of the tumor in the subject, a tumor load in the subject, and presence of tumor metastasis in the subject.

5. The method of any one of the preceding claims, wherein identifying the first set of genomic positions includes:

   analyzing, by a computer system, a second plurality of cell-free DNA molecules from at least one additional sample to identify ending positions of the second plurality of cell-free DNA molecules, the second plurality of cell-free DNA molecules being short cell-free DNA molecules having the first size, wherein the at least one additional sample is known to include the first tissue type and is of a same sample type as the biological sample; and
   for each genomic window of a plurality of genomic windows:

      computing a corresponding number of the second plurality of cell-free DNA molecules ending on the genomic window; and
      comparing the corresponding number to a reference value to determine whether a rate of cell-free DNA molecules ending on one or more genomic positions within the genomic window is above the first threshold.

**6.** The method of claim 5, wherein the reference value is determined from numbers of the second plurality of cell-free DNA molecules ending at genomic positions outside of the genomic window.

**7.** The method of claim 6, wherein a particular genomic position is identified to be in the first set of genomic positions when the particular genomic position it at a peak relative to numbers of the second plurality of cell-free DNA molecules ending at the genomic positions within a window around the particular genomic position.

**8.** The method of claim 5, wherein the reference value is determined using a number of the second plurality of cell-free DNA molecules ending at a window centered around a particular genomic position of the genomic window divided by a mean size of cell-free DNA molecules.

**9.** The method of claim 5, wherein the reference value is an expected number of cell-free DNA molecules ending within the genomic window according to a probability distribution and an average length of cell-free DNA molecules in the least one additional sample.

**10.** The method of any one of the preceding claims, further comprising:
identifying the second set of genomic positions at which ends of long cell-free DNA molecules occur at a second rate above a second threshold, wherein the long cell-free DNA molecules have a second size that is greater than the first size.

**11.** The method of claim 10, wherein the first size is a first range of sizes, and wherein the second size is a second range of sizes.

**12.** The method of claim 11, wherein the first range of sizes is less than the second range of sizes by a first maximum of the first range of sizes being less than a second maximum of the second range of sizes.

**13.** The method of any one of claims 1-9, wherein the second set of genomic positions includes all genomic positions corresponding to an end of at least one of the first plurality of cell-free DNA molecules.

**14.** The method of any one of the preceding claims, wherein comparing the relative abundance to the one or more calibration values uses a calibration function fit to calibration points comprising proportional contributions of the first tissue type measured in a plurality of calibration samples and respective relative abundances determined in the plurality of calibration samples.

**15.** A computer program comprising plurality of instructions capable of execution by a computer system that, when executed, control the computer system to perform the methods of any one of the preceding claims.

**Patentansprüche**

**1.** Computerimplementiertes Verfahren des Analysierens einer biologischen Probe, einschließlich eines Gemischs aus zellfreien DNA-Molekülen, aus einer Vielzahl von Gewebearten, die eine erste Gewebeart beinhaltet, um eine Klassifikation eines proportionalen Anteils der ersten Gewebeart im Gemisch zu bestimmen, wobei das Verfahren Folgendes umfasst:

Identifizieren eines ersten Satzes von genomischen Positionen, an denen Enden von kurzen zellfreien DNA-Molekülen in einer ersten Rate über einem ersten Schwellenwert für Proben vorkommen, die die erste Gewebeart enthalten, wobei die kurzen zellfreien DNA-Moleküle eine erste Größe aufweisen;
Analysieren einer ersten Vielzahl von zellfreien DNA-Molekülen aus der biologischen Probe eines Subjekts, wobei das Analysieren eines zellfreien DNA-Moleküls Folgendes beinhaltet:
Bestimmen einer genomischen Position in einem Referenzgenom, entsprechend mindestens einem Ende des zellfreien DNA-Moleküls;
basierend auf dem Analysieren der ersten Vielzahl von zellfreien DNA-Molekülen, Bestimmen, dass eine erste Anzahl der ersten Vielzahl von zellfreien DNA-Molekülen in einem aus einer Vielzahl von Fenstern endet, wobei jedes Fenster mindestens einen aus dem ersten Satz von genomischen Positionen beinhaltet;
Berechnen einer relativen Häufigkeit der ersten Vielzahl von zellfreien DNA-Molekülen, die in einem aus der Vielzahl von Fenstern enden, durch Normalisieren der ersten Anzahl der ersten Vielzahl von zellfreien DNA-Molekülen unter Verwendung einer zweiten Anzahl von zellfreien DNA-Molekülen, wobei die zweite Anzahl von zellfreien DNA-Molekülen zellfreie DNA-Moleküle beinhaltet, die an einem zweiten Satz von genomischen Positionen außerhalb der Vielzahl von Fenstern enden, die den ersten Satz von genomischen Positionen beinhalten; und
Bestimmen der Klassifikation des proportionalen Anteils der ersten Gewebeart durch Vergleichen der relativen Häufigkeit mit einem oder mehreren Kalibrierwerten, bestimmt aus einer oder mehreren Kalibrierproben, deren proportionalen Anteile der ersten Gewebeart bekannt sind.

**2.** Verfahren nach Anspruch 1, wobei die relative Häufigkeit ein Verhältnis der ersten Anzahl und der zweiten Anzahl beinhaltet.

**3.** Verfahren nach Anspruch 1 oder Anspruch 2, wobei die Klassifikation des proportionalen Anteils einem Bereich über einem bestimmten Prozentsatz entspricht.

**4.** Verfahren nach einem der vorhergehenden Ansprüche, wobei die erste Gewebeart ein Tumor ist und wobei die Klassifikation ausgewählt ist aus einer Gruppe bestehend aus: einer Menge von Tumorgewebe in dem Subjekt, einer Größe des Tumors in dem Subjekt, eines Stadiums des Tumors in dem Subjekt, einer Tumorlast in dem Subjekt und Anwesenheit von Tumormetastase in dem Subjekt.

**5.** Verfahren nach einem der vorhergehenden Ansprüche, wobei das Identifizieren des ersten Satzes von genomischen Positionen Folgendes beinhaltet:

Analysieren, durch ein Computersystem, einer zweiten Vielzahl von zellfreien DNA-Molekülen aus mindestens einer zusätzlichen Probe, um Endpositionen der zweiten Vielzahl von zellfreien DNA-Molekülen zu identifizieren, wobei die zweite Vielzahl von zellfreien DNA-Molekülen kurze zellfreie DNA-Moleküle mit der ersten Größe sind, wobei bekannt ist, dass die mindestens eine zusätzliche Probe die erste Gewebeart beinhaltet und von einer gleichen Probenart wie die biologische Probe ist; und
für jedes genomische Fenster aus einer Vielzahl von genomischen Fenstern:

Berechnen einer entsprechenden Anzahl der zweiten Vielzahl von zellfreien DNA-Molekülen, die an dem genomischen Fenster enden; und
Vergleichen der entsprechenden Anzahl mit einem Referenzwert, um zu bestimmen, ob eine Rate von zellfreien DNA-Molekülen, die an einer oder mehreren genomischen Positionen in dem genomischen Fenster enden, über dem ersten Schwellenwert ist.

**6.** Verfahren nach Anspruch 5, wobei der Referenzwert aus Zahlen der zweiten Vielzahl von zellfreien DNA-Molekülen bestimmt ist, die an genomischen Positionen außerhalb des genomischen Fensters enden.

**7.** Verfahren nach Anspruch 6, wobei identifiziert ist, dass eine bestimmte genomische Position in dem ersten Satz von genomischen Positionen ist, wenn die bestimmte genomische Position an einer Spitze relativ zu Zahlen der zweiten Vielzahl von zellfreien DNA-Molekülen ist, die an den genomischen Positionen in einem Fenster um die bestimmte genomische Position enden.

**8.** Verfahren nach Anspruch 5, wobei der Referenzwert unter Verwendung einer Anzahl der zweiten Vielzahl von zellfreien DNA-Molekülen bestimmt ist, die an einem Fenster enden, das um eine bestimmte genomische Position des genomischen Fensters zentriert ist, geteilt durch eine mittlere Größe von zellfreien DNA-Molekülen.

**9.** Verfahren nach Anspruch 5, wobei der Referenzwert eine erwartete Anzahl von zellfreien DNA-Molekülen ist, die in dem genomischen Fenster enden, gemäß einer Wahrscheinlichkeitsverteilung und einer durchschnittlichen Länge von zellfreien DNA-Molekülen in der mindestens einen zusätzlichen Probe.

**10.** Verfahren nach einem der vorhergehenden Ansprüche, ferner umfassend:
Identifizieren des zweiten Satzes von genomischen Positionen, an denen Enden von langen zellfreien DNA-Molekülen vorkommen, in einer zweiten Rate über einem zweiten Schwellenwert, wobei die langen zellfreien DNA-Moleküle eine zweite Größe aufweisen, die größer als die erste Größe ist.

**11.** Verfahren nach Anspruch 10, wobei die erste Größe ein erster Größenbereich ist und wobei die zweite Größe ein zweiter Größenbereich ist.

**12.** Verfahren nach Anspruch 11, wobei der erste Größenbereich kleiner als der zweite Größenbereich ist, wobei ein erstes Maximum des ersten Größenbereichs kleiner als ein zweites Maximum des zweiten Größenbereichs ist.

**13.** Verfahren nach einem der Ansprüche 1-9, wobei der zweite Satz von genomischen Positionen alle genomischen Positionen beinhaltet, die einem Ende von mindestens einem aus der ersten Vielzahl von zellfreien DNA-Molekülen entsprechen.

**14.** Verfahren nach einem der vorhergehenden Ansprüche, wobei das Vergleichen der relativen Häufigkeit mit dem einen oder den mehreren Kalibrierwerten eine Kalibrierfunktion verwendet, die an Kalibrierpunkte angepasst ist, umfassend proportionale Anteile der ersten Gewebeart, gemessen in einer Vielzahl von Kalibrierproben und jeweiligen relativen Häufigkeiten, die in der Vielzahl von Kalibrierproben bestimmt sind.

**15.** Computerprogramm, umfassend eine Vielzahl von Anweisungen, die in der Lage sind, durch ein Computersystem ausgeführt zu werden, die, wenn sie ausgeführt werden, das Computersystem steuern, um die Verfahren nach einem der vorhergehenden Ansprüche durchzuführen.

**Revendications**

1. Procédé mis en œuvre par ordinateur pour analyser un échantillon biologique, comprenant un mélange de molécules d'ADN acellulaire d'une pluralité de types de tissus qui comprend un premier type de tissu, afin de déterminer une classification de la contribution proportionnelle du premier type de tissu dans le mélange, le procédé comprenant :

l'identification d'un premier ensemble de positions génomiques auxquelles les extrémités de courtes molécules d'ADN acellulaire apparaissent à un premier taux supérieur à un premier seuil pour les échantillons contenant le premier type de tissu, les courtes molécules d'ADN acellulaire ayant une première taille ;
l'analyse d'une première pluralité de molécules d'ADN acellulaire issues d'un échantillon biologique d'un sujet, l'analyse d'une molécule d'ADN acellulaire comprenant :
la détermination d'une position génomique dans un génome de référence correspondant à au moins une extrémité de la molécule d'ADN acellulaire ;
en fonction de l'analyse de la première pluralité de molécules d'ADN acellulaire, la détermination qu'un premier nombre de molécules de la première pluralité de molécules d'ADN acellulaire se termine dans une fenêtre d'une pluralité de fenêtres, chaque fenêtre comprenant au moins une position du premier ensemble de positions génomiques ;
le calcul d'une abondance relative de la première pluralité de molécules d'ADN acellulaire se terminant dans une fenêtre de la pluralité de fenêtres par normalisation du premier nombre de molécules de la première pluralité de molécules d'ADN acellulaire au moyen d'un deuxième nombre de molécules d'ADN acellulaire, ledit deuxième nombre de molécules d'ADN acellulaire comprenant des molécules d'ADN acellulaire se terminant au niveau d'un deuxième ensemble de positions génomiques en dehors de la pluralité de fenêtres comprenant le premier ensemble de positions génomiques ; et
la détermination de la classification de la contribution proportionnelle du premier type de tissu par comparaison de l'abondance relative à une ou plusieurs valeurs d'étalonnage déterminées à partir d'un ou plusieurs échantillons d'étalonnage dont les contributions proportionnelles du premier type de tissu sont connues.

2. Procédé selon la revendication 1, dans lequel l'abondance relative comprend un rapport entre le premier nombre et le deuxième nombre.

3. Procédé selon la revendication 1 ou la revendication 2, dans lequel la classification de la contribution proportionnelle correspond à une plage supérieure à un certain pourcentage.

4. Procédé selon l'une quelconque des revendications précédentes, dans lequel le premier type de tissu est une tumeur, et dans lequel la classification est sélectionnée dans un groupe constitué de : la quantité de tissu tumoral chez le sujet, la taille de la tumeur chez le sujet, le stade de la tumeur chez le sujet, la charge tumorale chez le sujet et la présence de métastases tumorales chez le sujet.

5. Procédé selon l'une quelconque des revendications précédentes, dans lequel l'identification du premier ensemble de positions génomiques comprend :

l'analyse, par un système informatique, d'une deuxième pluralité de molécules d'ADN acellulaire provenant d'au moins un échantillon supplémentaire pour identifier les positions d'extrémité de la deuxième pluralité de molécules d'ADN acellulaire, la deuxième pluralité de molécules d'ADN acellulaire étant constituée de courtes molécules d'ADN acellulaire ayant la première taille, ledit au moins un échantillon supplémentaire étant connu pour comprendre le premier type de tissu et étant du même type d'échantillon que l'échantillon biologique ; et
pour chaque fenêtre génomique d'une pluralité de fenêtres génomiques :

le calcul d'un nombre correspondant de molécules de la deuxième pluralité de molécules d'ADN acellulaire se terminant sur la fenêtre génomique ; et
la comparaison du nombre correspondant de molécules à une valeur de référence pour déterminer si un taux de molécules d'ADN acellulaire se terminant sur une ou plusieurs positions génomiques dans la fenêtre génomique est supérieur au premier seuil.

6. Procédé selon la revendication 5, dans lequel la valeur de référence est déterminée à partir des nombres de molécules de la deuxième pluralité de molécules d'ADN acellulaire se terminant à des positions génomiques en dehors de la fenêtre génomique.

7. Procédé selon la revendication 6, dans lequel une position génomique particulière est identifiée comme étant dans le premier ensemble de positions génomiques lorsque la position génomique particulière se trouve à un maximum par rapport aux nombres de molécules de la deuxième pluralité de molécules d'ADN acellulaire se terminant aux positions génomiques dans une fenêtre autour de la position génomique particulière.

8. Procédé selon la revendication 5, dans lequel la valeur de référence est déterminée au moyen du nombre de molécules de la deuxième pluralité de molécules d'ADN acellulaire se terminant au niveau d'une fenêtre centrée autour d'une position génomique particulière de la fenêtre génomique divisé par la taille moyenne des molécules d'ADN acellulaire.

9. Procédé selon la revendication 5, dans lequel la valeur de référence est un nombre attendu de molécules d'ADN acellulaire se terminant dans la fenêtre génomique conformément à une distribution de probabilité et une longueur moyenne des molécules d'ADN acellulaire dans l'au moins un échantillon supplémentaire.

10. Procédé selon l'une quelconque des revendications précédentes, comprenant en outre :
l'identification du deuxième ensemble de positions génomiques auxquelles les extrémités de longues molécules d'ADN acellulaire apparaissent à un deuxième taux supérieur à un deuxième seuil, les longues molécules d'ADN acellulaire ayant une deuxième taille qui est supérieure à la première taille.

11. Procédé selon la revendication 10, dans lequel la première taille est une première plage de tailles, et dans lequel la deuxième taille est une deuxième plage de tailles.

12. Procédé selon la revendication 11, dans lequel la première plage de tailles est inférieure à la deuxième plage de tailles dans le sens où un premier maximum de la première plage de tailles est inférieur à un deuxième maximum de la deuxième plage de tailles.

13. Procédé selon l'une quelconque des revendications 1 à 9, dans lequel le deuxième ensemble de positions génomiques comprend toutes les positions génomiques correspondant à une extrémité d'au moins une molécule de la première pluralité de molécules d'ADN acellulaire.

14. Procédé selon l'une quelconque des revendications précédentes, dans lequel la comparaison de l'abondance relative à une ou plusieurs valeurs d'étalonnage utilise une fonction d'étalonnage ajustée aux points d'étalonnage comprenant des contributions proportionnelles du premier type de tissu mesuré dans une pluralité d'échantillons d'étalonnage et des abondances relatives respectives déterminées dans la pluralité d'échantillons d'étalonnage.

15. Programme informatique comprenant une pluralité d'instructions capables d'être exécutées par un système informatique qui, une fois exécutées, commandent le système informatique pour qu'il réalise les procédés selon l'une quelconque des revendications précédentes.

FIG. 1

*FIG. 2*

*FIG. 3*

**FIG. 4A**

**FIG. 4B**

**FIG. 5A**

**FIG. 5B**

*FIG. 6*

*FIG. 7A*

*FIG. 7B*

**Size distribution comparison**

FIG. 8

*FIG. 9*

**FIG. 10**

EP 3 788 172 B1

FIG. 11

FIG. 12

EP 3 788 172 B1

FIG. 13

EP 3 788 172 B1

chr8p

**FIG. 14**

EP 3 788 172 B1

chr8q

**FIG. 15**

**FIG. 16**

**FIG. 17A**

**FIG. 17B**

FIG. 18A

FIG. 18B

**FIG. 19**

**FIG. 20A**

**FIG. 20B**

**FIG. 20C**

**FIG. 20D**

Pregnancy

**FIG. 20E**

Non-Pregnancy

*FIG. 21A*

*FIG. 21B*

FIG. 22

2300

| |
|---|
| Identify a first set of genomic positions at which ends of short cell-free DNA molecules occur at a first rate above a first threshold for samples containing a first tissue type, wherein the short cell-free DNA have a first size |

2310

| |
|---|
| Analyze cell-free DNA molecules to determine genomic positions in a reference genome corresponding to at least one end of the cell-free DNA molecule |

2320

| |
|---|
| Determine that a first number of the first plurality of cell-free DNA molecules end within one of a plurality of windows |

2330

| |
|---|
| Compute a relative abundance by normalizing the first number using a second number of cell-free DNA molecules ending at a second set of genomic positions |

2340

| |
|---|
| Determining a classification of a proportion of the first tissue type based on the relative abundance |

2350

**FIG. 23**

2400

Identify a first set of genomic positions at which ends of short cell-free DNA molecules occur at a first rate above a first threshold for samples containing a first tissue type, wherein the short cell-free DNA have a first size

2410

Analyze cell-free DNA molecules to determine genomic positions in a reference genome corresponding to at least one end of the cell-free DNA molecule

2420

Identify a group of the first plurality of cell-free DNA molecules that end within one of a plurality of windows including the first set of genomic positions and located in a chromosomal region

2430

Determine a value of the group of cell-free DNA molecules

2440

Determine a classification of whether a sequence imbalance exists in the first tissue type in the chromosomal region of the subject based on a comparison of the value to a reference value

2450

*FIG. 24*

**FIG. 25A**  Nucleosome positioning

2505
2514
2510
2512

Nucleosome    Nucleosome         Nucleosome

Linker    Linker    Open chromatin region    Linker

**FIG. 25B**  CfDNA generated from apoptosis

2520

2522

**FIG. 25C**  Sequencing and fragment end extraction

2530

2532

**FIG. 25D**  CfDNA genomic coverage profile

2532

Genomic coordinate

**FIG. 25E**  CfDNA fragment ends profile

2550

U    D   U    D    U    D   2552

Genomic coordinate

**FIG. 25F**  Smoothed cfDNA fragment end signals

2560                            2562

2572        2575        2574

**FIG. 26A**

FIG. 26B

FIG. 26C

*FIG. 26D*

*FIG. 27A*

EP 3 788 172 B1

**FIG. 27B**

**FIG. 27C**

**FIG. 28A**

**FIG. 28B**

**FIG. 28C**

**FIG. 28D**

**FIG. 28E**

**FIG. 28F**

**FIG. 28G**

**FIG. 29A**

**FIG. 29B**

FIG. 30

| #Sid | Intestine | Liver | Lung | Ovary | Breast | Placenta | Tcell |
|------|-----------|-------|------|-------|--------|----------|-------|
| C309 | -0.5 | 8.1 | -4.0 | 0.7 | -10.9 | -6.3 | 24.8 |
| C310 | -7.4 | 17.8 | -4.3 | -0.1 | -14.8 | -4.4 | 21.0 |
| C311 | -4.4 | 8.0 | -2.2 | -1.3 | -13.5 | -6.5 | 21.8 |
| C312 | -7.4 | 8.8 | 0.8 | 0.4 | -13.0 | -3.4 | 18.8 |
| C313 | 0.2 | 10.1 | 1.7 | 2.1 | -3.1 | -2.4 | 29.3 |
| C314 | -0.5 | 10.3 | 0.3 | 1.4 | -1.0 | -1.8 | 20.3 |
| C315 | 1.2 | 14.7 | -4.1 | 1.4 | -4.4 | 1.0 | 29.1 |
| C316 | 1.8 | 10.8 | -2.0 | 1.8 | -6.3 | -2.1 | 22.2 |
| C325 | 0.3 | 17.7 | -1.1 | 4.1 | -2.2 | -0.7 | 23.0 |
| C326 | 2.8 | 0.2 | 1.7 | 1.2 | -9.4 | -3.5 | 22.3 |
| C327 | 1.0 | 8.9 | -1.2 | 0.0 | -5.1 | -0.4 | 19.7 |
| C329 | 0.1 | 17.6 | -0.5 | 0.7 | -4.5 | -1.0 | 30.9 |
| C330 | 5.6 | 11.6 | 9.2 | 0.9 | -6.0 | 2.2 | 18.6 |
| C331 | 2.9 | 5.5 | 2.2 | -1.2 | -5.5 | 2.1 | 28.0 |
| C332 | -0.4 | 12.2 | -0.4 | -0.1 | -4.0 | -3.5 | 21.3 |
| C333 | 4.5 | 12.9 | 7.0 | 5.4 | -2.5 | 3.8 | 25.1 |
| C343 | -6.0 | 0.8 | -8.5 | 0.2 | -6.7 | -1.4 | 16.5 |
| C345 | -4.4 | 14.5 | 3.9 | 2.2 | -1.3 | -2.9 | 21.3 |
| C346 | 0.5 | 7.6 | -2.9 | 1.3 | -10.0 | -4.8 | 17.5 |
| C347 | 4.4 | 14.6 | 6.4 | 3.5 | -1.0 | -4.1 | 31.0 |
| C348 | 1.3 | 14.1 | 0.5 | -3.8 | -8.8 | -7.2 | 20.7 |
| C350 | 1.0 | 15.0 | 1.8 | 2.1 | -6.6 | -3.5 | 26.5 |
| C351 | 6.7 | 8.5 | 3.8 | -1.7 | -7.0 | -1.1 | 28.4 |
| C352 | 0.7 | 5.5 | -2.0 | 0.3 | -4.6 | -4.6 | 23.2 |
| C353 | 3.9 | 17.5 | 0.0 | -3.3 | -11.2 | -3.1 | 22.7 |
| C354 | -0.9 | 16.9 | 0.8 | 5.2 | -6.2 | 2.1 | 22.9 |
| C355 | -1.4 | 7.0 | 4.0 | -4.8 | -9.6 | -1.8 | 24.7 |
| C356 | -1.1 | 12.0 | -0.2 | 2.8 | -13.4 | -0.8 | 19.4 |
| C357 | 3.1 | 13.3 | 2.3 | -1.6 | -2.9 | -0.5 | 17.9 |
| C358 | 3.2 | 18.2 | -0.6 | 1.9 | -7.8 | -3.8 | 24.7 |
| C359 | -0.5 | 13.1 | -0.1 | 0.1 | -4.0 | -0.2 | 22.9 |
| C360 | 1.2 | 6.9 | -7.6 | 1.2 | -0.7 | -0.1 | 23.6 |

*FIG. 31*

**FIG. 32A**

**FIG. 32B**

**FIG. 32C**

FIG. 32D

| #Sid | Intestine | Liver | Lung | Ovary | Breast | Placenta | Tcell | | Fetal DNA fraction (%) |
|---|---|---|---|---|---|---|---|---|---|
| 8378 | -3.3 | 15.8 | -6.2 | 5.1 | -6.3 | -3.5 | 17.1 | | 11.5 |
| 8547 | -0.6 | 7.8 | 13.5 | -0.1 | -4.7 | 3.0 | 18.4 | | 9.5 |
| 8553 | -2.6 | 11.0 | -1.7 | -3.4 | -5.8 | 2.1 | 20.4 | | 15.2 |
| 8667 | -9.9 | 22.6 | 2.8 | -4.1 | -10.5 | -1.1 | 19.9 | | 9.3 |
| 8715 | 1.3 | 5.4 | -1.3 | 5.9 | -9.2 | 12.5 | 11.1 | | 23.4 |
| 8736 | -3.2 | 9.1 | -4.7 | -3.0 | -2.7 | 4.3 | 19.9 | | 11.6 |
| 8777 | 3.4 | 24.1 | -1.5 | 2.0 | -9.7 | 2.7 | 10.0 | | 15.0 |
| 8816 | 3.4 | 2.8 | 3.3 | 1.8 | -4.8 | 2.8 | 13.4 | | 11.1 |
| 8822 | 2.2 | 0.1 | -2.9 | 2.1 | -9.6 | -2.4 | 9.2 | | 7.5 |
| 8868 | 3.2 | 19.4 | 2.2 | 2.1 | -8.5 | 4.4 | 16.6 | | 15.9 |
| 8872 | -3.4 | 10.1 | 0.4 | 1.2 | -7.6 | 5.2 | 19.8 | | 12.3 |
| 8901 | -1.6 | 10.3 | 2.2 | -0.7 | -10.5 | 2.3 | 18.1 | | 9.8 |
| 8903 | 2.5 | 6.4 | 5.3 | -3.5 | -8.0 | 3.2 | 14.9 | | 12.8 |
| 8941 | 1.6 | 21.8 | -5.6 | 5.2 | -2.8 | 3.7 | 19.8 | | 11.9 |
| 8949 | 3.5 | 9.2 | -5.2 | 5.9 | -11.4 | 4.9 | 18.3 | | 14.9 |
| 8950 | -2.4 | 5.6 | 8.8 | 0.1 | -7.4 | 0.5 | 20.6 | | 8.0 |
| 8957 | -4.2 | 18.2 | 6.4 | 0.8 | -9.3 | -3.7 | 12.5 | | 5.9 |
| 9018 | 6.1 | 13.2 | 1.6 | 1.1 | -18.9 | 5.9 | 8.8 | | 12.9 |
| 9087 | 6.1 | 10.7 | -3.2 | 3.2 | -7.6 | 8.4 | 9.3 | | 16.1 |
| 9100 | 0.4 | 21.2 | -3.4 | 7.2 | -13.6 | 5.8 | 10.7 | | 11.3 |
| 9109 | 2.1 | 2.1 | -6.3 | -0.8 | -15.7 | 4.5 | 22.8 | | 12.6 |
| 9160 | -3.7 | 16.4 | -0.1 | 1.5 | -8.8 | -0.7 | 17.3 | | 3.9 |
| 9195 | 1.9 | 10.5 | 7.4 | 2.6 | -6.6 | 1.9 | 21.3 | | 8.6 |
| 9229 | -5.3 | 2.6 | -4.4 | -2.5 | -11.9 | 1.5 | 15.0 | | 13.5 |
| 9238 | 3.4 | 10.3 | 0.0 | 4.9 | -4.8 | 5.3 | 20.0 | | 18.5 |
| 9242 | -7.4 | 12.7 | -2.3 | -4.0 | -13.8 | 1.6 | 16.6 | | 13.8 |

*FIG. 33*

| #Sid | Intestine | Liver | Lung | Ovary | Breast | Placenta | Tcell | | Donor DNA fraction (%) |
|---|---|---|---|---|---|---|---|---|---|
| T99 | -5.9 | 3.5 | -6.3 | -0.6 | -12.2 | -6.0 | 12.0 | | 11.6 |
| TBR1448 | 2.7 | 14.9 | -0.1 | 0.9 | -6.2 | -1.8 | 15.9 | | 2.6 |
| TBR1449 | 0.9 | 3.4 | 4.5 | -3.4 | -12.8 | -6.9 | 21.8 | | 5.3 |
| TBR1450 | 6.2 | 11.7 | -3.8 | -3.9 | -11.0 | -6.9 | 17.6 | | 23.7 |
| TBR1451 | -4.0 | 6.6 | -10.3 | -3.3 | -6.3 | -3.5 | 14.3 | | 3.3 |
| TBR1452 | -4.9 | 3.4 | -5.7 | -1.9 | -9.8 | -2.3 | 14.6 | | 2.6 |
| TBR1453 | 4.9 | 26.4 | -0.3 | -0.2 | -6.1 | -6.7 | 14.0 | | 39.9 |
| TBR1454 | -3.4 | 3.2 | -5.4 | 1.1 | -4.1 | -2.3 | 18.7 | | 4.8 |
| TBR1573 | -5.2 | 1.9 | -0.8 | 1.8 | -3.6 | -4.3 | 27.5 | | 1.8 |
| TBR1574 | -2.5 | 13.8 | -8.8 | 5.5 | -2.2 | -2.2 | 15.7 | | 16.5 |
| TBR1575 | 12.0 | 8.1 | -6.0 | 3.2 | -10.8 | -5.3 | 17.9 | | 13.4 |
| TBR1576 | 0.1 | 20.4 | -0.6 | 1.5 | -8.0 | -5.3 | 19.7 | | 10.1 |
| TBR1577 | -3.6 | 1.5 | -2.3 | -1.0 | -7.0 | -2.1 | 20.5 | | 2.1 |
| TBR1578 | 4.7 | 15.4 | -1.2 | 2.8 | -3.8 | -3.9 | 20.9 | | 18.4 |

*FIG. 34*

FIG. 35A

**FIG. 35B**

FIG. 35C

FIG. 35D

| #Sid | Intestine | Liver | Lung | Ovary | Breast | Placenta | Tcell | | Tumor DNA fraction (%) |
|---|---|---|---|---|---|---|---|---|---|
| H203 | -0.2 | 12.0 | -3.2 | 1.2 | -13.9 | -4.3 | 26.5 | | 0 |
| H209 | -1.9 | 4.4 | -3.2 | -1.3 | -3.6 | -0.7 | 21.8 | | 0 |
| H246 | -2.0 | 2.5 | -9.8 | -5.4 | -2.9 | -2.6 | 17.8 | | 0 |
| H247 | 5.4 | 6.2 | -5.4 | 4.8 | -4.7 | 1.7 | 25.7 | | 0 |
| H249 | -0.8 | 5.6 | -0.7 | 3.2 | -13.5 | -4.9 | 25.4 | | 0 |
| H253 | 1.1 | 7.6 | -7.0 | -0.8 | -10.2 | -3.7 | 22.2 | | 0 |
| H254 | 3.3 | 1.2 | 2.2 | -5.8 | -14.5 | -5.1 | 21.7 | | 0 |
| H269 | 4.3 | 8.3 | -1.5 | -2.6 | -0.8 | -3.5 | 20.5 | | 0 |
| H275 | 0.2 | 8.3 | -3.7 | 1.4 | -8.2 | 1.8 | 25.0 | | 0 |
| H288 | 4.4 | 4.5 | -3.1 | -4.1 | -5.2 | -4.2 | 21.3 | | 0 |
| H315 | 1.0 | 13.0 | -4.2 | -3.5 | -9.3 | -1.5 | 17.9 | | 0 |
| H324 | -10.0 | -4.5 | -11.6 | -2.3 | -15.6 | -10.6 | 16.7 | | 0 |
| H342 | -1.6 | -6.1 | -2.2 | -2.7 | -6.3 | -4.3 | 21.3 | | 0 |
| H421 | -3.0 | 1.9 | -0.8 | -4.5 | -1.6 | -4.5 | 20.6 | | 0 |
| H436 | 1.4 | -2.8 | -1.9 | -4.2 | -5.7 | -4.9 | 18.3 | | 0 |
| HOT417 | 4.4 | 19.7 | 6.3 | -0.2 | -6.4 | -1.6 | 24.9 | | 0 |
| H263 | -11.9 | -8.9 | -17.1 | -7.3 | -21.5 | -14.4 | 10.9 | | 0.9 |
| H220 | -7.0 | 13.1 | -11.9 | -11.0 | -12.3 | -9.3 | 17.4 | | 1.0 |
| HOT432 | 7.1 | 17.9 | 2.3 | -0.3 | -8.3 | 0.4 | 27.6 | | 1.0 |
| H250 | 2.4 | 3.7 | 0.0 | -1.4 | -3.0 | -0.2 | 23.7 | | 1.0 |
| H292 | -3.1 | -3.5 | -4.7 | 1.8 | -11.1 | -7.4 | 26.0 | | 1.1 |
| H424 | 1.6 | 6.2 | -5.1 | -1.6 | -5.3 | -2.4 | 20.5 | | 1.1 |

*FIG. 36A*

| | | | | | | | | | |
|---|---|---|---|---|---|---|---|---|---|
| H261 | -7.0 | -6.5 | -13.3 | -3.9 | -18.9 | -13.8 | 16.1 | | 1.1 |
| H206 | 0.7 | 15.3 | -6.0 | 1.2 | -11.6 | -6.5 | 18.5 | | 1.2 |
| H296 | 4.7 | 12.5 | -0.8 | -1.8 | -1.3 | -2.5 | 23.5 | | 1.2 |
| HOT410 | 4.3 | 17.9 | 0.9 | 1.2 | -4.1 | 3.3 | 22.1 | | 1.3 |
| H300 | -1.0 | 3.0 | -3.7 | -1.3 | -7.0 | -4.3 | 21.0 | | 1.3 |
| H196 | 0.3 | 10.2 | -2.9 | -1.9 | -7.4 | 0.4 | 23.3 | | 1.4 |
| H218 | -9.8 | 5.3 | -7.5 | 5.0 | -6.8 | -6.0 | 13.7 | | 1.5 |
| HOT428 | 0.5 | 21.7 | 5.1 | 3.5 | 1.8 | -1.2 | 23.7 | | 1.5 |
| H433 | -2.1 | 0.8 | -4.9 | 0.6 | -12.4 | -3.6 | 19.4 | | 1.5 |
| H341 | -2.2 | -2.8 | -4.8 | -0.9 | -9.1 | -9.3 | 24.3 | | 1.5 |
| HOT425 | 1.2 | 10.1 | -0.2 | 9.3 | -8.4 | -3.8 | 26.8 | | 1.5 |
| H311 | -1.7 | 4.6 | -9.7 | 4.3 | -13.0 | -8.3 | 12.4 | | 1.6 |
| H283 | -5.1 | 3.4 | -7.5 | -3.1 | -12.8 | -6.9 | 17.1 | | 1.6 |
| H374 | -3.4 | 7.4 | -4.2 | -2.8 | -15.3 | -4.5 | 23.6 | | 1.7 |
| HOT413 | 4.8 | 17.9 | 2.1 | 6.3 | -2.8 | -3.3 | 18.9 | | 1.7 |
| H285 | 0.7 | 6.8 | -9.4 | -4.5 | -7.6 | -4.3 | 19.9 | | 1.7 |
| HOT427 | 3.6 | 20.8 | 4.9 | -0.6 | -2.0 | 6.1 | 30.7 | | 1.7 |
| H223 | 0.6 | 13.8 | 0.2 | 2.9 | -9.3 | -3.3 | 14.0 | | 1.8 |
| HOT393 | -3.8 | 13.1 | -4.3 | 2.1 | -6.8 | -0.7 | 10.5 | | 1.8 |
| H323 | 1.0 | 14.2 | -9.2 | 4.5 | -6.3 | -6.1 | 11.1 | | 1.9 |
| HOT386 | -1.6 | 8.5 | -1.7 | -2.9 | -4.7 | -5.3 | 21.4 | | 2.0 |
| H225 | 4.6 | -0.9 | 5.7 | 5.8 | -11.0 | -5.2 | 14.0 | | 2.0 |
| H402 | -0.1 | 20.6 | -8.5 | 1.7 | -11.5 | -7.7 | 15.4 | | 2.0 |
| H228 | 1.4 | 10.5 | 4.5 | -2.0 | -9.3 | -0.2 | 30.0 | | 2.1 |
| H419 | 4.5 | 23.6 | -3.3 | 9.1 | -7.1 | -3.3 | 9.5 | | 2.1 |
| H377 | 4.6 | 6.7 | -5.4 | 5.3 | -7.1 | -2.4 | 15.8 | | 2.1 |
| HOT422 | 2.5 | 38.1 | 5.2 | 5.7 | -9.7 | 1.7 | 11.4 | | 2.1 |
| H278 | 4.3 | 18.3 | -5.6 | 0.4 | -9.4 | -5.2 | 25.8 | | 2.1 |

**FIG. 36B**

| | | | | | | | | | |
|---|---|---|---|---|---|---|---|---|---|
| H264 | -5.9 | -4.9 | -11.7 | -13.0 | -15.4 | -15.2 | 13.0 | | 2.1 |
| H232 | -3.0 | 17.2 | -5.7 | 8.7 | -8.9 | -7.2 | 6.1 | | 2.3 |
| H252 | 3.6 | 12.8 | 1.9 | -0.2 | -10.8 | 1.8 | 27.6 | | 2.3 |
| HOT397 | -0.4 | -1.7 | -14.3 | 4.4 | -9.3 | -6.4 | 13.0 | | 2.4 |
| H352 | -6.8 | 2.9 | -6.2 | -4.4 | -12.3 | -13.3 | 19.5 | | 2.4 |
| H230 | -0.4 | 9.4 | 0.9 | 1.0 | -9.8 | -3.6 | 23.1 | | 2.5 |
| H301 | 4.2 | 38.4 | 7.2 | 12.8 | 2.0 | 8.0 | 28.3 | | 2.5 |
| HOT394 | -0.6 | 7.0 | -11.0 | 1.9 | -9.5 | -7.8 | 17.4 | | 2.6 |
| H435 | 1.0 | -5.7 | -2.9 | 3.1 | -6.5 | -1.8 | 10.6 | | 2.6 |
| H321 | 1.8 | 9.3 | -9.3 | -3.2 | -12.9 | -9.3 | 17.1 | | 2.7 |
| H200 | -1.7 | 6.2 | -1.0 | 2.4 | -2.1 | -2.2 | 17.1 | | 2.8 |
| H256 | -3.8 | 0.4 | -7.8 | -5.2 | -16.2 | -12.1 | 17.8 | | 2.8 |
| H344 | -4.9 | 0.0 | -8.5 | -2.2 | -6.1 | -7.3 | 19.4 | | 2.9 |
| H226 | 3.5 | 4.9 | 0.1 | -1.4 | -6.7 | -1.2 | 24.4 | | 3.2 |
| H214 | 3.0 | 13.9 | -4.8 | 1.7 | -9.2 | -3.8 | 15.5 | | 3.4 |
| H390 | -1.5 | 10.6 | -5.6 | -3.3 | -11.1 | -5.5 | 19.0 | | 3.6 |
| H217 | -2.8 | 11.4 | -1.5 | 0.5 | -6.8 | -5.2 | 25.4 | | 3.6 |
| H420 | 2.8 | 17.0 | -7.0 | 4.0 | -3.6 | -5.2 | 10.8 | | 3.9 |
| HOT426 | 5.6 | 32.7 | 0.7 | 3.9 | 1.9 | -2.3 | 14.6 | | 4.1 |
| H239 | 3.0 | 16.8 | -1.4 | -0.6 | -11.6 | -4.4 | 21.1 | | 4.4 |
| GM1100 | 0.9 | 9.7 | -4.3 | 1.9 | -4.6 | -3.7 | 24.1 | | 5.0 |

FIG. 36C

| | | | | | | | | | |
|---|---|---|---|---|---|---|---|---|---|
| H381 | -4.4 | 1.2 | -9.9 | -2.1 | -13.1 | -7.3 | 17.7 | | 5.5 |
| H221 | 0.7 | 10.6 | -3.0 | -2.4 | -2.1 | -1.3 | 24.8 | | 5.6 |
| H266 | -9.2 | -3.2 | -9.3 | -9.0 | -24.8 | -17.0 | 10.8 | | 5.6 |
| H199 | 6.0 | 12.7 | 6.5 | -5.3 | -4.5 | -1.5 | 30.1 | | 5.7 |
| H267 | 1.9 | 10.0 | 3.0 | -3.1 | -9.1 | 1.4 | 28.4 | | 5.7 |
| H195 | 0.6 | 7.2 | -0.3 | 2.0 | -5.4 | -0.5 | 23.4 | | 6.2 |
| H216 | 0.5 | 22.4 | -1.7 | -1.7 | -11.9 | -6.5 | 13.9 | | 8.4 |
| H210 | 5.4 | 21.7 | -1.3 | -0.3 | -12.7 | -6.1 | 13.7 | | 8.6 |
| H262 | -3.7 | 3.3 | -9.2 | -7.3 | -15.1 | -11.9 | 11.1 | | 9.1 |
| H270 | 7.9 | 13.9 | -4.4 | 4.1 | -7.4 | -0.5 | 20.1 | | 9.3 |
| H272 | 5.2 | 23.0 | 1.2 | 1.3 | -6.0 | -3.3 | 20.6 | | 9.9 |
| H235 | 3.9 | 16.2 | -8.2 | -5.5 | -10.1 | -5.3 | 10.9 | | 10.8 |
| H234 | 11.5 | 24.3 | 7.3 | 0.2 | 2.4 | -0.1 | 18.2 | | 12.1 |
| HOT414 | 4.7 | 32.9 | -8.3 | 2.0 | -1.9 | -0.1 | 14.2 | | 18.8 |
| H258 | -0.4 | 14.6 | -13.2 | -7.1 | -10.8 | -8.2 | 22.8 | | 19.3 |
| HOT403 | 8.3 | 27.8 | 1.4 | -3.7 | -5.5 | -3.2 | 17.7 | | 19.5 |
| H423 | 2.5 | 21.8 | -2.5 | -3.5 | -7.4 | -2.7 | 17.5 | | 20.8 |
| HOT412 | 6.8 | 9.6 | -1.7 | -0.5 | -6.7 | -2.6 | 8.7 | | 27.5 |
| H291 | 4.4 | 29.0 | -13.3 | -1.3 | -9.3 | -4.5 | 6.9 | | 53.2 |

**FIG. 36D**

**FIG. 37A**

**FIG. 37B**

*FIG. 37C*

**FIG. 37D**

**FIG. 37E**

| #Sid | Intestine | Liver | Lung | Ovary | Breast | Placenta | Tcell |
|------|-----------|-------|------|-------|--------|----------|-------|
| IC05 | 5.1 | 23.0 | 13.1 | 4.1 | 1.8 | 4.2 | 24.0 |
| IC06 | 3.9 | 30.0 | 4.2 | 3.7 | -5.7 | 8.4 | 9.8 |
| IC10 | 3.1 | 13.2 | 0.8 | -3.2 | -12.8 | -5.3 | 9.1 |
| IC15 | 0.9 | 14.7 | -5.2 | -0.3 | -14.4 | -6.7 | 3.6 |
| IC20 | 3.2 | 13.8 | 4.3 | 1.2 | -3.0 | 3.0 | 5.5 |
| IC22 | 4.7 | 19.3 | 1.6 | 2.9 | 4.3 | 2.7 | 11.3 |
| IC28 | -0.2 | 18.6 | 5.0 | -0.4 | -3.1 | 1.2 | 11.8 |
| IC32 | 1.0 | 1.7 | 3.5 | 0.7 | -9.5 | -6.6 | 11.9 |
| IC42 | 2.9 | 13.0 | 3.3 | -1.0 | -8.3 | 4.0 | 9.6 |

*FIG. 38*

EP 3 788 172 B1

| #Sid | Intestine | Liver | Lung | Ovary | Breast | Placenta | Tcell | | Colon DNA contribution (%) |
|---|---|---|---|---|---|---|---|---|---|
| TBR901 | 18.6 | 43.6 | 13.3 | 6.2 | 0.4 | 9.0 | 25.8 | | 20.9 |
| TBR910 | 18.3 | 31.8 | 3.7 | -6.5 | 1.0 | 14.0 | 17.3 | | 16.0 |
| TBR911 | 4.5 | 20.7 | 10.1 | -1.1 | -10.0 | 4.8 | 13.2 | | 1.0 |
| TBR912 | 12.9 | 26.8 | 6.5 | 5.1 | -17.4 | 6.5 | 14.3 | | 15.7 |
| TBR914 | 6.7 | 17.9 | 11.1 | 3.0 | -9.4 | 9.4 | 18.4 | | 0.0 |
| TBR916 | 8.7 | 43.7 | 15.0 | 4.9 | -1.6 | 6.4 | 14.7 | | 5.5 |
| TBR917 | 3.8 | 28.2 | 3.2 | -2.5 | -7.9 | 8.4 | 19.1 | | 2.7 |
| TBR919 | 12.7 | 29.1 | 12.2 | 2.4 | -5.5 | 9.7 | 13.4 | | 3.6 |
| TBR921 | 14.3 | 32.2 | 7.1 | 1.5 | -9.6 | 9.4 | 15.5 | | 11.3 |
| TBR922 | 4.0 | 32.1 | 2.4 | -5.6 | -10.8 | 6.6 | 15.0 | | 0.8 |
| TBR924 | 7.2 | 21.5 | 5.7 | -1.5 | -8.5 | 5.2 | 14.9 | | 5.2 |

*FIG. 39*

EP 3 788 172 B1

4000

4010  Identify a first set of genomic positions that
have a specified distance from a center of a
tissue-specific open chromatin regions
corresponding to a first tissue type

4020  Analyze cell-free DNA molecules to (1) determine
genomic positions of both ends in a reference genome
and (2) classify one end as upstream and other end as
downstream

4030  Determine that a first number of the first
plurality of cell-free DNA molecules
have an upstream end at one of the first
set of genomic positions

4040  Determine that a second number of the
first plurality of cell-free DNA molecules
have an downstream end at one of the
first set of genomic positions

4050  Compute a separation value using between the first number and
the second number

4060  Determining a classification of a
proportion of the first tissue type based
on the relative abundance

*FIG. 40*

4100

4110 — Identify a first set of genomic positions that have a specified distance from a center of a tissue-specific open chromatin regions corresponding to a first tissue type

4120 — Analyze cell-free DNA molecules to (1) determine genomic positions of both ends in a reference genome and (2) classify one end as upstream and other end as downstream

4130 — Determine that a first number of the first plurality of cell-free DNA molecules have an upstream end at one of the first set of genomic positions

4140 — Determine that a second number of the first plurality of cell-free DNA molecules have an downstream end at one of the first set of genomic positions

4150 — Compute a separation value using between the first number and the second number

4160 — Determine a classification of whether a pathology exists in the first tissue type in the chromosomal region of the subject based on a comparison of the value to a reference value

*FIG. 41*

*FIG. 42*

| I/O CONTROLLER 71 | SYSTEM MEMORY 72 | CENTRAL PROCESSOR 73 | PRINTER 74 | Data Collection Device 85 |

75

| DISPLAY ADAPTER 82 | I/O PORT 77 | KEYBOARD 78 | STORAGE DEVICE(S) 79 | EXTERNAL INTERFACE 81 |

| MONITOR 76 |

10

**FIG. 43**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017012592 A1
- WO 2017012592 A
- US 20090087847 A
- US 20090029377 A
- US 20110105353 A
- US 20130040824 A
- US 20160201142 A
- US 20110276277 A
- WO 2017012544 A

### Non-patent literature cited in the description

- **SCHREIBER et al.** *Proc Natl Acad Sci*, 2013, vol. 110, 18910-18915
- **FLUSBERG et al.** *Nat Methods*, 2010, vol. 7, 461-465
- **PEPE et al.** Limitations of the Odds Ratio in Gauging the Performance of a Diagnostic, Prognostic, or Screening Marker. *Am. J. Epidemiol*, 2004, vol. 159 (9), 882-890
- **COOK**. Use and Misuse of the Receiver Operating Characteristic Curve in Risk Prediction. *Circulation*, 2007, vol. 115, 928-935
- **STRAVER**. *I Prenat Diagn*, 2016, vol. 36, 614-621
- **SNYDER et al.** *Cell*, 2016, vol. 164, 57-68
- **LUN et al.** *Proc Natl Acad Sci USA*, 2008, vol. 105, 19920-5
- **LO YMD et al.** Presence of fetal DNA in maternal plasma and serum. *Lancet*, 1997, vol. 350 (9076), 485-487
- **LO YMD et al.** Presence of donor-specific DNA in plasma of kidney and liver-transplant recipients. *Lancet*, 1998, vol. 351 (9112), 1329-1330
- **ULZ P** ; **HEITZER E** ; **GEIGL JB** ; **SPEICHER MR**. Patient monitoring through liquid biopsies using circulating tumor DNA. *Int J Cancer*, 2017, vol. 141 (5), 887-896
- **COHEN JD et al.** Detection and localization of surgically resectable cancers with a multi-analyte blood test. *Science*, 2018, vol. 359 (6378), 926-930
- **SCHUTZ E et al.** Graft-derived cell-free DNA, a noninvasive early rejection and graft damage marker in liver transplantation: A prospective, observational, multicenter cohort study. *PLoS Med*, 2017, vol. 14 (4), e1002286
- **CHAN KCA et al.** Analysis of plasma Epstein-Barr virus DNA to screen for nasopharyngeal cancer. *N Engl J Med*, 2017, vol. 377 (6), 513-522
- **LEHMANN-WERMAN R et al.** Identification of tissue-specific cell death using methylation patterns of circulating DNA. *Proc Natl Acad Sci U S A*, 2016, vol. 113 (13), E1826-1834
- **OPSTAL D et al.** Origin and clinical relevance of chromosomal aberrations other than the common trisomies detected by genome-wide NIPS: results of the TRIDENT study. *Genet Med Oct*, 2017, vol. 2
- **LO YMD et al.** Maternal plasma DNA sequencing reveals the genome-wide genetic and mutational profile of the fetus. *Sci Transl Med*, 2010, vol. 2 (61), 61ra91
- **STRUHL K & SEGAL E**. Determinants of nucleosome positioning. *Nat Struct Mol Biol*, 2013, vol. 20 (3), 267-273
- **CHIM SSC et al.** Detection of the placental epigenetic signature of the maspin gene in maternal plasma. *Proc Natl Acad Sci U S A*, 2005, vol. 102 (41), 14753-14758
- **SUN K et al.** Plasma DNA tissue mapping by genome-wide methylation sequencing for noninvasive prenatal, cancer, and transplantation assessments. *Proc Natl Acad Sci U S A*, 2015, vol. 112 (40), E5503-5512
- **LUI YYN et al.** Predominant hematopoietic origin of cell-free DNA in plasma and serum after sex-mismatched bone marrow transplantation. *Clin Chem*, 2002, vol. 48 (3), 421-427
- **CHAN KCA et al.** Size distributions of maternal and fetal DNA in maternal plasma. *Clin Chem*, 2004, vol. 50 (1), 88-92
- **SUN K et al.** Noninvasive reconstruction of placental methylome from maternal plasma DNA: potential for prenatal testing and monitoring. *Prenat Diagn*, 2018, vol. 38 (3), 196-203
- **SUN K et al.** COFFEE: control-free noninvasive fetal chromosomal examination using maternal plasma DNA. *Prenat Diagn*, 2017, vol. 37 (4), 336-340

- **YU SCY et al.** Size-based molecular diagnostics using plasma DNA for noninvasive prenatal testing. *Proc Natl Acad Sci U S A*, 2014, vol. 111 (23), 8583-8588
- **CIRIGLIANO V** ; **ORDONEZ E** ; **RUEDA L** ; **SYNGELAKI A** ; **NICOLAIDES KH**. Performance of the neoBona test: a new paired-end massively parallel shotgun sequencing approach for cell-free DNA-based aneuploidy screening. *Ultrasound Obstet Gynecol*, 2017, vol. 49 (4), 460-464
- **ZHANG L** ; **ZHU Q** ; **WANG H** ; **LIU S**. Count-based size-correction analysis of maternal plasma DNA for improved noninvasive prenatal detection of fetal trisomies 13, 18, and 21. *Am J Transl Res*, 2017, vol. 9 (7), 3469-3473
- **YU SCY et al.** High-resolution profiling of fetal DNA clearance from maternal plasma by massively parallel sequencing. *Clin Chem*, 2013, vol. 59 (8), 1228-1237
- **CHAN KCA et al.** Second generation noninvasive fetal genome analysis reveals de novo mutations, single-base parental inheritance, and preferred DNA ends. *Proc Natl Acad Sci U S A*, 2016, vol. 113 (50), E8159-E8168
- **JAHR S et al.** DNA fragments in the blood plasma of cancer patients: quantitations and evidence for their origin from apoptotic and necrotic cells. *Cancer Res*, 2001, vol. 61 (4), 1659-1665
- **STRAVER R** ; **OUDEJANS CB** ; **SISTERMANS EA** ; **REINDERS MJ**. Calculating the fetal fraction for noninvasive prenatal testing based on genome-wide nucleosome profiles. *Prenat Diagn*, 2016, vol. 36 (7), 614-621
- **SNYDER MW** ; **KIRCHER M** ; **HILL AJ** ; **DAZA RM** ; **SHENDURE J**. Cell-free DNA comprises an in vivo nucleosome footprint that informs its tissues-of-origin. *Cell*, 2016, vol. 164 (1-2), 57-68
- **IVANOV M** ; **BARANOVA A** ; **BUTLER T** ; **SPELLMAN P** ; **MILEYKO V**. Non-random fragmentation patterns in circulating cell-free DNA reflect epigenetic regulation. *BMC Genomics*, 2015, vol. 16, 13
- **CHIU RWK et al.** Noninvasive prenatal diagnosis of fetal chromosomal aneuploidy by massively parallel genomic sequencing of DNA in maternal plasma. *Proc Natl Acad Sci U S A*, 2008, vol. 105 (51), 20458-20463
- **DELONG ER** ; **DELONG DM** ; **CLARKE-PEARSON DL**. Comparing the areas under two or more correlated receiver operating characteristic curves: a nonparametric approach. *Biometrics*, 1988, vol. 44 (3), 837-845
- **JIANG P et al.** Lengthening and shortening of plasma DNA in hepatocellular carcinoma patients. *Proc Natl Acad Sci U S A*, 2015, vol. 112 (11), E1317-1325
- **VALOUEV A et al.** Determinants of nucleosome organization in primary human cells. *Nature*, 2011, vol. 474 (7352), 516-520
- **GAFFNEY DJ et al.** Controls of nucleosome positioning in the human genome. *PLoS Genet*, 2012, vol. 8 (11), e1003036
- **LAM WKJ et al.** DNA of erythroid origin is present in human plasma and informs the types of anemia. *Clin Chem*, 2017, vol. 63 (10), 1614-1623
- **ROADMAP EPIGENOMICS CONSORTIUM et al.** Integrative analysis of 111 reference human epigenomes. *Nature*, 2015, vol. 518 (7539), 317-330
- **JIANG C** ; **PUGH BF**. Nucleosome positioning and gene regulation: advances through genomics. *Nat Rev Genet*, 2009, vol. 10 (3), 161-172
- **HORLBECK MA et al.** Nucleosomes impede Cas9 access to DNA in vivo and in vitro. *Elife*, 2016, vol. 5, e12677
- **BUENROSTRO JD** ; **GIRESI PG** ; **ZABA LC** ; **CHANG HY** ; **GREENLEAF WJ**. Transposition of native chromatin for fast and sensitive epigenomic profiling of open chromatin, DNA-binding proteins and nucleosome position. *Nat Methods*, 2013, vol. 10 (12), 1213-1218
- **MUELLER B et al.** Widespread changes in nucleosome accessibility without changes in nucleosome occupancy during a rapid transcriptional induction. *Genes Dev*, 2017, vol. 31 (5), 451-462
- **BUENROSTRO JD** ; **WU B** ; **CHANG HY** ; **GREENLEAF WJ**. ATAC-seq: a method for assaying chromatin accessibility genome-wide. *Curr Protoc Mol Biol*, 2015, vol. 109, 21.29.1-9
- **SCHEP AN et al.** Structured nucleosome fingerprints enable high-resolution mapping of chromatin architecture within regulatory regions. *Genome Res*, 2015, vol. 25 (11), 1757-1770
- **CHODAVARAPU RK et al.** Relationship between nucleosome positioning and DNA methylation. *Nature*, 2010, vol. 466 (7304), 388-392
- **JENSEN TJ et al.** Whole genome bisulfite sequencing of cell-free DNA and its cellular contributors uncovers placenta hypomethylated domains. *Genome Biol*, 2015, vol. 16, 78
- **LUN FMF et al.** Noninvasive prenatal methylomic analysis by genomewide bisulfite sequencing of maternal plasma DNA. *Clin Chem*, 2013, vol. 59 (11), 1583-1594
- **JIANG P et al.** Gestational age assessment by methylation and size profiling of maternal plasma DNA: a feasibility study. *Clin Chem*, 2017, vol. 63 (2), 606-608
- **SCHROEDER DI et al.** The human placenta methylome. *Proc Natl Acad Sci U S A*, 2013, vol. 110 (15), 6037-6042
- **LEE JY** ; **LEE TH**. Effects of DNA methylation on the structure of nucleosomes. *J Am Chem Soc*, 2012, vol. 134 (1), 173-175
- **CHOY JS et al.** DNA methylation increases nucleosome compaction and rigidity. *J Am Chem Soc*, 2010, vol. 132 (6), 1782-1783

- **COLLINGS CK** ; **WADDELL PJ** ; **ANDERSON JN**. Effects of DNA methylation on nucleosome stability. *Nucleic Acids Res*, 2013, vol. 41 (5), 2918-2931
- **ROSE NR** ; **KLOSE RJ**. Understanding the relationship between DNA methylation and histone lysine methylation. *Biochim Biophys Acta*, 2014, vol. 1839 (12), 1362-1372
- **SOPPE WJ et al.** DNA methylation controls histone H3 lysine 9 methylation and heterochromatin assembly in Arabidopsis. *EMBO J*, 2002, vol. 21 (23), 6549-6559
- **SIMON M et al.** Histone fold modifications control nucleosome unwrapping and disassembly. *Proc Natl Acad Sci U S A*, 2011, vol. 108 (31), 12711-12716
- **EHRLICH M**. DNA hypomethylation in cancer cells. *Epigenomics*, 2009, vol. 1 (2), 239-259
- **CHAN KCA et al.** Noninvasive detection of cancer-associated genome-wide hypomethylation and copy number aberrations by plasma DNA bisulfite sequencing. *Proc Natl Acad Sci U S A*, 2013, vol. 110 (47), 18761-18768
- **HOLTAN SG** ; **CREEDON DJ** ; **HALUSKA P** ; **MARKOVIC SN**. Cancer and pregnancy: parallels in growth, invasion, and immune modulation and implications for cancer therapeutic agents. *Mayo Clin Proc*, 2009, vol. 84 (11), 985-1000
- **LI R et al.** SOAP2: an improved ultrafast tool for short read alignment. *Bioinformatics*, 2009, vol. 25 (15), 1966-1967
- **CHAN KCA** ; **JIANG P** ; **CHAN CW** ; **SUN K** ; **WONG J** ; **HUI EP** ; **CHAN SL** ; **CHAN WC** ; **HUI DS** ; **NG SS et al.** Noninvasive detection of cancer-associated genome-wide hypomethylation and copy number aberrations by plasma DNA bisulfite sequencing. *Proc Natl Acad Sci U S A*, 2013, vol. 110 (47), 18761-18768
- **CHAN KCA** ; **JIANG P** ; **SUN K** ; **CHENG YK** ; **TONG YK** ; **CHENG SH** ; **WONG AI** ; **HUDECOVA I** ; **LEUNG TY** ; **CHIU RWK et al.** Second generation noninvasive fetal genome analysis reveals de novo mutations, single-base parental inheritance, and preferred DNA ends. *Proc Natl Acad Sci U S A*, 2016, vol. 113 (50), E8159-E8168
- **CHAN KCA** ; **JIANG P** ; **ZHENG YW** ; **LIAO GJ** ; **SUN H** ; **WONG J** ; **SIU SS** ; **CHAN WC** ; **CHAN SL** ; **CHAN AT et al.** Cancer genome scanning in plasma: detection of tumor-associated copy number aberrations, single-nucleotide variants, and tumoral heterogeneity by massively parallel sequencing. *Clin Chem*, 2013, vol. 59 (1), 211-224
- **CHAN KCA** ; **WOO JKS** ; **KING A** ; **ZEE BCY** ; **LAM WKJ** ; **CHAN SL** ; **CHU SWI** ; **MAK C** ; **TSE IOL** ; **LEUNG SYM et al.** Analysis of plasma Epstein-Barr virus DNA to screen for nasopharyngeal cancer. *N Engl J Med*, 2017, vol. 377 (6), 513-522
- **CHIM SSC** ; **TONG YK** ; **CHIU RW** ; **LAU TK** ; **LEUNG TN** ; **CHAN LY** ; **OUDEJANS CB** ; **DING C** ; **LO YM**. Detection of the placental epigenetic signature of the maspin gene in maternal plasma. *Proc Natl Acad Sci U S A*, 2005, vol. 102 (41), 14753-14758
- **CHRISTIE EL** ; **FEREDAY S** ; **DOIG K** ; **PATTNAIK S** ; **DAWSON SJ** ; **BOWTELL DDL**. Reversion of BRCA1/2 germline mutations detected in circulating tumor DNA from patients with high-grade serous ovarian cancer. *J Clin Oncol*, 2017, vol. 35 (12), 1274-1280
- **CLEVELAND WS**. Robust locally weighted regression and smoothing scatterplots. *Journal of the American Statistical Association*, 1979, vol. 74 (368), 829-836
- **COHEN JD** ; **LI L** ; **WANG Y** ; **THOBURN C** ; **AFSARI B** ; **DANILOVA L** ; **DOUVILLE C** ; **JAVED AA** ; **WONG F** ; **MATTOX A et al.** Detection and localization of surgically resectable cancers with a multi-analyte blood test. *Science*, 2018, vol. 359 (6378), 926-930
- **EISENBERG E** ; **LEVANON EY**. Human housekeeping genes, revisited. *Trends Genet*, 2013, vol. 29 (10), 569-574
- **GAFFNEY DJ** ; **MCVICKER G** ; **PAI AA** ; **FONDUFE-MITTENDORF YN** ; **LEWELLEN N** ; **MICHELINI K** ; **WIDOM J** ; **GILAD Y** ; **PRITCHARD JK**. Controls of nucleosome positioning in the human genome. *PLoS Genet*, 2012, vol. 8 (11), e1003036
- **GAI W** ; **JI L** ; **LAM WKJ** ; **SUN K** ; **JIANG P** ; **CHAN AWH** ; **WONG J** ; **LAI PBS** ; **NG SSM** ; **MA BBY et al.** Liver- and colon-specific DNA methylation markers in plasma for investigation of colorectal cancers with or without liver metastases. *Clin Chem*, 2018
- **GRUNAU C** ; **CLARK SJ** ; **ROSENTHAL A**. Bisulfite genomic sequencing: systematic investigation of critical experimental parameters. *Nucleic Acids Res*, 2001, vol. 29 (13), E65-65
- **HULBERT A** ; **JUSUE-TORRES I** ; **STARK A** ; **CHEN C** ; **RODGERS K** ; **LEE B** ; **GRIFFIN C** ; **YANG A** ; **HUANG P** ; **WRANGLE J et al.** Early detection of lung cancer using DNA promoter hypermethylation in plasma and sputum. *Clin Cancer Res*, 2017, vol. 23 (8), 1998-2005
- **JAHR S** ; **HENTZE H** ; **ENGLISCH S** ; **HARDT D** ; **FACKELMAYER FO** ; **HESCH RD** ; **KNIPPERS R**. DNA fragments in the blood plasma of cancer patients: quantitations and evidence for their origin from apoptotic and necrotic cells. *Cancer Res*, 2001, vol. 61 (4), 1659-1665
- **JIANG P** ; **CHAN CW** ; **CHAN KC** ; **CHENG SH** ; **WONG J** ; **WONG VW** ; **WONG GL** ; **CHAN SL** ; **MOK TS** ; **CHAN HL et al.** Lengthening and shortening of plasma DNA in hepatocellular carcinoma patients. *Proc Natl Acad Sci U S A*, 2015, vol. 112 (11), E1317-1325

- **JIANG P** ; **SUN K** ; **LUN FMF** ; **GUO AM** ; **WANG H** ; **CHAN KCA** ; **CHIU RWK** ; **LO YMD** ; **SUN H.** Methy-pipe: an integrated bioinformatics pipeline for whole genome bisulfite sequencing data analysis. *PLoS One*, 2014, vol. 9 (6), e100360
- **KANG S** ; **LI Q** ; **CHEN Q** ; **ZHOU Y** ; **PARK S** ; **LEE G** ; **GRIMES B** ; **KRYSAN K** ; **YU M** ; **WANG W et al.** CancerLocator: non-invasive cancer diagnosis and tissue-of-origin prediction using methylation profiles of cell-free DNA. *Genome Biol*, 2017, vol. 18 (1), 53
- **KAPUSHESKY M** ; **EMAM I** ; **HOLLOWAY E** ; **KURNOSOV P** ; **ZORIN A** ; **MALONE J** ; **RUSTICI G** ; **WILLIAMS E** ; **PARKINSON H** ; **BRAZMA A.** Gene expression atlas at the European bioinformatics institute. *Nucleic Acids Res*, 2010, vol. 38, D690-698
- **KOOHY H** ; **DOWN TA** ; **SPIVAKOV M** ; **HUBBARD T.** A comparison of peak callers used for DNase-Seq data. *PLoS One*, 2014, vol. 9 (5), e96303
- **LAM WKJ** ; **GAI W** ; **SUN K** ; **WONG RSM** ; **CHAN RWY** ; **JIANG P** ; **CHAN NPH** ; **HUI WWI** ; **CHAN AWH** ; **SZETO CC et al.** DNA of erythroid origin is present in human plasma and informs the types of anemia. *Clin Chem*, 2017, vol. 63 (10), 1614-1623
- **LANGMEAD B** ; **TRAPNELL C** ; **POP M** ; **SALZBERG SL.** Ultrafast and memory-efficient alignment of short DNA sequences to the human genome. *Genome Biol*, 2009, vol. 10 (3), R25
- **LEHMANN-WERMAN R** ; **MAGENHEIM J** ; **MOSS J** ; **NEIMAN D** ; **ABRAHAM O** ; **PIYANZIN S** ; **ZEMMOUR H** ; **FOX I** ; **DOR T** ; **GROMPE M et al.** Monitoring liver damage using hepatocyte-specific methylation markers in cell-free circulating DNA. *JCI Insight*, 2018, vol. 3 (12)
- **LEHMANN-WERMAN R** ; **NEIMAN D** ; **ZEMMOUR H** ; **MOSS J** ; **MAGENHEIM J** ; **VAKNIN-DEMBINSKY A** ; **RUBERTSSON S** ; **NELLGARD B** ; **BLENNOW K** ; **ZETTERBERG H et al.** Identification of tissue-specific cell death using methylation patterns of circulating DNA. *Proc Natl Acad Sci U S A*, 2016, vol. 113 (13), E1826-1834
- **LI W** ; **LI Q** ; **KANG S** ; **SAME M** ; **ZHOU Y** ; **SUN C** ; **LIU CC** ; **MATSUOKA L** ; **SHER L** ; **WONG WH et al.** CancerDetector: ultrasensitive and non-invasive cancer detection at the resolution of individual reads using cell-free DNA methylation sequencing data. *Nucleic Acids Res*, 2018
- **LISTER R** ; **O'MALLEY RC** ; **TONTI-FILIPPINI J** ; **GREGORY BD** ; **BERRY CC** ; **MILLAR AH** ; **ECKER JR.** Highly integrated single-base resolution maps of the epigenome in Arabidopsis. *Cell*, 2008, vol. 133 (3), 523-536
- **LO YMD** ; **CHAN KCA** ; **SUN H** ; **CHEN EZ** ; **JIANG P** ; **LUN FM** ; **ZHENG YW** ; **LEUNG TY** ; **LAU TK** ; **CANTOR CR et al.** Maternal plasma DNA sequencing reveals the genome-wide genetic and mutational profile of the fetus. *Sci Transl Med*, 2010, vol. 2 (61), 61ra91
- **LO YMD** ; **CORBETTA N** ; **CHAMBERLAIN PF** ; **RAI V** ; **SARGENT IL** ; **REDMAN CW** ; **WAINSCOAT JS.** Presence of fetal DNA in maternal plasma and serum. *Lancet*, 1997, vol. 350 (9076), 485-487
- **LO YMD** ; **TEIN MS** ; **PANG CC** ; **YEUNG CK** ; **TONG KL** ; **HJELM NM.** Presence of donor-specific DNA in plasma of kidney and liver-transplant recipients. *Lancet*, 1998, vol. 351 (9112), 1329-1330
- **LUI YYN** ; **CHIK KW** ; **CHIU RW** ; **HO CY** ; **LAM CW** ; **LO YM.** Predominant hematopoietic origin of cell-free DNA in plasma and serum after sex-mismatched bone marrow transplantation. *Clin Chem*, 2002, vol. 48 (3), 421-427
- **LUN FMF** ; **CHIU RWK** ; **SUN K** ; **LEUNG TY** ; **JIANG P** ; **CHAN KC** ; **SUN H** ; **LO YM.** Noninvasive prenatal methylomic analysis by genomewide bisulfite sequencing of maternal plasma DNA. *Clin Chem*, 2013, vol. 59 (11), 1583-1594
- **MANDEL P** ; **METAIS P.** Les acides nucléiques du plasma sanguin chez l'homme. *C R Seances Soc Biol Fil*, 1948, vol. 142 (3-4), 241-243
- **MERTES F** ; **ELSHARAWY A** ; **SAUER S** ; **HELVOORT JM** ; **ZAAG PJ** ; **FRANKE A** ; **NILSSON M** ; **LEHRACH H** ; **BROOKES AJ.** Targeted enrichment of genomic DNA regions for next-generation sequencing. *Brief Funct Genomics*, 2011, vol. 10 (6), 374-386
- **O'LEARY B** ; **HREBIEN S** ; **MORDEN JP** ; **BEANEY M** ; **FRIBBENS C** ; **HUANG X** ; **LIU Y** ; **BARTLETT CH** ; **KOEHLER M** ; **CRISTOFANILLI M et al.** Early circulating tumor DNA dynamics and clonal selection with palbociclib and fulvestrant for breast cancer. *Nat Commun*, 2018, vol. 9 (1), 896
- **OLOVA N** ; **KRUEGER F** ; **ANDREWS S** ; **OXLEY D** ; **BERRENS RV** ; **BRANCO MR** ; **REIK W.** Comparison of whole-genome bisulfite sequencing library preparation strategies identifies sources of biases affecting DNA methylation data. *Genome Biol*, 2018, vol. 19 (1), 33
- **PEDERSEN JS** ; **VALEN E** ; **VELAZQUEZ AM** ; **PARKER BJ** ; **RASMUSSEN M** ; **LINDGREEN S** ; **LILJE B** ; **TOBIN DJ** ; **KELLY TK** ; **VANG S et al.** Genome-wide nucleosome map and cytosine methylation levels of an ancient human genome. *Genome Res*, 2014, vol. 24 (3), 454-466
- **PHALLEN J** ; **SAUSEN M** ; **ADLEFF V** ; **LEAL A** ; **HRUBAN C** ; **WHITE J** ; **ANAGNOSTOU V** ; **FIKSEL J** ; **CRISTIANO S** ; **PAPP E et al.** Direct detection of early-stage cancers using circulating tumor DNA. *Sci Transl Med*, 2017, vol. 9 (403)

- **RADMAN-LIVAJA M** ; **RANDO OJ**. Nucleosome positioning: how is it established, and why does it matter?. *Dev Biol*, 2010, vol. 339 (2), 258-266
- **ROADMAP EPIGENOMICS CONSORTIUM** ; **KUNDAJE A** ; **MEULEMAN W** ; **ERNST J** ; **BILENKY M** ; **YEN A** ; **HERAVI-MOUSSAVI A** ; **KHERADPOUR P** ; **ZHANG Z** ; **WANG J et al.** Integrative analysis of 111 reference human epigenomes. *Nature*, 2015, vol. 518 (7539), 317-330
- **SAMEJIMA K** ; **EARNSHAW WC**. Trashing the genome: the role of nucleases during apoptosis. *Nat Rev Mol Cell Biol*, 2005, vol. 6 (9), 677-688
- **SCHEP AN** ; **BUENROSTRO JD** ; **DENNY SK** ; **SCHWARTZ K** ; **SHERLOCK G** ; **GREENLEAF WJ**. Structured nucleosome fingerprints enable high-resolution mapping of chromatin architecture within regulatory regions. *Genome Res*, 2015, vol. 25 (11), 1757-1770
- **SCHONES DE** ; **CUI K** ; **CUDDAPAH S** ; **ROH TY** ; **BARSKI A** ; **WANG Z** ; **WEI G** ; **ZHAO K**. Dynamic regulation of nucleosome positioning in the human genome. *Cell*, 2008, vol. 132 (5), 887-898
- **SCHUTZ E** ; **FISCHER A** ; **BECK J** ; **HARDEN M** ; **KOCH M** ; **WUENSCH T** ; **STOCKMANN M** ; **NASHAN B** ; **KOLLMAR O** ; **MATTHAEI J et al.** Graft-derived cell-free DNA, a noninvasive early rejection and graft damage marker in liver transplantation: A prospective, observational, multicenter cohort study. *PLoS Med*, 2017, vol. 14 (4), e1002286
- **STRICKLER JH** ; **LOREE JM** ; **AHRONIAN LG** ; **PARIKH AR** ; **NIEDZWIECKI D** ; **PEREIRA AAL** ; **MCKINNEY M** ; **KORN WM** ; **ATREYA CE** ; **BANKS KC et al.** Genomic landscape of cell-free DNA in patients with colorectal cancer. *Cancer Discov*, 2018, vol. 8 (2), 164-173
- **STROUN M** ; **ANKER P** ; **MAURICE P** ; **LYAUTEY J** ; **LEDERREY C** ; **BELJANSKI M**. Neoplastic characteristics of the DNA found in the plasma of cancer patients. *Oncology*, 1989, vol. 46 (5), 318-322
- **STRUHL K** ; **SEGAL E**. Determinants of nucleosome positioning. *Nat Struct Mol Biol*, 2013, vol. 20 (3), 267-273
- **SUN K** ; **JIANG P** ; **CHAN KCA** ; **WONG J** ; **CHENG YK** ; **LIANG RH** ; **CHAN WK** ; **MA ES** ; **CHAN SL** ; **CHENG SH et al.** Plasma DNA tissue mapping by genome-wide methylation sequencing for noninvasive prenatal, cancer, and transplantation assessments. *Proc Natl Acad Sci U S A*, 2015, vol. 112 (40), E5503-5512
- **SUN K** ; **JIANG P** ; **WONG AIC** ; **CHENG YKY** ; **CHENG SH** ; **ZHANG H** ; **CHAN KCA** ; **LEUNG TY** ; **CHIU RWK** ; **LO YMD**. Size-tagged preferred ends in maternal plasma DNA shed light on the production mechanism and show utility in noninvasive prenatal testing. *Proc Natl Acad Sci U S A*, 2018, vol. 115 (22), E5106-E5114
- The ENCODE Project Consortium. 2012. An integrated encyclopedia of DNA elements in the human genome. *Nature*, vol. 489 (7414), 57-74
- **ULZ P** ; **THALLINGER GG** ; **AUER M** ; **GRAF R** ; **KASHOFER K** ; **JAHN SW** ; **ABETE L** ; **PRISTAUZ G** ; **PETRU E** ; **GEIGL JB et al.** Inferring expressed genes by whole-genome sequencing of plasma DNA. *Nat Genet*, 2016, vol. 48 (10), 1273-1278
- **VALOUEV A** ; **JOHNSON SM** ; **BOYD SD** ; **SMITH CL** ; **FIRE AZ** ; **SIDOW A**. Determinants of nucleosome organization in primary human cells. *Nature*, 2011, vol. 474 (7352), 516-520
- **OPSTAL D** ; **MAARLE MC** ; **LICHTENBELT K** ; **WEISS MM** ; **SCHURING-BLOM H** ; **BHOLA SL** ; **HOFFER MJV** ; **HUIJSDENS-VAN AMSTERDAM K** ; **MACVILLE MV** ; **KOOPER AJA et al.** Origin and clinical relevance of chromosomal aberrations other than the common trisomies detected by genome-wide NIPS: results of the TRIDENT study. *Genet Med*, 2017, vol. 20 (5), 480-485
- **ZEMMOUR H** ; **PLANER D** ; **MAGENHEIM J** ; **MOSS J** ; **NEIMAN D** ; **GILON D** ; **KORACH A** ; **GLASER B** ; **SHEMER R** ; **LANDESBERG G et al.** Non-invasive detection of human cardiomyocyte death using methylation patterns of circulating DNA. *Nat Commun*, 2018, vol. 9 (1), 1443
- **ZHANG Y** ; **LIU T** ; **MEYER CA** ; **EECKHOUTE J** ; **JOHNSON DS** ; **BERNSTEIN BE** ; **NUSBAUM C** ; **MYERS RM** ; **BROWN M** ; **LI W et al.** Model-based analysis of ChIP-Seq (MACS). *Genome Biol*, 2008, vol. 9 (9), R137